# EUROPEAN PATENT SPECIFICATION

(11) **EP 4 244 225 B1**
(45) Date of publication and mention of the grant of the patent: **03.06.2026**
(21) Application number: 21830607.4
(22) Date of filing: 09.11.2021
(51) Int. Cl.: C07D 471/04, A61P 35/00, A61P 35/02, A61K 31/437, A61K 45/06

(54) **N-[2-({4-[3-(ANILINO)-4-OXO-4,5,6,7-TETRAHYDRO-1H-PYRROLO[3,2-C]PYRIDIN-2-YL]PYRIDIN-3-YL}OXY)ETHYL]PROP-2-ENAMIDE DERIVATIVES AND SIMILAR COMPOUNDS AS EGFR INHIBITORS FOR THE TREATMENT OF CANCER**
N-[2-({4-[3-(ANILINO)-4-OXO-4,5,6,7-TETRAHYDRO-1H-PYRROLO[3,2-C]PYRIDIN-2-YL]PYRIDIN-3-YL}OXY)ETHYL]PROP-2-ENAMID-DERIVATE UND ÄHNLICHE VERBINDUNGEN ALS EGFR-INHIBITOREN ZUR BEHANDLUNG VON KREBS
DÉRIVÉS DE N-[2-({4-[3-(ANILINO)-4-OXO-4,5,6,7-TETRAHYDRO-1H-PYRROLO[3,2-C]PYRIDIN-2-YL]PYRIDIN-3-YL}OXY)ÉTHYL]PROP-2-ÉNAMIDE ET COMPOSÉS SIMILAIRES EN TANT QU'INHIBITEURS D'EGFR POUR LE TRAITEMENT DU CANCER

(30) Priority: 11.11.2020 US 202063112498 P
(43) Date of publication of application: 20.09.2023
(73) Proprietor: Bayer Aktiengesellschaft, 51373 Leverkusen (DE); Bayer Pharma Aktiengesellschaft, 13353 Berlin (DE); The Broad Institute, Inc., Cambridge, MA 02142 (US); Dana-Farber Cancer Institute, Inc., Boston, Massachusetts 02215 (US)
(72) Inventor: SIEGEL, Stephan, 10779 Berlin (DE); SIEGEL, Franziska, Whippany, NJ 07981-1544 (US); SCHULZE, Volker, 16562 Hohen Neuendorf, OT Bergfelde (DE); BERGER, Markus, 12167 Berlin (DE); GRAHAM, Keith, 10405 Berlin (DE); KLAR, Ulrich, 13503 Berlin (DE); MORTIER, Jeremie Xavier G., 12055 Berlin (DE); SÜLZLE, Detlev, 13465 Berlin (DE); BÖMER, Ulf, 16548 Glienicke (DE); KORR, Daniel, 10247 Berlin (DE); SCHRÖDER, Jens, 99425 Weimar (DE); MEYERSON, Matthew, Boston, Massachusetts 02215 (US); GREULICH, Heidi, Cambridge, Massachusetts 02142 (US); KAPLAN, Bethany, Cambridge, Massachusetts 02142 (US)
(74) Representative: BIP Patents
(86) International application number: PCT/EP2021/081081
(87) International publication number: WO 2022/101184

(56) References cited:
- WO-A1-2020/001350
- WO-A1-2020/216781

## Description

### Field of application of the invention

The present invention covers acrylamide derivatives of general formula (I) as described and defined herein, methods of preparing said compounds, intermediate compounds useful for preparing said compounds, pharmaceutical compositions and combinations comprising said compounds, and the use of said compounds for manufacturing pharmaceutical compositions for the treatment or prophylaxis of diseases, in particular cancer, as a sole agent or in combination with other active ingredients.

### BACKGROUND OF THE INVENTION

The present invention covers acrylamide derivatives of general formula (I) which inhibit EGFR.

The Epidermal Growth Factor Receptor (EGFR or EGF-receptor) receptor tyrosine kinase family consists of 4 members: EGFR (Erbb1, Her1), ERBB2 (Her2), ERBB3 (Her3), and ERBB4 (Her4). EGFR mediates activation of MAPK and PI3K signaling pathways and thereby regulates cell proliferation, differentiation, migration and survival (Pao et al., 2010). EGFR gene amplification, overexpression, and mutations are frequently observed in various cancer indications and are associated with a poor prognosis (Gridelli et al., 2015).

In lung adenocarcinoma, mutations of EGFR are prevalent in approximately 15% of Western patients and up to 50% of East Asian patients (Paez et al., 2004). These mutations typically occur in one of four exons, exons 18-21, in the kinase domain of EGFR (Paez et al., 2004). The most common activating mutations in EGFR are a point mutation in exon 21, substituting an arginine for a leucine (L858R), and a small in-frame deletion in exon 19 that removes four amino acids (del 19/del746-750) (Pao et al., 2010). The FDA-approved inhibitors gefitinib, erlotinib, and afatinib, targeting mutations in exons 18, 19, and 21 of EGFR, are effective in patients but the response is often not durable (Mok et al., 2009; Sequist et al., 2013). Resistance frequently occurs in these patients in response to acquisition of a second mutation, T790M (Pao et al., 2005). Second generation inhibitors, e.g. afatinib, irreversibly target this mutation but are still potent inhibitors of wild-type EGFR, leading to dose-limiting toxicity and lack of efficacy in patients.

Several irreversible EGFR inhibitors are published in CN 110857292, IN 201821027709, CN 110698461, WO 2020001351, WO 2020001350, WO 2019233459, CN 110407852, CN 110357863, WO 2019070167, WO20061470. WO2019/081486 describes 4H-Pyrrolo[3,2-c]pyridine-4-one derivatives.

A third-generation irreversible inhibitor, osimertinib, that maximizes activity towards T790M while minimizing activity towards wild-type EGFR, is effective in T790M mutant patients and is currently the standard treatment for T790M positive patients (Mok et al., 2017). Osimertinib is also approved as a front-line therapy for patients with mutations of EGFR exons 19 or 21 (Soria et al., 2018).

However, patients also develop resistance to irreversible third-generation EGFR inhibitors, such as osimertinib. One of the major osimertinib resistance mechanisms identified is mutation of the cysteine in position 797 to a serine, resulting in loss of the covalently interacting cysteine and loss of sensitivity to irreversible EGFR inhibitors, at which point progressing patients have currently only limited treatment options (Thress et al., 2015; Oxnard et al., 2018). Such C797S mutations can also occur when osimertinib is used as a first-line therapy, in the absence of the T790M mutation (Ramalingham et al., 2018a; Ramalingham et al., 2018b). A novel targeted therapy that is able to specifically address the EGFR-C797S acquired resistance mutation would be highly beneficial for those patients.

By contrast, and with the exception of A763_Y764insFQEA, small in-frame insertions of EGFR exon20 are resistant to all clinically-approved EGFR inhibitors at doses achievable in lung cancer patients and comprise an unmet medical need (Yasuda et. al., 2013).

Patients with EGFR exon20 insertions, such as V769_D770insASV, D770_N771insSVD, D770_N771insNPG, N771_P772insH, H773_V774insH, H773_V774insNPH, V774_C775insHV show particular low response rates to all currently approved EGFR-targeted therapies, resulting in significantly reduced progression-free survival as well as overall survival (Chen et al., 2016). This has been shown for the first-generation inhibitors erlotinib and gefitinib as well as for the second-generation inhibitor afatinib (Chen et al., 2016; Yang et al., 2015).

Therefore, the standard treatment for EGFR exon20 insertion patients is currently chemotherapy.

The same resistance profile has been observed for exon20 insertion mutations in ERBB2 (e.g. ERBB2 A775_G776insYVMA with the highest prevalence), another member of the EGF-receptor family (Arcila et al., 2012) and some of the uncommon EGFR mutations like L681Q (Chiu et al., 2015).

Several irreversible inhibitors are currently in clinical trials for the treatment of EGFR exon20 insertion patients: Osimertinib, initially approved for the treatment of T790M mutant NSCLC patients (Floc'h et al., 2018); poziotinib (HM-781-36B), a non-approved pan-Her inhibitor targeting EGFR, Her2/neu, and Her4 (Robichaux et al., 2018); as well as TAK-788 (AP32788) (Doebele et al., ASCO 2018). Of these, the first clinical data have been published for poziotinib and TAK-788. Both compounds clearly show clinical efficacy in EGFR exon20 insertion patients. However, major adverse events, mediated by inhibition of wild-type EGFR, have been reported for both clinical trials and these adverse events may limit clinical utility.

More recently, new preclinical data has been published for two additional compounds showing activity on EGFR exon20 insertions: TAS6417 (TCP-064) and compound 1a (Hasako et al., 2018; Jang et al., 2018). No clinical results are yet available for these two compounds.

PCT patent application published as WO 2020/216781 A1 (Bayer Aktiengesellschaft, The Broad Institute, Inc. and Dana-Farber Cancer Institute, Inc.) relates to 4H-pyrrolo[3,2-c]pyridine-4-one compounds, a process for their preparation and uses thereof.

The compounds of the present document differ from the compounds in WO 2020/216781 A1 at least in the substituent R6 (WO 2020/216781 A1 : e.g. page 153 to page 257 ; Examples 1 to 75 ; Tables 1 to 9 ; claims 1, 4, 6, 8, 16 and 20).

In summary, mutant EGFR is a promising drug target for cancer therapy. In particular, patients with primary resistance to approved anti-EGFR therapies, due to EGFR exon20 insertions, have only few treatment options to date and there is a great need for novel alternative and/or improved therapeutics to provide these patients with an efficacious, well-tolerable therapy (Oxnard et al., 2013). Therefore, potent inhibitors of mutant EGFR, particularly of mutant EGFR with exon20 insertion mutations that show improved selectivity versus wild-type EGFR, represent valuable compounds that should complement therapeutic options either as single agents or in combination with other drugs.

### SUMMARY OF THE INVENTION

The invention provides compounds that inhibit a mutant EGFR; specifically, an EGFR comprising one or more exon 20 insertion mutations, an L858R mutation, or a small in-frame deletion of exon 19, in the presence or absence of a T790M mutation.

It has now been found that the compounds of the present invention have surprising and advantageous properties.

In particular, said compounds of the present invention have surprisingly been found to effectively inhibit mutant EGFR with exon 20 insertion mutations, particularly those harboring a D770_N771ins SVD exon 20 insertion. Furthermore it has been found that these compounds additionally show high cellular potency in EGFR V769_D770insASV, D770_N771insSVD, D770_N771insNPG, N771_P772insH, or H773_V774insNPH exon 20 insertion harboring BA/F3 cell lines.

Surprisingly, the here described compounds retain high cellular activity in BA/F3 cell lines harboring D770_N771insSVD and the T790M mutation.

In addition, the here described compounds potently inhibit proliferation of BA/F3 cell lines carrying EGFR activating mutations with or without T790M acquired resistance mutations (EGFR E746_A750del, L858R, E746_A750del T790M, L858R T790M).

Based on the described properties the here described compounds can therefore be used for the treatment or prophylaxis of diseases of uncontrolled cell growth, proliferation and/or survival, inappropriate cellular immune responses, or inappropriate cellular inflammatory responses or diseases which are accompanied with uncontrolled cell growth, proliferation and/or survival, inappropriate cellular immune responses, or inappropriate cellular inflammatory responses mediated by mutant EGFR with exon 20 insertion mutations, a L858R mutation, or a small in-frame deletion of exon 19 (e.g. EGFR E746_A750del) in the presence or absence of a T790M mutation and/or reduce (or block) proliferation in cells harboring EGFR with exon 20 insertion mutations, a L858R mutation, or a small in-frame deletion of exon 19 (e.g. EGFR E746_A750del) in the presence or absence of a T790M mutation, for example, haematological tumours, solid tumours, and/or metastases thereof, *e.g.* leukaemias and myelodysplastic syndrome, malignant lymphomas, head and neck tumours including brain tumours and brain metastases, tumours of the thorax including non-small cell and small cell lung tumours, gastrointestinal tumours, endocrine tumours, mammary and other gynaecological tumours, urological tumours including renal, bladder and prostate tumours, skin tumours, and sarcomas, and/or metastases thereof.

### Description of the invention

In accordance with a first aspect, the invention relates to compounds of formula (I), 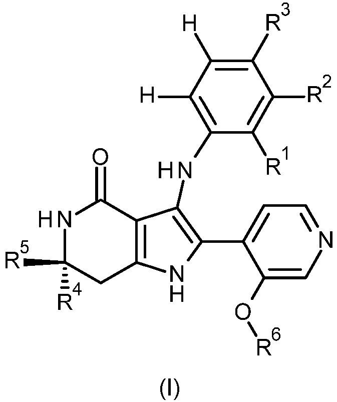 in which:
- R¹: represents hydrogen, methyl, ethyl, trifluoromethyl, 2,2-difluoroethyl, cyano, chloro, bromo, methoxy, or difluoromethoxy;
- R²: represents hydrogen, methyl, ethyl, fluoro, chloro, or bromo;
- R³: represents hydrogen or fluoro;
- R⁴: represents hydrogen, methyl, or trifluoromethyl;
- R⁵: represents hydrogen, methyl, or trifluoromethyl, or
- R⁴ and R⁵,: together with the carbon atom to which they are attached, represent a C₃-C₆-cycloalkyl group;
- R⁶: represents a group selected from the group: 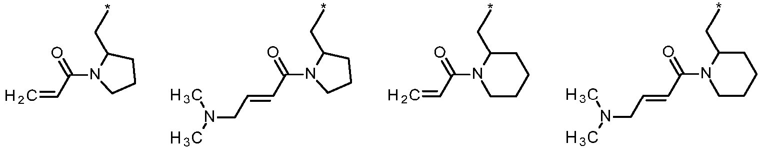 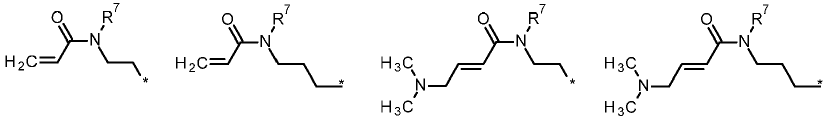 wherein * indicates the point of attachment of said group to the oxygen atom of the compound of formula (I);
- R⁷: represents hydrogen or methyl;
or an N-oxide, a salt or a tautomer of said compound, or a salt of said N-oxide or tautomer.

In a second aspect, the invention relates to compounds of formula (I) as described *supra,* wherein:
- R¹: represents hydrogen, methyl, methoxy, or difluoromethoxy;
- R²: represents hydrogen, fluoro, or chloro;
- R³: represents hydrogen or fluoro;
- R⁴: represents hydrogen or methyl;
- R⁵: represents hydrogen or methyl, or
- R⁴ and R⁵,: together with the carbon atom to which they are attached, represent a C₃-C₅-cycloalkyl group;
- R⁶: represents a group selected from the group: 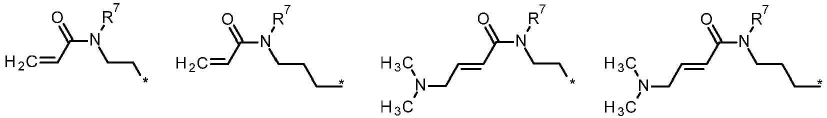 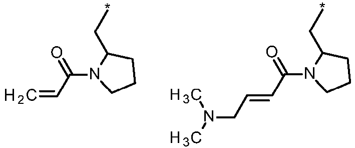 wherein * indicates the point of attachment of said group to the oxygen atom of the compound of formula (I);
- R⁷: represents hydrogen or methyl;
or an N-oxide, a salt or a tautomer of said compound, or a salt of said N-oxide or tautomer.

In a third aspect, the invention relates to compounds of formula (I) as described *supra,* wherein:
- R¹: represents methyl, methoxy, or difluoromethoxy;
- R²: represents hydrogen, fluoro, or chloro;
- R³: represents hydrogen;
- R⁴: represents hydrogen or methyl;
- R⁵: represents hydrogen or methyl, or
- R⁴ and R⁵,: together with the carbon atom to which they are attached, represent a cyclobutyl group;
- R⁶: represents a group selected from the group: 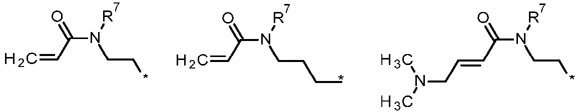 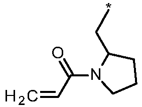 wherein * indicates the point of attachment of said group to the oxygen atom of the compound of formula (I);
- R⁷: represents hydrogen or methyl;
or an N-oxide, a salt or a tautomer of said compound, or a salt of said N-oxide or tautomer.

A further aspect of the invention relates to compounds of formula (I), which are present as their salts.

It is to be understood that the present invention relates to any sub-combination within any embodiment or aspect of the present invention of compounds of general formula (I), supra.

More particularly still, the present invention covers compounds of general formula (I) which are disclosed in the Example section of this text, infra.

In accordance with another aspect, the present invention covers methods of preparing compounds of the present invention, said methods comprising the steps as described in the Experimental Section herein.

Another embodiment of the invention are compounds according as disclosed in the Claims section or disclosed analogs of the exemplified compounds and subcombinations thereof.

### Definitions

The term "comprising" when used in the specification includes "consisting of".

If it is referred to "as mentioned above" or "mentioned above", *"supra"* within the description it is referred to any of the disclosures made within the specification in any of the preceding pages.

If it is referred to "as mentioned herein", "described herein", "provided herein," or "as mentioned in the present text," or "stated herein" within the description it is referred to any of the disclosures made within the specification in any of the preceding or subsequent pages.

"Suitable" within the sense of the invention means chemically possible to be made by methods within the knowledge of a skilled person.

The terms as mentioned in the present text may have the following meanings:
The term "C₃-C₆-cycloalkyl" means a saturated, monovalent, monocyclic hydrocarbon ring which contains 3, 4, 5, or 6 carbon atoms. Said C₃-C₆-cycloalkyl group is for example, a cyclopropyl, cyclobutyl, cyclopentyl, or cyclohexyl group.

Further, as used herein, the term "C₃-C₆", as used throughout this text, e.g. in the context of the definition of "C₃-C₆-cycloalkyl", is to be understood as meaning a cycloalkyl group having a finite number of carbon atoms of 3 to 6, *i.e.* 3, 4, 5 or 6 carbon atoms. It is to be understood further that said term "C₃-C₆" is to be interpreted as any sub-range comprised therein, e.g. C₃-C₆, C₄-C₅, C₃-C₅, C₃-C₄ , C₄-C₆, C₅-C₆; particularly C₃-C₆.

The term "substituted" means that one or more hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds.

As used herein, the term "one or more", e.g. in the definition of the substituents of the compounds of the general formulae of the present invention, is understood as meaning "one, two, three, four, five, etc. particularly one, two, three or four, more particularly one, two or three, even more particularly one or two".

The compounds of general formula (I) may exist as isotopic variants. The invention therefore includes one or more isotopic variant(s) of the compounds of general formula (I), particularly deuterium-containing compounds of general formula (I).

The term "isotopic variant" of a compound or a reagent is defined as a compound exhibiting an unnatural proportion of one or more of the isotopes that constitute such a compound.

The term "isotopic variant of the compound of general formula (I)" is defined as a compound of general formula (I) exhibiting an unnatural proportion of one or more of the isotopes that constitute such a compound.

The expression "unnatural proportion" is to be understood as meaning a proportion of such isotope which is higher than its natural abundance. The natural abundances of isotopes to be applied in this context are described in "Isotopic Compositions of the Elements 1997", Pure Appl. Chem., 70(1), 217-235, 1998. Examples of such isotopes include stable and radioactive isotopes of hydrogen, carbon, nitrogen, oxygen, phosphorus, sulfur, fluorine, chlorine, bromine and iodine, such as ²H (deuterium), ³H (tritium), ¹¹C, ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁵I, ¹²⁹I and ¹³¹I, respectively.

With respect to the treatment and/or prophylaxis of the disorders specified herein the isotopic variant(s) of the compounds of general formula (I) in one embodiment contain deuterium ("deuterium-containing compounds of general formula (I)"). Isotopic variants of the compounds of general formula (I) in which one or more radioactive isotopes, such as ³H or ¹⁴C, are incorporated are useful e.g. in drug and/or substrate tissue distribution studies. These isotopes are particularly preferred for the ease of their incorporation and detectability. Positron emitting isotopes such as ¹⁸F or ¹¹C may be incorporated into a compound of general formula (I). These isotopic variants of the compounds of general formula (I) are useful for *in vivo* imaging applications. Deuterium-containing and ¹³C-containing compounds of general formula (I) can be used in mass spectrometry analyses (H. J. Leis et al., Curr. Org. Chem., 1998, 2, 131) in the context of preclinical or clinical studies.

Isotopic variants of the compounds of general formula (I) can generally be prepared by methods known to a person skilled in the art, such as those described in the schemes and/or examples herein, by substituting a reagent for an isotopic variant of said reagent, in one embodiment for a deuterium-containing reagent. Depending on the desired sites of deuteration, in some cases deuterium from D₂O can be incorporated either directly into the compounds or into reagents that are useful for synthesizing such compounds (Esaki et al., Tetrahedron, 2006, 62, 10954; Esaki et al., Chem. Eur. J., 2007, 13, 4052). Deuterium gas is also a useful reagent for incorporating deuterium into molecules. Catalytic deuteration of olefinic bonds (H. J. Leis et al., Curr. Org. Chem., 1998, 2, 131; J. R. Morandi et al., J. Org. Chem., 1969, 34 (6), 1889) and acetylenic bonds (N. H. Khan, J. Am. Chem. Soc., 1952, 74 (12), 3018; S. Chandrasekhar et al., Tetrahedron, 2011, 52, 3865) is a rapid route for incorporation of deuterium. Metal catalysts (i.e. Pd, Pt, and Rh) in the presence of deuterium gas can be used to directly exchange deuterium for hydrogen in functional groups containing hydrocarbons (J. G. Atkinson et al., US Patent 3966781). A variety of deuterated reagents and synthetic building blocks are commercially available from companies such as for example C/D/N Isotopes, Quebec, Canada; Cambridge Isotope Laboratories Inc., Andover, MA, USA; and CombiPhos Catalysts, Inc., Princeton, NJ, USA. Further information on the state of the art with respect to deuterium-hydrogen exchange is given for example in Hanzlik et al., J. Org. Chem. 55, 3992-3997, 1990; R. P. Hanzlik et al., Biochem. Biophys. Res. Commun. 160, 844, 1989; P. J. Reider et al., J. Org. Chem. 52, 3326-3334, 1987; M. Jarman et al., Carcinogenesis 16(4), 683-688, 1993; J. Atzrodt et al., Angew. Chem., Int. Ed. 2007, 46, 7744; K. Matoishi et al., J. Chem. Soc, Chem. Commun. 2000, 1519-1520; K. Kassahun et al., WO2012/112363.

The term "deuterium-containing compound of general formula (I)" is defined as a compound of general formula (I), in which one or more hydrogen atom(s) is/are replaced by one or more deuterium atom(s) and in which the abundance of deuterium at each deuterated position of the compound of general formula (I) is higher than the natural abundance of deuterium, which is about 0.015%. Particularly, in a deuterium-containing compound of general formula (I) the abundance of deuterium at each deuterated position of the compound of general formula (I) is higher than 10%, 20%, 30%, 40%, 50%, 60%, 70% or 80%, in one embodiment higher than 90%, 95%, 96% or 97%, in other embodiments higher than 98% or 99% at said position(s). It is understood that the abundance of deuterium at each deuterated position is independent of the abundance of deuterium at other deuterated position(s).

The selective incorporation of one or more deuterium atom(s) into a compound of general formula (I) may alter the physicochemical properties (such as for example acidity [A. Streitwieser et al., J. Am. Chem. Soc., 1963, 85, 2759; C. L. Perrin, et al., J. Am. Chem. Soc., 2007, 129, 4490], basicity [C. L. Perrin, et al., J. Am. Chem. Soc., 2003, 125, 15008; C. L. Perrin in Advances in Physical Organic Chemistry, 44, 144; C. L. Perrin et al., J. Am. Chem. Soc., 2005, 127, 9641], lipophilicity [B. Testa et al., Int. J. Pharm., 1984, 19(3), 271]) and/or the metabolic profile of the molecule and may result in changes in the ratio of parent compound to metabolites or in the amounts of metabolites formed. Such changes may result in certain therapeutic advantages and hence may be preferred in some circumstances. Reduced rates of metabolism and metabolic switching, where the ratio of metabolites is changed, have been reported (D. J. Kushner et al., Can. J. Physiol. Pharmacol., 1999, 77, 79; A. E. Mutlib et al., Toxicol. Appl. Pharmacol., 2000, 169, 102). These changes in the exposure to parent drug and metabolites can have important consequences with respect to the pharmacodynamics, tolerability and efficacy of a deuterium-containing compound of general formula (I). In some cases deuterium substitution reduces or eliminates the formation of an undesired or toxic metabolite and enhances the formation of a desired metabolite (*e.g.* Nevirapine: A. M. Sharma et al., Chem. Res.Toxicol., 2013, 26, 410; Uetrecht et al., Chemical Research in Toxicology, 2008, 21, 9, 1862; Efavirenz: A. E. Mutlib et al., Toxicol. Appl. Pharmacol., 2000, 169, 102). In other cases the major effect of deuteration is to reduce the rate of systemic clearance. As a result, the biological half-life of the compound is increased. The potential clinical benefits would include the ability to maintain similar systemic exposure with decreased peak levels and increased trough levels. This could result in lower side effects and enhanced efficacy, depending on the particular compound's pharmacokinetic/ pharmacodynamic relationship. Indiplon (A. J. Morales et al., Abstract 285, The 15th North American Meeting of the International Society of Xenobiotics, San Diego, CA, October 12-16, 2008), ML-337 (C. J. Wenthur et al., J. Med. Chem., 2013, 56, 5208), and Odanacatib (K. Kassahun et al., WO2012/112363) are examples for this deuterium effect. Still other cases have been reported in which reduced rates of metabolism result in an increase in exposure of the drug without changing the rate of systemic clearance (*e.g.* Rofecoxib: F. Schneider et al., Arzneim. Forsch. Drug. Res., 2006, 56, 295; Telaprevir: F. Maltais et al., J. Med. Chem., 2009, 52, 7993). Deuterated drugs showing this effect may have reduced dosing requirements (e.g. lower number of doses or lower dosage to achieve the desired effect) and/or may produce lower metabolite loads.

A compound of general formula (I) may have multiple potential sites of attack for metabolism. To optimize the above-described effects on physicochemical properties and metabolic profile, deuterium-containing compounds of general formula (I) having a certain pattern of one or more deuterium-hydrogen exchange(s) can be selected. Particularly, the deuterium atom(s) of deuterium- containing compound(s) of general formula (I) is/are attached to a carbon atom and/or is/are located at those positions of the compound of general formula (I), which are sites of attack for metabolizing enzymes such as *e.g.* cytochrome P₄₅₀.

Where the plural form of the word compounds or salts is used herein, this is taken to mean also a single compound or salt.

By "stable compound' or "stable structure" is meant a compound that is sufficiently robust to survive isolation to a useful degree of purity from a reaction mixture, and formulation into an efficacious therapeutic agent.

The compounds of this invention may contain one or more asymmetric centre, depending upon the location and nature of the various substituents desired. Asymmetric carbon atoms may be present in the (R) or (S) configuration, resulting in racemic mixtures in the case of a single asymmetric centre, and diastereomeric mixtures in the case of multiple asymmetric centres. In certain instances, asymmetry may also be present due to restricted rotation about a given bond, for example, the central bond adjoining two substituted aromatic rings of the specified compounds.

Substituents on a ring may also be present in either cis or trans form. It is intended that all such configurations (including enantiomers and diastereomers), are included within the scope of the present invention.

Preferred compounds are those which produce the more desirable biological activity. Separated, pure or partially purified isomers and stereoisomers or racemic or diastereomeric mixtures of the compounds of this invention are also included within the scope of the present invention. The purification and the separation of such materials can be accomplished by standard techniques known in the art.

The optical isomers can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, ditoluoyltartaric and camphorsulfonic acid. Mixtures of diastereoisomers can be separated into their individual diastereomers on the basis of their physical and/or chemical differences by methods known in the art, for example, by chromatography or fractional crystallisation. The optically active bases or acids are then liberated from the separated diastereomeric salts. A different process for separation of optical isomers involves the use of chiral chromatography (*e.g.,* chiral HPLC columns), with or without conventional derivatisation, optimally chosen to maximise the separation of the enantiomers. Suitable chiral HPLC columns are manufactured by Daicel, *e.g.,* Chiracel OD and Chiracel OJ among many others, all routinely selectable. Enzymatic separations, with or without derivatisation, are also useful. The optically active compounds of this invention can likewise be obtained by chiral syntheses utilizing optically active starting materials.

In order to limit different types of isomers from each other reference is made to IUPAC Rules Section E (Pure Appl Chem 45, 11-30, 1976).

The present invention includes all possible stereoisomers of the compounds of the present invention as single stereoisomers, or as any mixture of said stereoisomers, *e.g.* R- or S- isomers, or E- or Z-isomers, in any ratio. Isolation of a single stereoisomer, e.g. a single enantiomer or a single diastereomer, of a compound of the present invention may be achieved by any suitable state of the art method, such as chromatography, especially chiral chromatography, for example.

Further, the compounds of the present invention may exist as tautomers. For example, any compound of the present invention which contains a pyrazole moiety as a heteroaryl group for example can exist as a 1H tautomer, or a 2H tautomer, or even a mixture in any amount of the two tautomers, or a triazole moiety for example can exist as a 1H tautomer, a 2H tautomer, or a 4H tautomer, or even a mixture in any amount of said 1H, 2H and 4H tautomers, namely :

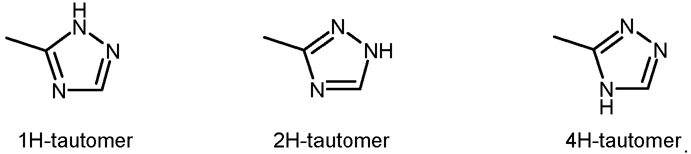

The present invention includes all possible tautomers of the compounds of the present invention as single tautomers, or as any mixture of said tautomers, in any ratio.

Further, the compounds of the present invention can exist as N-oxides, which are defined in that at least one nitrogen of the compounds of the present invention is oxidised. The present invention includes all such possible N-oxides.

Further, the compounds of the present invention can exist in free form, *e.g.* as a free base, or as a free acid, or as a zwitterion, or can exist in the form of a salt. Said salt may be any salt, either an organic or inorganic addition salt, particularly any pharmaceutically acceptable organic or inorganic addition salt, customarily used in pharmacy.

The term "pharmaceutically acceptable salt" refers to a relatively non-toxic, inorganic or organic acid addition salt of a compound of the present invention. For example, see S. M. Berge, et al. "Pharmaceutical Salts," J. Pharm. Sci. 1977, 66, 1-19.

A suitable pharmaceutically acceptable salt of the compounds of the present invention may be, for example, an acid-addition salt of a compound of the present invention bearing a nitrogen atom, in a chain or in a ring, for example, which is sufficiently basic, such as an acid-addition salt with an inorganic acid, such as hydrochloric, hydrobromic, hydroiodic, sulfuric, bisulfuric, phosphoric or nitric acid, for example, or with an organic acid, such as formic, acetic, acetoacetic, pyruvic, trifluoroacetic, propionic, butyric, hexanoic, heptanoic, undecanoic, lauric, benzoic, salicylic, 2-(4-hydroxybenzoyl)-benzoic, camphoric, cinnamic, cyclopentanepropionic, digluconic, 3-hydroxy-2-naphthoic, nicotinic, pamoic, pectinic, persulfuric, 3-phenylpropionic, picric, pivalic, 2-hydroxyethanesulfonate, itaconic, sulfamic, trifluoromethanesulfonic, dodecylsulfuric, ethansulfonic, benzenesulfonic, para-toluenesulfonic, methansulfonic, 2-naphthalenesulfonic, naphthalinedisulfonic, camphorsulfonic acid, citric, tartaric, stearic, lactic, oxalic, malonic, succinic, malic, adipic, alginic, maleic, fumaric, D-gluconic, mandelic, ascorbic, glucoheptanoic, glycerophosphoric, aspartic, sulfosalicylic, hemisulfuric or thiocyanic acid, for example.

Further, another suitably pharmaceutically acceptable salt of a compound of the present invention which is sufficiently acidic, is an alkali metal salt, for example a sodium or potassium salt, an alkaline earth metal salt, for example a calcium or magnesium salt, an ammonium salt or a salt with an organic base which affords a physiologically acceptable cation, for example a salt with N-methyl-glucamine, dimethyl-glucamine, ethyl-glucamine, lysine, dicyclohexylamine, 1,6-hexadiamine, ethanolamine, glucosamine, sarcosine, serinol, tris-hydroxy-methyl-aminomethane, aminopropandiol, sovak-base, 1-amino-2,3,4-butantriol. Additionally, basic nitrogen containing groups may be quaternised with such agents as lower alkyl halides such as methyl, ethyl, propyl, and butyl chlorides, bromides and iodides; dialkyl sulfates like dimethyl, diethyl, and dibutyl sulfate; and diamyl sulfates, long chain halides such as decyl, lauryl, myristyl and strearyl chlorides, bromides and iodides, aralkyl halides like benzyl and phenethyl bromides and others.

Those skilled in the art will further recognise that acid addition salts of the claimed compounds may be prepared by reaction of the compounds with the appropriate inorganic or organic acid via any of a number of known methods. Alternatively, alkali and alkaline earth metal salts of acidic compounds of the invention are prepared by reacting the compounds of the invention with the appropriate base via a variety of known methods.

The present invention includes all possible salts of the compounds of the present invention as single salts, or as any mixture of said salts, in any ratio.

In the present text, in particular in the Experimental Section, for the synthesis of intermediates and of examples of the present invention, when a compound is mentioned as a salt form with the corresponding base or acid, the exact stoichiometric composition of said salt form, as obtained by the respective preparation and/or purification process, is, in most cases, unknown.

Unless specified otherwise, suffixes to chemical names or structural formulae such as "hydrochloride", "trifluoroacetate", "sodium salt", or "x HCl", "x CF₃COOH", "x Na⁺", for example, are to be understood as not a stoichiometric specification, but solely as a salt form.

This applies analogously to cases in which synthesis intermediates or example compounds or salts thereof have been obtained, by the preparation and/or purification processes described, as solvates, such as hydrates with (if defined) unknown stoichiometric composition.

The salts include water-insoluble and, particularly, water-soluble salts.

Furthermore, the present invention includes all possible crystalline forms, or polymorphs, of the compounds of the present invention, either as single polymorphs, or as a mixture of more than one polymorphs, in any ratio.

In the context of the properties of the compounds of the present invention the term "pharmacokinetic profile" means one single parameter or a combination thereof including permeability, bioavailability, exposure, and pharmacodynamic parameters such as duration, or magnitude of pharmacological effect, as measured in a suitable experiment. Compounds with improved pharmacokinetic profiles can, for example, be used in lower doses to achieve the same effect, may achieve a longer duration of action, or a may achieve a combination of both effects.

The term "combination" in the present invention is used as known to persons skilled in the art and may be present as a fixed combination, a non-fixed combination or kit-of-parts.

A "fixed combination" in the present invention is used as known to persons skilled in the art and is defined as a combination wherein the said first active ingredient and the said second active ingredient are present together in one unit dosage or in a single entity. One example of a "fixed combination" is a pharmaceutical composition wherein the said first active ingredient and the said second active ingredient are present in admixture for simultaneous administration, such as in a formulation. Another example of a "fixed combination" is a pharmaceutical combination wherein the said first active ingredient and the said second active ingredient are present in one unit without being in admixture.

A non-fixed combination or "kit-of-parts" in the present invention is used as known to persons skilled in the art and is defined as a combination wherein the said first active ingredient and the said second active ingredient are present in more than one unit. One example of a non-fixed combination or kit-of-parts is a combination wherein the said first active ingredient and the said second active ingredient are present separately. The components of the non-fixed combination or kit-of-parts may be administered separately, sequentially, simultaneously, concurrently or chronologically staggered. Any such combination of a compound of formula (I) of the present invention with an anti-cancer agent as defined below is an embodiment of the invention.

The term "(chemotherapeutic) anti-cancer agents" relates to any agent that reduces the survival or proliferation of a cancer cell, and includes but is not limited to
131I-chTNT, abarelix, abiraterone, aclarubicin, ado-trastuzumab emtansine, afatinib, aflibercept, aldesleukin, alemtuzumab, Alendronic acid, alitretinoin, altretamine, amifostine, aminoglutethimide, Hexyl aminolevulinate, amrubicin, amsacrine, anastrozole, ancestim, anethole dithiolethione, angiotensin II, antithrombin III, aprepitant, arcitumomab, arglabin, arsenic trioxide, asparaginase, axitinib, azacitidine, basiliximab, belotecan, bendamustine, belinostat, bevacizumab, bexarotene, bicalutamide, bisantrene, bleomycin, bortezomib, buserelin, bosutinib, brentuximab vedotin, busulfan, cabazitaxel, cabozantinib, calcium folinate, calcium levofolinate, capecitabine, capromab, carboplatin, carfilzomib, carmofur, carmustine, catumaxomab, celecoxib, celmoleukin, ceritinib, cetuximab, chlorambucil, chlormadinone, chlormethine, cidofovir, cinacalcet, cisplatin, cladribine, clodronic acid, clofarabine, copanlisib, crisantaspase, cyclophosphamide, cyproterone, cytarabine, dacarbazine, dactinomycin, darbepoetin alfa, dabrafenib, dasatinib, daunorubicin, decitabine, degarelix, denileukin diftitox, denosumab, depreotide, deslorelin, dexrazoxane, dibrospidium chloride, dianhydrogalactitol, diclofenac, docetaxel, dolasetron, doxifluridine, doxorubicin, doxorubicin + estrone, dronabinol, eculizumab, edrecolomab, elliptinium acetate, eltrombopag, endostatin, enocitabine, enzalutamide, epirubicin, epitiostanol, epoetin alfa, epoetin beta, epoetin zeta, eptaplatin, eribulin, erlotinib, esomeprazole, estradiol, estramustine, etoposide, everolimus, exemestane, fadrozole, fentanyl, filgrastim, fluoxymesterone, floxuridine, fludarabine, fluorouracil, flutamide, folinic acid, formestane, fosaprepitant, fotemustine, fulvestrant, gadobutrol, gadoteridol, gadoteric acid meglumine, gadoversetamide, gadoxetic acid, gallium nitrate, ganirelix, gefitinib, gemcitabine, gemtuzumab, Glucarpidase, glutoxim, GM-CSF, goserelin, granisetron, granulocyte colony stimulating factor, histamine dihydrochloride, histrelin, hydroxycarbamide, I-125 seeds, lansoprazole, ibandronic acid, ibritumomab tiuxetan, ibrutinib, idarubicin, ifosfamide, imatinib, imiquimod, improsulfan, indisetron, incadronic acid, ingenol mebutate, interferon alfa, interferon beta, interferon gamma, iobitridol, iobenguane (123I), iomeprol, ipilimumab, irinotecan, Itraconazole, ixabepilone, lanreotide, lapatinib, lasocholine, lenalidomide, lenograstim, lentinan, letrozole, leuprorelin, levamisole, levonorgestrel, levothyroxine sodium, lisuride, lobaplatin, lomustine, lonidamine, masoprocol, medroxyprogesterone, megestrol, melarsoprol, melphalan, mepitiostane, mercaptopurine, mesna, methadone, methotrexate, methoxsalen, methylaminolevulinate, methylprednisolone, methyltestosterone, metirosine, mifamurtide, miltefosine, miriplatin, mitobronitol, mitoguazone, mitolactol, mitomycin, mitotane, mitoxantrone, mogamulizumab, molgramostim, mopidamol, morphine hydrochloride, morphine sulfate, nabilone, nabiximols, nafarelin, naloxone + pentazocine, naltrexone, nartograstim, nedaplatin, nelarabine, neridronic acid, nivolumabpentetreotide, nilotinib, nilutamide, nimorazole, nimotuzumab, nimustine, nitracrine, nivolumab, obinutuzumab, octreotide, ofatumumab, omacetaxine mepesuccinate, omeprazole, ondansetron, oprelvekin, orgotein, orilotimod, osimertinib, oxaliplatin, oxycodone, oxymetholone, ozogamicine, p53 gene therapy, paclitaxel, palifermin, palladium-103 seed, palonosetron, pamidronic acid, panitumumab, pantoprazole, pazopanib, pegaspargase, PEG-epoetin beta (methoxy PEG-epoetin beta), pembrolizumab, pegfilgrastim, peginterferon alfa-2b, pemetrexed, pentazocine, pentostatin, peplomycin, Perflubutane, perfosfamide, Pertuzumab, picibanil, pilocarpine, pirarubicin, pixantrone, plerixafor, plicamycin, poliglusam, polyestradiol phosphate, polyvinylpyrrolidone + sodium hyaluronate, polysaccharide-K, pomalidomide, ponatinib, porfimer sodium, poziotinib, pralatrexate, prednimustine, prednisone, procarbazine, procodazole, propranolol, quinagolide, rabeprazole, racotumomab, radium-223 chloride, radotinib, raloxifene, raltitrexed, ramosetron, ramucirumab, ranimustine, rasburicase, razoxane, refametinib, regorafenib, risedronic acid, rhenium-186 etidronate, rituximab, romidepsin, romiplostim, romurtide, roniciclib, samarium (153Sm) lexidronam, sargramostim, satumomab, secretin, sipuleucel-T, sizofiran, sobuzoxane, sodium glycididazole, sorafenib, stanozolol, streptozocin, sunitinib, talaporfin, tamibarotene, tamoxifen, tapentadol, tasonermin, teceleukin, technetium (99mTc) nofetumomab merpentan, 99mTc-HYNIC-[Tyr3]-octreotide, tegafur, tegafur + gimeracil + oteracil, temoporfin, temozolomide, temsirolimus, teniposide, testosterone, tetrofosmin, thalidomide, thiotepa, thymalfasin, thyrotropin alfa, tioguanine, tocilizumab, topotecan, toremifene, tositumomab, trabectedin, tramadol, trastuzumab, trastuzumab emtansine, treosulfan, tretinoin, trifluridine + tipiracil, trilostane, triptorelin, trametinib, trofosfamide, thrombopoietin, tryptophan, ubenimex, valatinib, valrubicin, vandetanib, vapreotide, vemurafenib, vinblastine, vincristine, vindesine, vinflunine, vinorelbine, vismodegib, vorinostat, vorozole, yttrium-90 glass microspheres, zinostatin, zinostatin stimalamer, zoledronic acid, zorubicin.

By "Epidermal Growth Factor Receptor (EGFR) Polypeptide" is meant a polypeptide having at least about 95% amino acid sequence identity to the sequence provided at UniProt Accession No. P00533-1 or a fragment thereof. In some embodiments, the EGFR fragment binds an EFGR ligand and/or has kinase activity. Mutant EGFR polypeptides include those having an insertion between, for example, amino acids V769 and D770 or between D770 and N771. In other embodiments, the amino acid sequence identity is 96, 97, 98, 99, or 100% to UniProt Accession No. P00533-1.

An exemplary full length sequence of human EGFR, which indicates V769, D770, and N771 in bold, is provided at UniProt Accession No. P00533-1, which is reproduced below:

10 20 30 40 50

60 70 80 90 100

110 120 130 140 150

160 170 180 190 200

210 220 230 240 250

260 270 280 290 300

310 320 330 340 350


360 370 380 390 400

410 420 430 440 450

460 470 480 490 500

510 520 530 540 550

560 570 580 590 600

610 620 630 640 650

660 670 680 690 700

710 720 730 740 750

760 770 780 790 800

810 820 830 840 850

860 870 880 890 900

910 920 930 940 950

960 970 980 990 1000

1010 1020 1030 1040 1050

1060 1070 1080 1090 1100

1110 1120 1130 1140 1150

1160 1170 1180 1190 1200


1210


An exemplary polynucleotide encoding EGFR is provided at NCBI Reference Sequence: NM_001346897.1, which is reproduced below:

1 gtccgggcag cccccggcgc agcgcggccg cagcagcctc cgccccccgc acggtgtgag
61 cgcccgacgc ggccgaggcg gccggagtcc cgagctagcc ccggcggccg ccgccgccca
121 gaccggacga caggccacct cgtcggcgtc cgcccgagtc cccgcctcgc cgccaacgcc
181 acaaccaccg cgcacggccc cctgactccg tccagtattg atcgggagag ccggagcgag
241 ctcttcgggg agcagcgatg cgaccctccg ggacggccgg ggcagcgctc ctggcgctgc
301 tggctgcgct ctgcccggcg agtcgggctc tggaggaaaa gaaagtttgc caaggcacga
361 gtaacaagct cacgcagttg ggcacttttg aagatcattt tctcagcctc cagaggatgt
421 tcaataactg tgaggtggtc cttgggaatt tggaaattac ctatgtgcag aggaattatg
481 atctttcctt cttaaagacc atccaggagg tggctggtta tgtcctcatt gccctcaaca
541 cagtggagcg aattcctttg gaaaacctgc agatcatcag aggaaatatg tactacgaaa
601 attcctatgc cttagcagtc ttatctaact atgatgcaaa taaaaccgga ctgaaggagc
661 tgcccatgag aaatttacag ggccaaaagt gtgatccaag ctgtcccaat gggagctgct
721 ggggtgcagg agaggagaac tgccagaaac tgaccaaaat catctgtgcc cagcagtgct
781 ccgggcgctg ccgtggcaag tcccccagtg actgctgcca caaccagtgt gctgcaggct
841 gcacaggccc ccgggagagc gactgcctgg tctgccgcaa attccgagac gaagccacgt
901 gcaaggacac ctgcccccca ctcatgctct acaaccccac cacgtaccag atggatgtga
961 accccgaggg caaatacagc tttggtgcca cctgcgtgaa gaagtgtccc cgtaattatg
1021 tggtgacaga tcacggctcg tgcgtccgag cctgtggggc cgacagctat gagatggagg
1081 aagacggcgt ccgcaagtgt aagaagtgcg aagggccttg ccgcaaagtg tgtaacggaa
1141 taggtattgg tgaatttaaa gactcactct ccataaatgc tacgaatatt aaacacttca
1201 aaaactgcac ctccatcagt ggcgatctcc acatcctgcc ggtggcattt aggggtgact
1261 ccttcacaca tactcctcct ctggatccac aggaactgga tattctgaaa accgtaaagg
1321 aaatcacagg gtttttgctg attcaggctt ggcctgaaaa caggacggac ctccatgcct
1381 ttgagaacct agaaatcata cgcggcagga ccaagcaaca tggtcagttt tctcttgcag
1441 tcgtcagcct gaacataaca tccttgggat tacgctccct caaggagata agtgatggag
1501 atgtgataat ttcaggaaac aaaaatttgt gctatgcaaa tacaataaac tggaaaaaac
1561 tgtttgggac ctccggtcag aaaaccaaaa ttataagcaa cagaggtgaa aacagctgca
1621 aggccacagg ccaggtctgc catgccttgt gctcccccga gggctgctgg ggcccggagc
1681 ccagggactg cgtctcttgc cggaatgtca gccgaggcag ggaatgcgtg gacaagtgca
1741 accttctgga gggtgagcca agggagtttg tggagaactc tgagtgcata cagtgccacc

1801 cagagtgcct gcctcaggcc atgaacatca cctgcacagg acggggacca gacaactgta
1861 tccagtgtgc ccactacatt gacggccccc actgcgtcaa gacctgcccg gcaggagtca
1921 tgggagaaaa caacaccctg gtctggaagt acgcagacgc cggccatgtg tgccacctgt
1981 gccatccaaa ctgcacctac ggatgcactg ggccaggtct tgaaggctgt ccaacgaatg
2041 ggcctaagat cccgtccatc gccactggga tggtgggggc cctcctcttg ctgctggtgg
2101 tggccctggg gatcggcctc ttcatgcgaa ggcgccacat cgttcggaag cgcacgctgc
2161 ggaggctgct gcaggagagg gagcttgtgg agcctcttac acccagtgga gaagctccca
2221 accaagctct cttgaggatc ttgaaggaaa ctgaattcaa aaagatcaaa gtgctgggct
2281 ccggtgcgtt cggcacggtg tataagggac tctggatccc agaaggtgag aaagttaaaa
2341 ttcccgtcgc tatcaaggaa ttaagagaag caacatctcc gaaagccaac aaggaaatcc
2401 tcgatgaagc ctacgtgatg gccagcgtgg acaaccccca cgtgtgccgc ctgctgggca
2461 tctgcctcac ctccaccgtg cagctcatca cgcagctcat gcccttcggc tgcctcctgg
2521 actatgtccg ggaacacaaa gacaatattg gctcccagta cctgctcaac tggtgtgtgc
2581 agatcgcaaa gggcatgaac tacttggagg accgtcgctt ggtgcaccgc gacctggcag
2641 ccaggaacgt actggtgaaa acaccgcagc atgtcaagat cacagatttt gggctggcca
2701 aactgctggg tgcggaagag aaagaatacc atgcagaagg aggcaaagtg cctatcaagt
2761 ggatggcatt ggaatcaatt ttacacagaa tctataccca ccagagtgat gtctggagct
2821 acggggtgac tgtttgggag ttgatgacct ttggatccaa gccatatgac ggaatccctg
2881 ccagcgagat ctcctccatc ctggagaaag gagaacgcct ccctcagcca cccatatgta
2941 ccatcgatgt ctacatgatc atggtcaagt gctggatgat agacgcagat agtcgcccaa
3001 agttccgtga gttgatcatc gaattctcca aaatggcccg agacccccag cgctaccttg
3061 tcattcaggg ggatgaaaga atgcatttgc caagtcctac agactccaac ttctaccgtg
3121 ccctgatgga tgaagaagac atggacgacg tggtggatgc cgacgagtac ctcatcccac
3181 agcagggctt cttcagcagc ccctccacgt cacggactcc cctcctgagc tctctgagtg
3241 caaccagcaa caattccacc gtggcttgca ttgatagaaa tgggctgcaa agctgtccca
3301 tcaaggaaga cagcttcttg cagcgataca gctcagaccc cacaggcgcc ttgactgagg
3361 acagcataga cgacaccttc ctcccagtgc ctggtgagtg gcttgtctgg aaacagtcct
3421 gctcctcaac ctcctcgacc cactcagcag cagccagtct ccagtgtcca agccaggtgc
3481 tccctccagc atctccagag ggggaaacag tggcagattt gcagacacag tgaagggcgt
3541 aaggagcaga taaacacatg accgagcctg cacaagctct ttgttgtgtc tggttgtttg
3601 ctgtacctct gttgtaagaa tgaatctgca aaatttctag cttatgaagc aaatcacgga
3661 catacacatc tgtgtgtgtg agtgttcatg atgtgtgtac atctgtgtat gtgtgtgtgt
3721 gtatgtgtgt gtttgtgaca gatttgatcc ctgttctctc tgctggctct atcttgacct
3781 gtgaaacgta tatttaacta attaaatatt agttaatatt aataaatttt aagctttatc
3841 cagaaaaaaa aaaaaaaaa

The intermediates used for the synthesis of the compounds of claims 1-4 as described below, as well as their use for the synthesis of the compounds of claims 1-4, are one further aspect of the present invention. Preferred intermediates are the Intermediate Examples as disclosed below.

### General Procedures

The compounds according to the invention can be prepared according to the following schemes 1 - 5.

The schemes and procedures described below illustrate synthetic routes to the compounds of general formula (I) of the invention and are not intended to be limiting. It is obvious to the person skilled in the art that the order of transformations as exemplified in the schemes can be modified in various ways. The order of transformations exemplified in the schemes is therefore not intended to be limiting. In addition, interconversion of any of the substituents R¹, R², R³, R⁴, R⁵, R⁶, R⁷, R⁸, R⁹, and PG can be achieved before and/or after the exemplified transformations. These modifications can be such as the introduction of protecting groups, cleavage of protecting groups, reduction or oxidation of functional groups, halogenation, metallation, substitution or other reactions known to the person skilled in the art. These transformations include those which introduce a functionality which allows for further interconversion of substituents. Appropriate protecting groups and their introduction and cleavage are well-known to the person skilled in the art. Specific examples are described in the subsequent paragraphs.

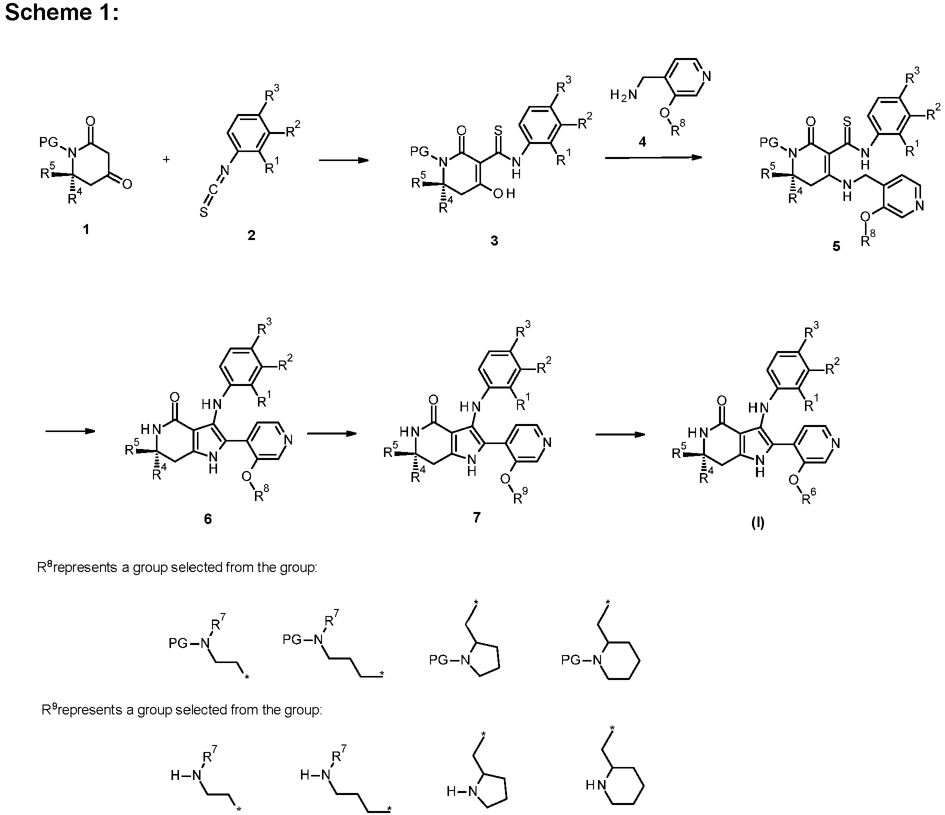

*Scheme 1:* Route for the preparation of compounds of general formula (I), wherein R¹, R², R³, R⁴, R⁵, R⁶ and R⁷ have the meaning as given for general formula (I), PG can be hydrogen or optionally a suitable protecting group, *e.g.* tert-butoxycarbonyl (Boc).

Compounds of formula **1, 2,** and **4** are either commercially available or can be prepared according to procedures available from the public domain, as understandable to the person skilled in the art. Specific examples are described in the subsequent paragraphs.

A suitably substituted piperidine-2,4-dione (Compound of formula **1),** such as, for example, piperidine-2,4-dione, can be reacted with a suitably substituted isothiocyanate (Compound of formula **2),** such as, for example, 3-fluorophenylisothiocyanate, in a suitable solvent system, such as, for example, acetonitrile, in the presence of a suitable base, such as, for example, triethylamine or DBU, at temperatures ranging from -78°C to +100°C, in some embodiments the reaction is carried out at 0°C or +100°C, to furnish a compound of general formula (3). Similar reactions have been performed in the literature.

Intermediates of general formula **(3)** can be converted to intermediates of general formula **(5)** by reaction with a suitable amine (compounds of general formula **4),** such as, for example 4-(aminomethyl)pyridine, in a suitable solvent system, such as, for example, ethanol and ethyl acetate, at a temperature between room temperature and the boiling point of the respective solvents, in some embodiments the reaction is carried out at the boiling point of the respective solvents, whereby the water formed in the reaction is removed from the reaction by methods known to those skilled in the art, such as, for example, azeotropic removal of water (Dean-Stark conditions) or with molecular sieves, to furnish a compound of general formula **(5).**

Intermediates of general formula **(3)** and intermediates of general formula **(5)** in which PG represents a protecting group can be converted to intermediates in which PG represents a hydrogen atom using standard deprotection conditions known to those skilled in the art. When PG is a protecting group such as, for example, tert-butoxycarbonyl (Boc), the deprotection can be carried out using acids, such as, for example, hydrochloric acid and trifluoroacetic acid, in a suitable solvent system, such as, for example, dichloromethane and dioxane, at a temperature between 0°C and the boiling point of the respective solvents, in one embodiment the reaction is carried out at room temperature, to furnish compounds of general formula **(3)** and intermediates of general formula **(5)** whereby PG is a hydrogen atom.

Intermediates of general formula **(5)** are reacted with a base and/or oxidizing reagent, in one embodiment an oxidizing agent, such as, for example hydrogen peroxide or SIBX (stabilized iodoxybenoic acid), in a suitable solvent system, such as, for example, methanol, in a temperature range from -30°C to the boiling point of the respective solvent, in one embodiment the reaction is carried out at the boiling point of the respective solvent, to furnish compounds of general formula **(6).** Optionally, these reactions can be carried out with an additive, such as, for example, an acid or base, such as, for example, acetic acid or trifluoroacetic acid (not-limiting), and triethylamine or diispropylethylamine (not-limiting).

Intermediates of general formula **(5)** can be converted to intermediates of general formula **(6)** by thermal heating in a suitable solvent at elevated temperatures, which can be above the boiling point of the solvent, such as, for example, RT to +250°C. These reactions can optionally be carried out in a vessel, whereby the pressure can be increased, such as, for example, in an autoclave. Intermediates of general formula **(5)** can also be converted to compounds of general formula **(6)** by thermal heating in the presence of a metal catalyst, such as, for example, palladium on activated charcoal, in a suitable solvent, such as, for example, DMF, DMA, ethanol, methanol, NMP (not-limiting) at elevated temperatures, such as, for example, RT to +150°C. Optionally, these reactions can be carried out with an additive, such as, for example, an acid or a base, such as, for example, acetic acid or trifluoroacetic acid (not-limiting), and triethylamine or diispropylethylamine (not-limiting), to furnish compounds of general formula **(6).** Intermediates of general formula **(6)** can be converted to intermediates of general formula **(7)** using conditions suitable for removing the group PG. When PG is a protecting group such as, for example, tert-butoxycarbonyl (Boc), the deprotection can be carried out using acids, such as, for example, hydrochloric acid and trifluoroacetic acid, in a suitable solvent system, such as, for example, dichloromethane and dioxane, at a temperature between 0°C and the boiling point of the respective solvents to furnish compounds of general formula **(7).** Intermediates of general formula **(7)** can be converted to compounds of general formula **(I)** by using suitable conditions for amide bond formation such as coupling reagents e.g. propanephosphonic acid anhydride or HATU (1-[Bis(dimethylamino)methylene]-1H-1,2,3-triazolo[4,5-b]pyridinium 3-oxide hexafluorophosphate).

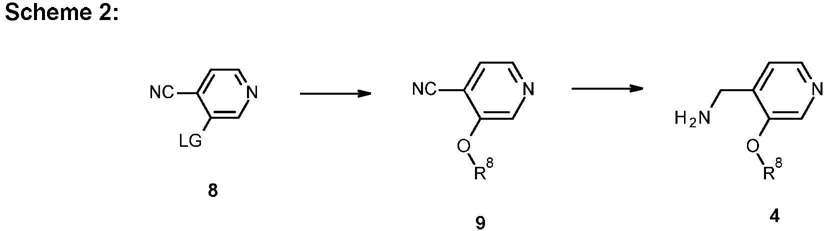

*Scheme 2:* Process for the preparation of compounds of general formula **(4),** wherein R⁸ has the meaning as shown in scheme 1.

Compounds of general formula **(8)** can be converted to compounds of general formula **(9)** by treatment with a suitable nucleophile, such as for example, amines, alcohols, metal alkoxides, azides, thiols or metal thiolates, under either basic, neutral, acidic, or catalytic conditions, in one embodiment basic conditions, in a suitable solvent or using the nucleophile as solvent, such as, for example, DMF, tetrahydrofuran (THF), in a temperature range from -78°C to the boiling point of the respective solvent, in one embodiment the reaction is carried out in a temperature range from -10°C to the boiling point of the respective solvent, to furnish a compound of general formula **(9).** Such substitution reactions have been previously reported.

Compounds of general formula **(9)** can be converted to compounds of general formula **(4)** by reducing reactions known to those skilled in the art, using numerous different reagents and reaction conditions; such methods and reagents can be carried out with metal hydrides, such as, for example, lithium aluminum hydride in THF, or using zinc in acetic acid, or using diborane, or using catalytic hydrogenation methods, for example, hydrogen and palladium on carbon under acidic conditions.

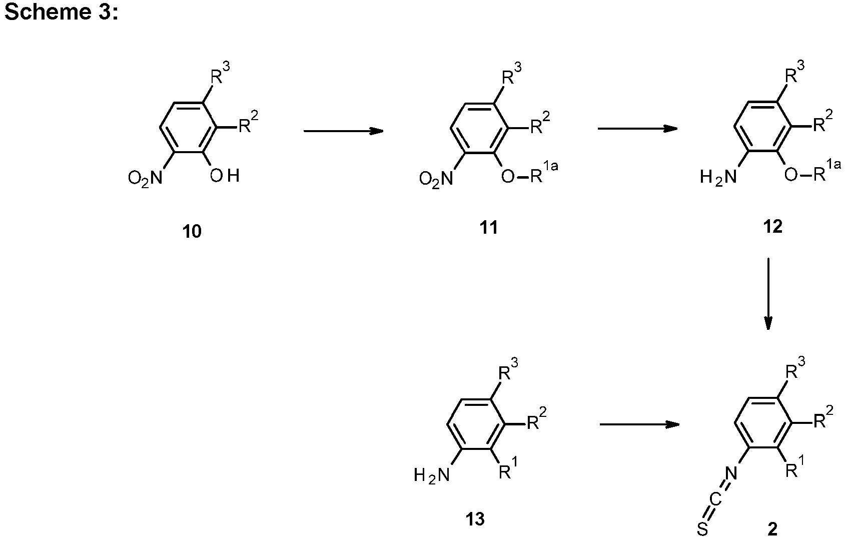

*Scheme 3:* Process for the preparation of compounds of general formula **2,** wherein R² and R³ have the meaning as given for general formula (I), and R^{1a} represents methyl or difluoromethyl corresponding to R¹ in general formula **(I)** with the meaning of methoxy and difluoromethoxy. The synthesis of compounds **11** and **12** relates to alkoxy substitution of the phenyl ring. However, the isothiocyanate containing product **2** and the synthesis thereof (*i.e.,* **10 → 2** or **13 → 2)** is general to R¹ groups according to general formula (**I**).

Compounds of general formula **(10)** can be converted to compounds of general formula **(11),** using various methods which are known to those skilled in the art. Such transformations can be, for example, to alkylate the phenolic alcohol with alkylating reagents, such as, for example, alkyl halides, alkyl sulfonates, in which these alkyl groups can optionally contain fluorides or alkoxy groups. These alkylation reactions are known to those skilled in the art using a variety of methods: i) K₂CO₃ in a solvent such as, DMF, acetone, DMFA; ii) KOH in ethanol; iii) Mitsunobu reaction to furnish intermediates of general formula **(11).**

Compounds of general formula **(11)** can be converted to compounds of general formula **(12)** by reduction methods and these methods are known to those skilled in the art. These reductions can be carried using: i) hydrogen gas and a catalyst (Pd/C, platinum, or Raney-Nickel); ii) iron and ammonium chloride; iii) sodium dithionite; iv) zinc and ammonium chloride to furnish intermediates of general formula **(12).**

Compounds of general formula **(12)** can be converted to compounds of general formula **(2)** by using reagents such as, for example, thiophosgene, carbon disulphide, 1,1"-thiocarbonyldi-2(1H)-pyridone or 1,1'-thiocarbonyldiimidazole, in one embodiment thiophosgene, under basic conditions, in a suitable solvent, such as, for example, dichloromethane, chloroform, acetone, or biphasic mixtures, such as, for example, dichloromethane, chloroform with aqueous basic solutions, in another embodiment, dichloromethane with an aqueous saturated solution of sodium hydrogen carbonate or sodium carbonate, in a temperature range from -78°C to the boiling point of the respective solvent, in another embodiment the reaction is carried out from 0°C to room temperature, to furnish compounds of general formula **(2).** Such transformations reactions have been previously reported.

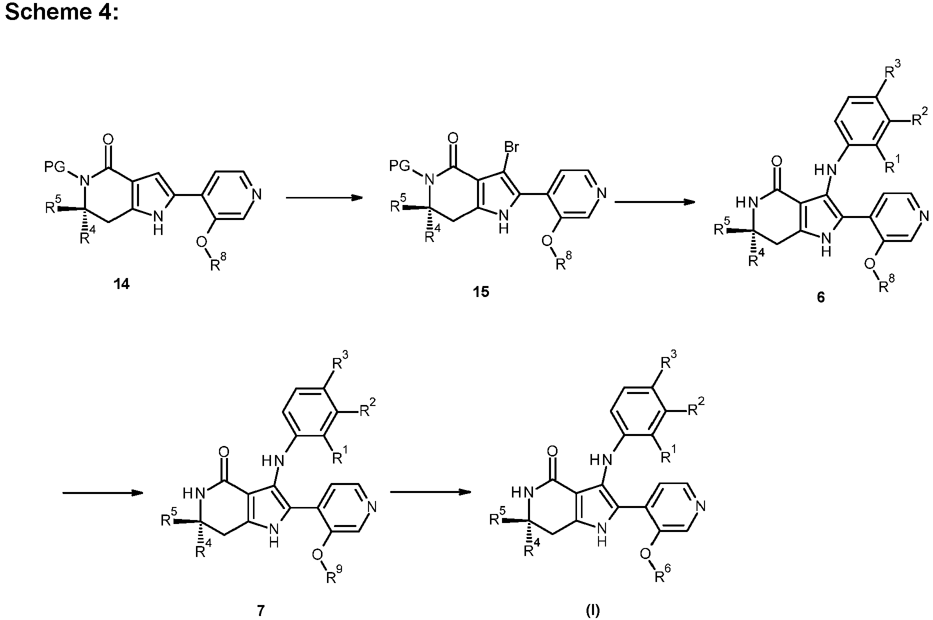

*Scheme 4:* Route for the preparation of compounds of general formula (I), wherein R¹, R², R³, R⁴, R⁵, and R⁶ have the meaning as given for general formula (I) and PG represents hydrogen or a suitable protecting group, *e.g.* tert-butoxycarbonyl (Boc). R⁸ and R⁹ have the meaning as shown in scheme 1.

Compounds similar to those of general formula **14** are known to those skilled in the art and their syntheses have been reported in the literature.

Compounds of general formula **(14)** can be converted to compounds of general formula **(15)** using standard bromination methods which are known to those skilled in the art. Such brominations can be carried out using a brominating agent, such as, for example, N-bromosuccinimide, in a suitable solvent, such as, for example, DMF, in a temperature range from -78°C to the boiling point of said solvent, in one embodiment the temperature range is from 0°C to RT.

Intermediates of general formula **(15)** can be reacted with suitable anilines in the presence of a base, such as, for example, lithium bis(trimethylsilyl)amide (LHMDS), in the presence of a catalyst, such as, for example 2-(di-tert-butylphosphino)-2',4',6'- triisopropyl-3,6-dimethoxy-1,1'-biphenyl (tBuBrettPhos) and a pre-catalyst, such as, for example a palladium pre-catalyst, chloro[2-(dicyclohexylphosphino)-3,6-dimethoxy-2',4',6'-triisopropyl-1,1'-biphenyl][2-(2-aminoethyl)phenyl]palladium(II) (BrettPhos-PreCat MTBE ether adduct) in a suitable solvent system, such as, for example, tetrahydrofuran (THF), at a temperature range from 0°C to 200°C. In one embodiment, the reaction is carried out at 80°C, to furnish compounds of general formula **(6).** Similar transformations have been carried out and have been reported. Intermediates of formula **(6)** can be converted to compounds of general formula **(I)** according to the strategy shown in scheme 1.

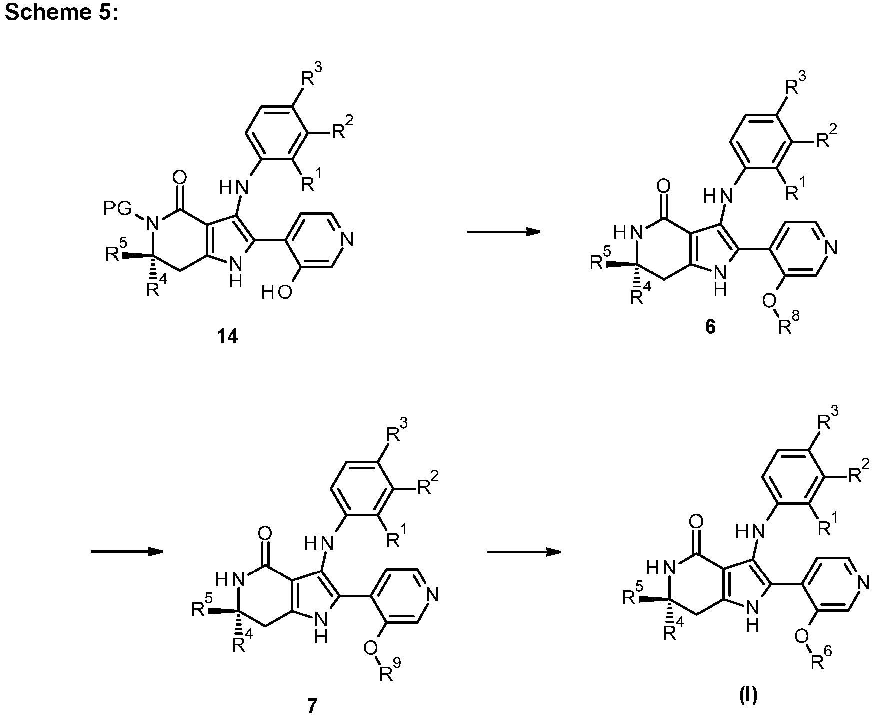

*Scheme 5:* Route for the preparation of compounds of general formula (I), wherein R¹, R², R³, R⁴, R⁵, and R⁶ have the meaning as given for general formula (I) and PG represents hydrogen or a suitable protecting group, *e.g.* tert-butoxycarbonyl (Boc). R⁸ and R⁹ have the meaning as shown in scheme 1.

Compounds similar to those of general formula **(14)** can be prepared according to the procedure described by Scheme 1 under the use of 4-(aminomethyl)-3-hydroxypyridine instead of intermediate **(4).** Intermediates of general formula **(14)** can be converted to compounds of general formula **(I)** by reaction with a suitable alcohol under Mitsunobu conditions such as, for example oxetan-3-ylmethanol, in the presence of (tributylphosphoranylidene)acetonitrile or triphenylphosphin together with diisopropyl azodicarboxylate in a suitable solvent system, such as, for example, dioxane or THF, at a temperature between room temperature and the boiling point of the respective solvents. Intermediates of formula **(6)** can then be converted to compounds of general formula **(I)** according to the strategy shown in scheme 1.

It is known to the person skilled in the art that, if there are a number of reactive centers on a starting or intermediate compound, it may be necessary to block one or more reactive centers temporarily by protective groups in order to allow a reaction to proceed specifically at the desired reaction center.

The compounds according to the invention are isolated and purified in a manner known per se, *e.g.* by distilling off the solvent *in vacuo* and recrystallizing the residue obtained from a suitable solvent or subjecting it to one of the customary purification methods, such as chromatography on a suitable support material. Furthermore, reverse phase preparative HPLC may be applied. The compounds of the present invention which possess a sufficiently basic or acidic functionality, may result as a salt, such as, in the case of a compound of the present invention which is sufficiently basic, a trifluoroacetate or formate salt for example, or, in the case of a compound of the present invention which is sufficiently acidic, an ammonium salt for example. Salts of this type can either be transformed into its free base or free acid form, respectively, by various methods known to the person skilled in the art, or be used as salts in subsequent biological assays. Additionally, the drying process during the isolation of the compounds of the present invention may not fully remove traces of cosolvents, especially such as formic acid or trifluoroacetic acid, to give solvates or inclusion complexes. The person skilled in the art will recognise which solvates or inclusion complexes are acceptable to be used in subsequent biological assays. It is to be understood that the specific form (*e.g.* salt, free base, free acid, solvate, inclusion complex) of a compound of the present invention as isolated and described herein is not necessarily the only form in which said compound can be applied to a biological assay in order to quantify the specific biological activity.

Salts of the compounds of formula (I) according to the invention can be obtained by dissolving the free compound in a suitable solvent (for example a ketone such as acetone, methylethylketone or methylisobutylketone, an ether such as diethyl ether, tetrahydrofuran or dioxane, a chlorinated hydrocarbon such as methylene chloride or chloroform, or a low molecular weight aliphatic alcohol such as methanol, ethanol or isopropanol) which contains the desired acid or base, or to which the desired acid or base is then added. The acid or base can be employed in salt preparation, depending on whether a mono- or polybasic acid or base is concerned and depending on which salt is desired, in an equimolar ratio or one differing therefrom. The salts are obtained by filtering, reprecipitating, precipitating with a non-solvent for the salt or by evaporating the solvent. Salts obtained can be converted into the free compounds which, in turn, can be converted into salts. In this manner, pharmaceutically unacceptable salts, which can be obtained, for example, as process products in the manufacturing on an industrial scale, can be converted into pharmaceutically acceptable salts by processes known to the person skilled in the art. Especially preferred are hydrochlorides and the process used in the example section.

Pure diastereomers and pure enantiomers of the compounds and salts according to the invention can be obtained e.g. by asymmetric synthesis, by using chiral starting compounds in synthesis or by splitting up enantiomeric and diasteriomeric mixtures obtained in synthesis.

Enantiomeric and diastereomeric mixtures can be split up into the pure enantiomers and pure diastereomers by methods known to the person skilled in the art. In one embodiment, diastereomeric mixtures are separated by crystallization, in particular fractional crystallization, or chromatography. Enantiomeric mixtures can be separated *e.g*. by forming diastereomers with a chiral auxiliary agent, resolving the diastereomers obtained and removing the chiral auxiliary agent. As chiral auxiliary agents, for example, chiral acids can be used to separate enantiomeric bases such as e.g. mandelic acid and chiral bases can be used to separate enantiomeric acids by formation of diastereomeric salts. Furthermore, diastereomeric derivatives such as diastereomeric esters can be formed from enantiomeric mixtures of alcohols or enantiomeric mixtures of acids, respectively, using chiral acids or chiral alcohols, respectively, as chiral auxiliary agents. Additionally, diastereomeric complexes or diastereomeric clathrates may be used for separating enantiomeric mixtures. Alternatively, enantiomeric mixtures can be split up using chiral separating columns in chromatography. Another suitable method for the isolation of enantiomers is the enzymatic separation.

One preferred aspect of the invention is the process for the preparation of the compounds of claims 1-4 according to the examples as well as the intermediates used for their preparation.

Optionally, compounds of the formula (I) can be converted into their salts, or, optionally, salts of the compounds of the formula (I) can be converted into the free compounds. Corresponding processes are customary for the skilled person.

### Commercial utility

As mentioned supra, the compounds of the present invention have surprisingly been found to effectively inhibit mutant EGFR in a cell (*e.g.,* a cancer cell) contacted with the compound, thereby inducing cell death (*e.g.,* apoptosis) and may therefore be used for the treatment or prophylaxis of diseases of uncontrolled cell growth, proliferation and/or survival, inappropriate cellular immune responses, or inappropriate cellular inflammatory responses, or diseases which are accompanied with uncontrolled cell growth, proliferation and/or survival, inappropriate cellular immune responses, or inappropriate cellular inflammatory responses, particularly in which the uncontrolled cell growth, proliferation and/or survival, inappropriate cellular immune responses, or inappropriate cellular inflammatory responses is mediated by mutant EGFR, such as, for example, benign and malignant neoplasia, more specifically haematological tumours, solid tumours, and/or metastases thereof, e.g. leukaemias and myelodysplastic syndrome, malignant lymphomas, head and neck tumours including brain tumours and brain metastases, tumours of the thorax including non-small cell and small cell lung tumours, gastrointestinal tumours, endocrine tumours, mammary and other gynaecological tumours, urological tumours including renal, bladder and prostate tumours, skin tumours, and sarcomas, and/or metastases thereof, especially haematological tumours, solid tumours, and/or metastases of breast, bladder, bone, brain, central and peripheral nervous system, cervix, colon, endocrine glands (*e.g.,* thyroid and adrenal cortex), endocrine tumours, endometrium, esophagus, gastrointestinal tumours, germ cells, kidney, liver, lung, larynx and hypopharynx, mesothelioma, ovary, pancreas, prostate, rectum, renal, small intestine, soft tissue, stomach, skin, testis, ureter, vagina and vulva as well as malignant neoplasias including primary tumours in said organs and corresponding secondary tumours in distant organs ("tumour metastases"). Haematological tumours can, e.g., be exemplified by aggressive and indolent forms of leukemia and lymphoma, namely non-Hodgkins disease, chronic and acute myeloid leukemia (CML / AML), acute lymphoblastic leukemia (ALL), Hodgkins disease, multiple myeloma and T-cell lymphoma. Also included are myelodysplastic syndrome, plasma cell neoplasia, paraneoplastic syndromes, and cancers of unknown primary site, as well as AIDS related malignancies.

In accordance with an aspect of the present invention therefore the invention relates to a compound of general formula I as defined in the claims herein for use in the treatment or prophylaxis of a disease, especially for use in the treatment of a disease.

In accordance with an embodiment as defined in claim 6 herein, the present invention relates to said compound for use, wherein the diseases are hyperproliferative diseases and/or disorders responsive to induction of cell death.

In accordance with an embodiment as defined in claim 7 herein, the present invention relates to said compound for use, wherein the hyperproliferative diseases and/or disorders responsive to induction of cell death are haematological tumours, solid tumours and/or metastases thereof.

In accordance with an embodiment as defined in claim 8 herein, the present invention relates to said compound for use, wherein the tumour harbors a mutant EGFR and/or metastases thereof.

In accordance with an embodiment as defined in claim 9 herein, the present invention relates to said compound for use, wherein the tumour is lung cancer, particularly lung cancer harboring a mutant EGFR with exon 20 insertion mutation, and/or metastases thereof.

In accordance with an embodiment as defined in claim 10 herein, the present invention relates to said compound for use, wherein the tumour is lung cancer, particularly lung cancer harboring a mutant EGFR with in-frame deletions in exon 19 (such as EGFR E746_A750del) or point mutations in exon 21 (e.g. L858R), and/or metastases thereof.

In accordance with an embodiment as defined in claim 11 herein, the present invention relates to said compound for use, wherein the tumour is lung cancer, particularly lung cancer harboring a mutant EGFR with an exon 20 insertion and a T790M mutation, e.g. a D770_N771insSVD T790M mutation, and/or metastases thereof.

In accordance with an embodiment as defined in claim 12 herein, the present invention relates to said compound for use, wherein the tumour is lung cancer, particularly lung cancer harboring a mutant EGFR with inframe deletion in exon 19 such as E746_A750del and a T790M mutation, and/or metastases thereof.

In accordance with an embodiment as defined in claim 13 herein, the present invention relates to said compound for use, wherein the tumour is lung cancer, particularly lung cancer harboring a mutant EGFR with a point mutation in exon 21 such as L858R and a T790M mutation, and/or metastases thereof.

In accordance with an embodiment as defined in claim 14 herein, the present invention relates to said compound for use, wherein the tumour is lung cancer, particularly lung cancer harboring a mutant ERBB2 with exon 20 insertion mutations (such as ERBB2 A775_G776insYVMA), and/or metastases thereof.

By "hyperproliferative disease" is meant a disease, such as cancer, associated with inappropriately high levels of cell division, inappropriately low levels of apoptosis, or both. The term "inappropriate" within the context of the present invention, in particular in the context of "inappropriate cellular immune responses, or inappropriate cellular inflammatory responses", as used herein, is to be understood as generally meaning a response, which is less than, or greater than normal, and which is associated with, responsible for, or results in, the pathology of said diseases.

Hyper-proliferative disorders include psoriasis, keloids, and other hyperplasias affecting the skin, benign prostate hyperplasia (BPH), solid tumours, such as cancers of the breast, respiratory tract, brain, reproductive organs, digestive tract, urinary tract, eye, liver, skin, head and neck, thyroid, parathyroid and their distant metastases. Those disorders also include lymphomas, sarcomas, and leukaemias.

Examples of breast cancer include invasive ductal carcinoma, invasive lobular carcinoma, ductal carcinoma in situ, and lobular carcinoma *in situ.*

Examples of cancers of the respiratory tract include small-cell and non-small-cell lung carcinoma, as well as bronchial adenoma and pleuropulmonary blastoma.

Examples of brain cancers include brain stem and hypothalmic glioma, cerebellar and cerebral astrocytoma, medulloblastoma, ependymoma, as well as neuroectodermal and pineal tumour.

Tumours of the male reproductive organs include prostate and testicular cancer. Tumours of the female reproductive organs include endometrial, cervical, ovarian, vaginal, and vulvar cancer, as well as sarcoma of the uterus.

Tumours of the digestive tract include anal, colon, colorectal, oesophageal, gallbladder, gastric, pancreatic, rectal, small-intestine, and salivary gland cancers.

Tumours of the urinary tract include bladder, penile, kidney, renal pelvis, ureter, urethral and human papillary renal cancers.

Eye cancers include intraocular melanoma and retinoblastoma.

Examples of liver cancers include hepatocellular carcinoma (liver cell carcinomas with or without fibrolamellar variant), cholangiocarcinoma (intrahepatic bile duct carcinoma), and mixed hepatocellular cholangiocarcinoma.

Skin cancers include squamous cell carcinoma, Kaposi's sarcoma, malignant melanoma, inverted sinonasal papilloma, inverted sinonasal papilloma-associated sinonasal squamous cell carcinoma, Merkel cell skin cancer, and non-melanoma skin cancer.

Head-and-neck cancers include laryngeal, hypopharyngeal, nasopharyngeal, oropharyngeal cancer, inverted sinonasal papilloma, inverted sinonasal papilloma-associated sinonasal squamous cell carcinoma, lip and oral cavity cancer and squamous cell. Lymphomas include AIDS-related lymphoma, non-Hodgkin's lymphoma, cutaneous T-cell lymphoma, Burkitt lymphoma, Hodgkin's disease, and lymphoma of the central nervous system.

Sarcomas include sarcoma of the soft tissue, osteosarcoma, malignant fibrous histiocytoma, lymphosarcoma, and rhabdomyosarcoma.

Leukemias include acute myeloid leukemia, acute lymphoblastic leukemia, chronic lymphocytic leukemia, chronic myelogenous leukemia, and hairy cell leukemia.

These disorders have been well characterized in humans, but also exist with a similar etiology in other mammals, and can be treated by administering pharmaceutical compositions of the present invention.

The term "treating" or "treatment" as stated throughout this document is used conventionally, *e.g.,* the management or care of a subject for the purpose of combating, alleviating, reducing, relieving, improving the condition of, *etc.,* of a disease or disorder, such as a carcinoma.

### Pharmaceutical compositions of the compounds of the invention

This invention also relates to pharmaceutical compositions containing one or more compounds of the present invention as defined in the claims herein. These compositions can be utilised to achieve the desired pharmacological effect by administration to a patient in need thereof. A patient, for the purpose of this invention, is a mammal, including a human, in need of treatment for the particular condition, disorder, or disease.

Therefore, the present invention includes pharmaceutical compositions that are comprised of a pharmaceutically acceptable carrier or auxiliary and a pharmaceutically effective amount of a compound, or salt thereof, of the present invention.

Another aspect of the invention is a pharmaceutical composition comprising a pharmaceutically effective amount of a compound of formula (I) and a pharmaceutically acceptable auxiliary for the treatment of a disease mentioned supra, especially for the treatment of haematological tumours, solid tumours and/or metastases thereof.

A pharmaceutically acceptable carrier or auxiliary may be a carrier that is non-toxic and innocuous to a patient at concentrations consistent with effective activity of the active ingredient so that any side effects ascribable to the carrier do not vitiate the beneficial effects of the active ingredient. Carriers and auxiliaries are all kinds of additives assisting to the composition to be suitable for administration.

A pharmaceutically effective amount of compound may be that amount which produces a result or exerts the intended influence on the particular condition being treated.

The compounds of the present invention can be administered with pharmaceutically-acceptable carriers or auxiliaries well known in the art using any effective conventional dosage unit forms, including immediate, slow and timed release preparations, orally, parenterally, topically, nasally, ophthalmically, optically, sublingually, rectally, vaginally, and the like.

For oral administration, the compounds can be formulated into solid or liquid preparations such as capsules, pills, tablets, troches, lozenges, melts, powders, solutions, suspensions, or emulsions, and may be prepared according to methods known to the art for the manufacture of pharmaceutical compositions. The solid unit dosage forms can be a capsule that can be of the ordinary hard- or soft-shelled gelatine type containing auxiliaries, for example, surfactants, lubricants, and inert fillers such as lactose, sucrose, calcium phosphate, and corn starch.

In another embodiment, the compounds of this invention may be tableted with conventional tablet bases such as lactose, sucrose and cornstarch in combination with binders such as acacia, corn starch or gelatine, disintegrating agents intended to assist the break-up and dissolution of the tablet following administration, such as potato starch, alginic acid, corn starch, and guar gum, gum tragacanth, acacia, lubricants intended to improve the flow of tablet granulation and to prevent the adhesion of tablet material to the surfaces of the tablet dies and punches, for example talc, stearic acid, or magnesium, calcium or zinc stearate, dyes, colouring agents, and flavouring agents such as peppermint, oil of wintergreen, or cherry flavouring, intended to enhance the aesthetic qualities of the tablets and make them more acceptable to the patient. Suitable excipients for use in oral liquid dosage forms include dicalcium phosphate and diluents such as water and alcohols, for example, ethanol, benzyl alcohol, and polyethylene alcohols, either with or without the addition of a pharmaceutically acceptable surfactant, suspending agent or emulsifying agent. Various other materials may be present as coatings or to otherwise modify the physical form of the dosage unit. For instance tablets, pills or capsules may be coated with shellac, sugar or both.

Dispersible powders and granules are suitable for the preparation of an aqueous suspension. They provide the active ingredient in admixture with a dispersing or wetting agent, a suspending agent and one or more preservatives. Suitable dispersing or wetting agents and suspending agents are exemplified by those already mentioned above. Additional excipients, for example those sweetening, flavouring and colouring agents described above, may also be present.

The pharmaceutical compositions of this invention may also be in the form of oil-in-water emulsions. The oily phase may be a vegetable oil such as liquid paraffin or a mixture of vegetable oils. Suitable emulsifying agents may be (1) naturally occurring gums such as gum acacia and gum tragacanth, (2) naturally occurring phosphatides such as soy bean and lecithin, (3) esters or partial esters derived from fatty acids and hexitol anhydrides, for example, sorbitan monooleate, (4) condensation products of said partial esters with ethylene oxide, for example, polyoxyethylene sorbitan monooleate. The emulsions may also contain sweetening and flavouring agents.

Oily suspensions may be formulated by suspending the active ingredient in a vegetable oil such as, for example, arachis oil, olive oil, sesame oil or coconut oil, or in a mineral oil such as liquid paraffin. The oily suspensions may contain a thickening agent such as, for example, beeswax, hard paraffin, or cetyl alcohol. The suspensions may also contain one or more preservatives, for example, ethyl or n-propyl p-hydroxybenzoate; one or more colouring agents; one or more flavouring agents; and one or more sweetening agents such as sucrose or saccharin.

Syrups and elixirs may be formulated with sweetening agents such as, for example, glycerol, propylene glycol, sorbitol or sucrose. Such formulations may also contain a demulcent, and preservative, such as methyl and propyl parabens and flavouring and colouring agents.

The compounds of this invention may also be administered parenterally, that is, subcutaneously, intravenously, intraocularly, intrasynovially, intramuscularly, or interperitoneally, as injectable dosages of the compound in, for example, a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1, 1-dioxolane-4-methanol, ethers such as poly(ethylene glycol) 400, an oil, a fatty acid, a fatty acid ester or, a fatty acid glyceride, or an acetylated fatty acid glyceride, with or without the addition of a pharmaceutically acceptable surfactant such as a soap or a detergent, suspending agent such as pectin, carbomers, methycellulose, hydroxypropylmethylcellulose, or carboxymethylcellulose, or emulsifying agent and other pharmaceutical adjuvants.

Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum and mineral oil. Suitable fatty acids include oleic acid, stearic acid, isostearic acid and myristic acid. Suitable fatty acid esters are, for example, ethyl oleate and isopropyl myristate. Suitable soaps include fatty acid alkali metal, ammonium, and triethanolamine salts and suitable detergents include cationic detergents, for example dimethyl dialkyl ammonium halides, alkyl pyridinium halides, and alkylamine acetates; anionic detergents, for example, alkyl, aryl, and olefin sulfonates, alkyl, olefin, ether, and monoglyceride sulfates, and sulfosuccinates; non-ionic detergents, for example, fatty amine oxides, fatty acid alkanolamides, and poly(oxyethylene-oxypropylene)s or ethylene oxide or propylene oxide copolymers; and amphoteric detergents, for example, alkyl-beta-aminopropionates, and 2-alkylimidazoline quarternary ammonium salts, as well as mixtures.

The parenteral compositions of this invention will typically contain from about 0.5% to about 25% by weight of the active ingredient in solution. Preservatives and buffers may also be used advantageously. In order to minimise or eliminate irritation at the site of injection, such compositions may contain a non-ionic surfactant having a hydrophile-lipophile balance (HLB) in one embodiment of from about 12 to about 17. The quantity of surfactant in such formulation in one embodiment ranges from about 5% to about 15% by weight. The surfactant can be a single component having the above HLB or can be a mixture of two or more components having the desired HLB.

Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

The pharmaceutical compositions may be in the form of sterile injectable aqueous suspensions. Such suspensions may be formulated according to known methods using suitable dispersing or wetting agents and suspending agents such as, for example, sodium carboxymethylcellulose, methylcellulose, hydroxypropylmethyl-cellulose, sodium alginate, polyvinylpyrrolidone, gum tragacanth and gum acacia; dispersing or wetting agents which may be a naturally occurring phosphatide such as lecithin, a condensation product of an alkylene oxide with a fatty acid, for example, polyoxyethylene stearate, a condensation product of ethylene oxide with a long chain aliphatic alcohol, for example, heptadeca-ethyleneoxycetanol, a condensation product of ethylene oxide with a partial ester derived form a fatty acid and a hexitol such as polyoxyethylene sorbitol monooleate, or a condensation product of an ethylene oxide with a partial ester derived from a fatty acid and a hexitol anhydride, for example polyoxyethylene sorbitan monooleate.

The sterile injectable preparation may also be a sterile injectable solution or suspension in a non-toxic parenterally acceptable diluent or solvent. Diluents and solvents that may be employed are, for example, water, Ringer's solution, isotonic sodium chloride solutions and isotonic glucose solutions. In addition, sterile fixed oils are conventionally employed as solvents or suspending media. For this purpose, any bland, fixed oil may be employed including synthetic mono- or diglycerides. In addition, fatty acids such as oleic acid can be used in the preparation of injectables.

A composition of the invention may also be administered in the form of suppositories for rectal administration of the drug. These compositions can be prepared by mixing the drug with a suitable non-irritation excipient which is solid at ordinary temperatures but liquid at the rectal temperature and will therefore melt in the rectum to release the drug. Such materials are, for example, cocoa butter and polyethylene glycol.

Controlled release formulations for parenteral administration include liposomal, polymeric microsphere and polymeric gel formulations that are known in the art.

It may be desirable or necessary to introduce the pharmaceutical composition to the patient via a mechanical delivery device. The construction and use of mechanical delivery devices for the delivery of pharmaceutical agents is well known in the art. Direct techniques for administration, for example, administering a drug directly to the brain usually involve placement of a drug delivery catheter into the patient's ventricular system to bypass the blood-brain barrier. One such implantable delivery system, used for the transport of agents to specific anatomical regions of the body, is described in US Patent No. 5,011,472, issued April 30, 1991.

The compositions of the invention can also contain other conventional pharmaceutically acceptable compounding ingredients, generally referred to as carriers or diluents, as necessary or desired. Conventional procedures for preparing such compositions in appropriate dosage forms can be utilized.

Such ingredients and procedures include those described in the following references: Powell, M.F. et al., "Compendium of Excipients for Parenteral Formulations" PDA Journal of Pharmaceutical Science & Technology 1998, 52(5), 238-311; Strickley, R.G "Parenteral Formulations of Small Molecule Therapeutics Marketed in the United States (1999)-Part-1" PDA Journal of Pharmaceutical Science & Technology 1999, 53(6), 324-349; and Nema, S. et al., "Excipients and Their Use in Injectable Products" PDA Journal of Pharmaceutical Science & Technology 1997, 51(4), 166-171.

Commonly used pharmaceutical ingredients that can be used as appropriate to formulate the composition for its intended route of administration include:
acidifying agents (examples include but are not limited to acetic acid, citric acid, fumaric acid, hydrochloric acid, nitric acid);
alkalinizing agents (examples include but are not limited to ammonia solution, ammonium carbonate, diethanolamine, monoethanolamine, potassium hydroxide, sodium borate, sodium carbonate, sodium hydroxide, triethanolamine, trolamine);
adsorbents (examples include but are not limited to powdered cellulose and activated charcoal); aerosol propellants (examples include but are not limited to carbon dioxide, CCl₂F₂, F₂ClC-CClF₂ and CClF₃);
air displacement agents (examples include but are not limited to nitrogen and argon);
antifungal preservatives (examples include but are not limited to benzoic acid, butylparaben, ethylparaben, methylparaben, propylparaben, sodium benzoate);
antimicrobial preservatives (examples include but are not limited to benzalkonium chloride, benzethonium chloride, benzyl alcohol, cetylpyridinium chloride, chlorobutanol, phenol, phenylethyl alcohol, phenylmercuric nitrate and thimerosal);
antioxidants (examples include but are not limited to ascorbic acid, ascorbyl palmitate, butylated hydroxyanisole, butylated hydroxytoluene, hypophosphorus acid, monothioglycerol, propyl gallate, sodium ascorbate, sodium bisulfite, sodium formaldehyde sulfoxylate, sodium metabisulfite);
binding materials (examples include but are not limited to block polymers, natural and synthetic rubber, polyacrylates, polyurethanes, silicones, polysiloxanes and styrene-butadiene copolymers);
buffering agents (examples include but are not limited to potassium metaphosphate, dipotassium phosphate, sodium acetate, sodium citrate anhydrous and sodium citrate dihydrate);
carrying agents (examples include but are not limited to acacia syrup, aromatic syrup, aromatic elixir, cherry syrup, cocoa syrup, orange syrup, syrup, corn oil, mineral oil, peanut oil, sesame oil, bacteriostatic sodium chloride injection and bacteriostatic water for injection);
chelating agents (examples include but are not limited to edetate disodium and edetic acid);
colourants (examples include but are not limited to FD&C Red No. 3, FD&C Red No. 20, FD&C Yellow No. 6, FD&C Blue No. 2, D&C Green No. 5, D&C Orange No. 5, D&C Red No. 8, caramel and ferric oxide red);
clarifying agents (examples include but are not limited to bentonite);
emulsifying agents (examples include but are not limited to acacia, cetomacrogol, cetyl alcohol, glyceryl monostearate, lecithin, sorbitan monooleate, polyoxyethylene 50 monostearate);
encapsulating agents (examples include but are not limited to gelatin and cellulose acetate phthalate);
flavourants (examples include but are not limited to anise oil, cinnamon oil, cocoa, menthol, orange oil, peppermint oil and vanillin);
humectants (examples include but are not limited to glycerol, propylene glycol and sorbitol);
levigating agents (examples include but are not limited to mineral oil and glycerin);
oils (examples include but are not limited to arachis oil, mineral oil, olive oil, peanut oil, sesame oil and vegetable oil);
ointment bases (examples include but are not limited to lanolin, hydrophilic ointment, polyethylene glycol ointment, petrolatum, hydrophilic petrolatum, white ointment, yellow ointment, and rose water ointment);
penetration enhancers (transdermal delivery) (examples include but are not limited to monohydroxy or polyhydroxy alcohols, mono-or polyvalent alcohols, saturated or unsaturated fatty alcohols, saturated or unsaturated fatty esters, saturated or unsaturated dicarboxylic acids, essential oils, phosphatidyl derivatives, cephalin, terpenes, amides, ethers, ketones and ureas);
plasticizers (examples include but are not limited to diethyl phthalate and glycerol);
solvents (examples include but are not limited to ethanol, corn oil, cottonseed oil, glycerol, isopropanol, mineral oil, oleic acid, peanut oil, purified water, water for injection, sterile water for injection and sterile water for irrigation);
stiffening agents (examples include but are not limited to cetyl alcohol, cetyl esters wax, microcrystalline wax, paraffin, stearyl alcohol, white wax and yellow wax);
suppository bases (examples include but are not limited to cocoa butter and polyethylene glycols (mixtures));
surfactants (examples include but are not limited to benzalkonium chloride, nonoxynol 10, oxtoxynol 9, polysorbate 80, sodium lauryl sulfate and sorbitan mono-palmitate);
suspending agents (examples include but are not limited to agar, bentonite, carbomers, carboxymethylcellulose sodium, hydroxyethyl cellulose, hydroxypropyl cellulose, hydroxypropyl methylcellulose, kaolin, methylcellulose, tragacanth and veegum);
sweetening agents (examples include but are not limited to aspartame, dextrose, glycerol, mannitol, propylene glycol, saccharin sodium, sorbitol and sucrose);
tablet anti-adherents (examples include but are not limited to magnesium stearate and talc);
tablet binders (examples include but are not limited to acacia, alginic acid, carboxymethylcellulose sodium, compressible sugar, ethylcellulose, gelatin, liquid glucose, methylcellulose, non-crosslinked polyvinyl pyrrolidone, and pregelatinized starch);
tablet and capsule diluents (examples include but are not limited to dibasic calcium phosphate, kaolin, lactose, mannitol, microcrystalline cellulose, powdered cellulose, precipitated calcium carbonate, sodium carbonate, sodium phosphate, sorbitol and starch);
tablet coating agents (examples include but are not limited to liquid glucose, hydroxyethyl cellulose, hydroxypropyl cellulose, hydroxypropyl methylcellulose, methylcellulose, ethylcellulose, cellulose acetate phthalate and shellac);
tablet direct compression excipients (examples include but are not limited to dibasic calcium phosphate);
tablet disintegrants (examples include but are not limited to alginic acid, carboxymethylcellulose calcium, microcrystalline cellulose, polacrillin potassium, cross-linked polyvinylpyrrolidone, sodium alginate, sodium starch glycollate and starch);
tablet glidants (examples include but are not limited to colloidal silica, corn starch and talc);
tablet lubricants (examples include but are not limited to calcium stearate, magnesium stearate, mineral oil, stearic acid and zinc stearate);
tablet/capsule opaquants (examples include but are not limited to titanium dioxide);
tablet polishing agents (examples include but are not limited to carnuba wax and white wax);
thickening agents (examples include but are not limited to beeswax, cetyl alcohol and paraffin); tonicity agents (examples include but are not limited to dextrose and sodium chloride);
viscosity increasing agents (examples include but are not limited to alginic acid, bentonite, carbomers, carboxymethylcellulose sodium, methylcellulose, polyvinyl pyrrolidone, sodium alginate and tragacanth); and
wetting agents (examples include but are not limited to heptadecaethylene oxycetanol, lecithins, sorbitol monooleate, polyoxyethylene sorbitol monooleate, and polyoxyethylene stearate).

Pharmaceutical compositions according to the present invention can be illustrated as follows:
Sterile i.v. solution: A 5 mg/ml solution of the desired compound of this invention can be made using sterile, injectable water, and the pH is adjusted if necessary. The solution is diluted for administration to 1 - 2 mg/ml with sterile 5% dextrose and is administered as an i.v. infusion over about 60 minutes.
Lyophilised powder for i.v. administration: A sterile preparation can be prepared with (i) 100 - 1000 mg of the desired compound of this invention as a lyophilised powder, (ii) 32- 327 mg/ml sodium citrate, and (iii) 300 - 3000 mg Dextran 40. The formulation is reconstituted with sterile, injectable saline or dextrose 5% to a concentration of 10 to 20 mg/ml, which is further diluted with saline or dextrose 5% to 0.2 - 0.4 mg/ml, and is administered either IV bolus or by IV infusion over 15 - 60 minutes.
Intramuscular suspension: The following solution or suspension can be prepared, for intramuscular injection:
   50 mg/ml of the desired, water-insoluble compound of this invention
   5 mg/ml sodium carboxymethylcellulose
   4 mg/ml TWEEN 80
   9 mg/ml sodium chloride
   9 mg/ml benzyl alcohol
Hard Shell Capsules: A large number of unit capsules are prepared by filling standard two-piece hard galantine capsules each with 100 mg of powdered active ingredient, 150 mg of lactose, 50 mg of cellulose and 6 mg of magnesium stearate.
Soft Gelatin Capsules: A mixture of active ingredient in a digestible oil such as soybean oil, cottonseed oil or olive oil is prepared and injected by means of a positive displacement pump into molten gelatin to form soft gelatin capsules containing 100 mg of the active ingredient. The capsules are washed and dried. The active ingredient can be dissolved in a mixture of polyethylene glycol, glycerin and sorbitol to prepare a water miscible medicine mix.
Tablets: A large number of tablets are prepared by conventional procedures so that the dosage unit is 100 mg of active ingredient, 0.2 mg. of colloidal silicon dioxide, 5 mg of magnesium stearate, 275 mg of microcrystalline cellulose, 11 mg. of starch, and 98.8 mg of lactose. Appropriate aqueous and non-aqueous coatings may be applied to increase palatability, improve elegance and stability or delay absorption.
Immediate Release Tablets/Capsules: These are solid oral dosage forms made by conventional and novel processes. These units are taken orally without water for immediate dissolution and delivery of the medication. The active ingredient is mixed in a liquid containing ingredient such as sugar, gelatin, pectin and sweeteners. These liquids are solidified into solid tablets or caplets by freeze drying and solid state extraction techniques. The drug compounds may be compressed with viscoelastic and thermoelastic sugars and polymers or effervescent components to produce porous matrices intended for immediate release, without the need of water.

### Dose and administration

Based upon standard laboratory techniques known to evaluate compounds useful for the treatment of hyper-proliferative disorders and angiogenic disorders, by standard toxicity tests and by standard pharmacological assays for the determination of treatment of the conditions identified above in mammals, and by comparison of these results with the results of known medicaments that are used to treat these conditions, the effective dosage of the compounds of this invention can readily be determined for treatment of each desired indication. The amount of the active ingredient to be administered in the treatment of one of these conditions can vary widely according to such considerations as the particular compound and dosage unit employed, the mode of administration, the period of treatment, the age and sex of the patient treated, and the nature and extent of the condition treated.

The total amount of the active ingredient to be administered will generally range from about 0.001 mg/kg to about 200 mg/kg body weight per day, and in particular embodiments from about 0.01 mg/kg to about 20 mg/kg body weight per day. Clinically useful dosing schedules will range from one to three times a day dosing to once every four weeks dosing. In addition, "drug holidays" in which a patient is not dosed with a drug for a certain period of time, may be beneficial to the overall balance between pharmacological effect and tolerability. A unit dosage may contain from about 0.5 mg to about 1500 mg of active ingredient, and can be administered one or more times per day or less than once a day. The average daily dosage for administration by injection, including intravenous, intramuscular, subcutaneous and parenteral injections, and use of infusion techniques will in other embodiments be from 0.01 to 200 mg/kg of total body weight. The average daily rectal dosage regimen will in particular embodiments be from 0.01 to 200 mg/kg of total body weight. The average daily vaginal dosage regimen will in other embodiments be from 0.01 to 200 mg/kg of total body weight. The average daily topical dosage regimen will in still other embodiments be from 0.1 to 200 mg administered between one to four times daily. The transdermal concentration will in other embodiments be that required to maintain a daily dose of from 0.01 to 200 mg/kg. The average daily inhalation dosage regimen will in other embodiments be from 0.01 to 100 mg/kg of total body weight.

Of course the specific initial and continuing dosage regimen for each patient will vary according to the nature and severity of the condition as determined by the attending diagnostician, the activity of the specific compound employed, the age and general condition of the patient, time of administration, route of administration, rate of excretion of the drug, drug combinations, and the like. The desired mode of treatment and number of doses of a compound of the present invention or a pharmaceutically acceptable salt or ester or composition thereof can be ascertained by those skilled in the art using conventional treatment tests.

### Combination Therapies

The compounds of this invention can be administered as the sole pharmaceutical agent or in combination with one or more other pharmaceutical agents where the combination causes no unacceptable adverse effects. Those combined pharmaceutical agents can be other agents having anti proliferative effects such as for example for the treatment of haematological tumours, solid tumours and/or metastases thereof and/or agents for the treatment of undesired side effects. The present invention relates also to such combinations.

Other anti-hyper-proliferative agents suitable for use with the composition of the invention include but are not limited to those compounds acknowledged to be used in the treatment of neoplastic diseases in Goodman and Gilman's The Pharmacological Basis of Therapeutics (Ninth Edition), editor Molinoff et al., publ. by McGraw-Hill, pages 1225-1287, (1996) especially (chemotherapeutic) anti-cancer agents as defined supra. The combination can be a non-fixed combination or a fixed-dose combination as the case may be.

Methods of testing for a particular pharmacological or pharmaceutical property are well known to persons skilled in the art.

The example testing experiments described herein serve to illustrate the present invention.

As will be appreciated by persons skilled in the art, the invention is as defined by the appended claims.

The following examples illustrate the invention in greater detail. Further compounds according to the invention, of which the preparation is not explicitly described, can be prepared in an analogous way.

The term "according to" within the experimental section is used in the sense that the procedure referred to is to be used "analogously to".

### EXPERIMENTAL SECTION

Chemical names were generated using the ACD/Name software from ACD/Labs. In some cases generally accepted names of commercially available reagents were used in place of ACD/Name generated names.

The following table 1 lists the abbreviations used in this paragraph and in the Examples section as far as they are not explained within the text body. Other abbreviations have their meanings customary *per se* to the skilled person.

**Table 1: Abbreviations**

| **Abbreviation** | **Meaning** |
|---|---|
| ACN, MeCN | Acetonitrile |
| br | broad signal (NMR) |
| d | doublet (NMR) |
| DAD | Diode Array Detector |
| DBU | 1,8-Diazabicyclo(5.4.0)undec-7-ene |
| DCM | Dichloromethane |
| dd | doublet of doublet (NMR) |
| DIPEA | Diisopropylethylamine |
| DMA | N,N-dimethylacetamide |
| DMF | N,N-dimethylformamide |
| DMSO | Dimethylsulfoxide |
| ESI | electrospray (ES) ionization |
| EtOAc | Ethyl acetate |
| EtOH | Ethanol |
| PTFE | Poly-trifluoro-ethylene |
| h, hr (hrs) | hour(s) |
| HCl | hydrogen chloride, hydrochloric acid |
| HPLC | high performance liquid chromatography |
| LC-MS | liquid chromatography-mass spectrometry |
| m | multiplet (NMR) |
| MeOH | Methanol |
| DCM | Dichloromethane |
| min | minute(s) |
| MS | mass spectrometry |
| MTBE | Methyl-*tert*-butylether |
| MWD | Multiple wavelength detector |
| NMR | Nuclear Magnetic Resonance spectroscopy : chemical shifts (δ) are given in ppm. The chemical shifts were corrected by setting the DMSO signal to 2.50 ppm using unless otherwise stated. |
| q | quartet (NMR) |
| Rt or RT | room temperature |
| Rₜ, Rt | retention time |
| s | singulet (NMR) |
| sat. | saturated |
| t | triplet (NMR) |
| td | triplet of doublet (NMR) |
| TEA | Triethylamine |
| TFA | Trifluoroacetic acid |
| THF | Tetrahydrofuran |
| δ | chemical shift |

Other abbreviations have their meanings customary *per se* to the skilled person.

The various aspects of the invention described in this application are illustrated by the following examples.

The example testing experiments described herein serve to illustrate the present invention.

### EXPERIMENTAL SECTION - GENERAL PART

All reagents, for which the synthesis is not described in the experimental part, are either commercially available, or are known compounds or may be formed from known compounds by known methods by a person skilled in the art.

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallization. In some cases, impurities may be removed by trituration using a suitable solvent. In some cases, the compounds may be purified by chromatography, particularly flash column chromatography, using for example prepacked silica gel cartridges, *e.g.* Biotage SNAP cartridges KP-Sil^{®} or KP-NH^{®} in combination with a Biotage autopurifier system (SP4^{®} or Isolera Four^{®}) and eluents such as gradients of hexane/ethyl acetate or DCM/methanol. In flash column chromatography, unmodified ("regular") silica gel may be used as well as aminophase functionalized silica gel. If reference is made to flash column chromatography or to flash chromatography in the experimental section without specification of a stationary phase, regular silica gel was used.

In some cases, the compounds may be purified by preparative HPLC using for example a Waters autopurifier equipped with a diode array detector and/or on-line electrospray ionization mass spectrometer in combination with a suitable prepacked reverse phase column and eluents such as gradients of water and acetonitrile which may contain additives such as trifluoroacetic acid, formic acid or aqueous ammonia.

In some cases, purification methods as described above can provide those compounds of the present invention which possess a sufficiently basic or acidic functionality in the form of a salt, such as, in the case of a compound of the present invention which is sufficiently basic, a trifluoroacetate or formate salt for example, or, in the case of a compound of the present invention which is sufficiently acidic, an ammonium salt for example. A salt of this type can either be transformed into its free base or free acid form, respectively, by various methods known to the person skilled in the art, or be used as salts in subsequent biological assays. It is to be understood that the specific form (*e.g.* salt, free base etc.) of a compound of the present invention as isolated and as described herein is not necessarily the only form in which said compound can be applied to a biological assay in order to quantify the specific biological activity.

### Analytical LC-MS Methods:

### Method 1

Instrument: Agilent 1290 UPLCMS 6230 TOF; Säule: BEH C 18 1.7 µm, 50x2.1mm; Eluent A: water + 0.05% formic acid (99%); Eluent B: acetonitrile + 0.05% formic acid (99%); Gradient: 0-1.7 2-90% B, 1.7-2.0 90% B; flow 1.2 ml/min; temperature: 60°C; DAD scan: 190-400 nm.

### Method 2

Instrument: Waters Acquity UPLCMS SingleQuad; Column: Acquity UPLC BEH C18 1.7 µm, 50x2.1mm; eluent A: water + 0.1 vol. % formic acid (99%), eluent B: acetonitrile; gradient: 0-1.6 min. 1-99% B, 1.6-2.0 min. 99% B; flow 0.8 ml/min; temperature: 60 °C; DAD scan: 210-400 nm.

### Method 3

Instrument: Waters Acquity UPLCMS SingleQuad; Column: Acquity UPLC BEH C18 1.7 µm, 50x2.1mm; eluent A: water + 0.2 vol. % aqueous ammonia (32%), eluent B: acetonitrile; gradient: 0-1.6 min. 1-99% B, 1.6-2.0 min. 99% B; flow 0.8 ml/min; temperature: 60 °C; DAD scan: 210-400 nm.

### Method 4

Instrument: Waters Acquity UPLCMS Single Quad; column: Kinetex 2.6 µm, 50x2.1mm; Eluent A: water + 0.05 % formic acid (99%); Eluent B: acetonitrile + 0.05 % formic acid (99%); gradient: 0-1.9 1-99% B, 1.9-2.1 99% B; flow 1.3 ml/min; temperature: 60°C; DAD scan: 200-400 nm.

### Method 5

Instrument: Waters Acquity UPLCMS SingleQuad; Column: Acquity UPLC BEH C18 1.7 µm, 50x2.1mm; eluent A: water + 0.1 vol % formic acid (99%), eluent B: acetonitrile; gradient: 0-1.6 min 1-99% B, 1.6-2.0 min 99% B; flow 0.8 ml/min; temperature: 60 °C; DAD scan: 210-400 nm.

### Preparative LC-MS Methods:

### Method 6

Instrument: Labomatic HD-5000, pump head HDK-280, gradient module NDB-1000, fraction collector Labomatic Labocol Vario 2000, ECOM TOY 18 DAD, Prepcon 5 software. Column: Chromatorex C18 10µM 120x30 mm; Eluent A: water + 0.1% formic acid; Eluent B: acetonitrile; gradient: given for intermediates and examples, rate 150 mL/min, temperature 25°C

### Method 7

Instrument: Labomatic HD-5000, pump head HDK-280, gradient module NDB-1000, fraction collector Labomatic Labocol Vario 2000, ECOM TOY 18 DAD, Prepcon 5 software. Column: Chromatorex C18 10µM 120x30 mm; Eluent A: 0.1% ammonia in water; Eluent B: acetonitrile; gradient: given for intermediates and examples, rate 150 mL/min, temperature 25°C.

### Method 8

Instrument: Labomatic HD-5000, pump head HDK-280, gradient module NDB-1000, fraction collector Labomatic Labocol Vario 2000, ECOM TOY 18 DAD, Prepcon 5 software. Column: Chromatorex C18 10µM 120x30 mm; Eluent A: water; Eluent B: acetonitrile; gradient: given for intermediates and examples, rate 150 mL/min, temperature 25°C.

### NMR Spectra:

The multiplicities of proton signals in ¹H NMR spectra given in the following paragraphs reflect the observed signal form and do not take into account any higher-order signal phenomena. As a rule, the chemical shift data refers to the center of the signal in question. In the case of wide multiplets, a range is specified. Signals hidden by solvent or water were either assigned tentatively or are not listed. Strongly broadened signals - *e.g.* caused by rapid rotation of molecular moieties or by interchanging protons - have also been assigned tentatively (often referred to as a broad multiplet or broad singlet) or are not shown.

The ¹H-NMR data of selected compounds are listed in the form of ¹H-NMR peaklists. Therein, for each signal peak the δ value in ppm is given, followed by the signal intensity, reported in round brackets. The δ value-signal intensity pairs from different peaks are separated by commas. Therefore, a peaklist is described by the general form: δ₁ (intensity₁), δ₂ (intensity₂), ... , δᵢ (intensityᵢ), ... , δₙ (intensityₙ).

The intensity of a sharp signal correlates with the height (in cm) of the signal in a printed NMR spectrum. When compared with other signals, this data can be correlated to the real ratios of the signal intensities. In the case of broad signals, more than one peak, or the center of the signal along with their relative intensity, compared to the most intense signal displayed in the spectrum, are shown. A ¹H-NMR peaklist is similar to a classical ¹H-NMR readout, and thus usually contains all the peaks listed in a classical NMR interpretation. Moreover, similar to classical ¹H-NMR printouts, peaklists can show solvent signals, signals derived from stereoisomers of the particular target compound, peaks of impurities, ¹³C satellite peaks, and/or spinning sidebands. The peaks of stereoisomers, and/or peaks of impurities are typically displayed with a lower intensity compared to the peaks of the target compound (*e.g.,* with a purity of >90%). Such stereoisomers and/or impurities may be typical for the particular manufacturing process, and therefore their peaks may help to identify a reproduction of the manufacturing process on the basis of "by-product fingerprints". An expert who calculates the peaks of the target compound by known methods (MestReC, ACD simulation, or by use of empirically evaluated expectation values), can isolate the peaks of the target compound as required, optionally using additional intensity filters. Such an operation would be similar to peak-picking in classical ¹H-NMR interpretation. A detailed description of the reporting of NMR data in the form of peaklists can be found in the publication "Citation of NMR Peaklist Data within Patent Applications" (cf. http://www.researchdisclosure.com/searching-disclosures, Research Disclosure Database Number 605005, 2014, 01 Aug 2014). In the peak picking routine, as described in the Research Disclosure Database Number 605005, the parameter "MinimumHeight" can be adjusted between 1% and 4%. However, depending on the chemical structure and/or depending on the concentration of the measured compound it may be reasonable to set the parameter "MinimumHeight" <1%.

### Syntheses of Intermediate 1 Compounds

### Intermediate 1-1

### tert-butyl {2-[(4-cyanopyridin-3-yl)oxy]ethyl}carbamate

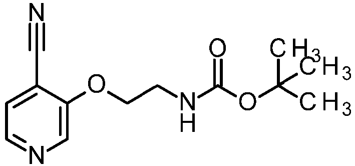

To a solution of tert-butyl (2-hydroxyethyl)carbamate (12 ml, 79 mmol) in THF (20 ml) at 0°C was added potionswise sodiumhydride (3.40 g, 60 % purity, 85.0 mmol). The reaction mixture was stirred for 1 h at RT. 3-Chloropyridine-4-carbonitrile (10.0 g, 72.2 mmol) in THF (50 ml) was added. The slurry was diluted with 16 ml THF and stirred at RT for 2 days. The reaction mixture was quenched with 1 mol/l hydrochloric acid until pH 6, extracted twice with ethyl acetate. The organic layers were concentrated under reduced pressure and purified by column chromatography (silica, gradient DCM/ethanol 0-5%) to give 3.00 g (85 % purity, 13 % yield) of the title compound.

¹H-NMR (400MHz, DMSO-d₆ 1H-NMR (400 MHz, DMSO-d6) delta [ppm]: 1.107 (0.78), 1.330 (0.55), 1.366 (16.00), 2.518 (0.62), 3.337 (2.20), 3.351 (1.77), 3.365 (0.61), 4.296 (1.14), 4.310 (2.42), 4.324 (1.08), 7.050 (0.48), 7.768 (0.96), 7.780 (1.04), 8.369 (1.64), 8.381 (1.59), 8.706 (1.99), 8.786 (0.43), 8.798 (0.42), 8.995 (0.47).

LC-MS (method 1): Rₜ = 0.88 min; MS (ESlpos): m/z = 264 [M+H]⁺

### Intermediate 1-2

### tert-butyl {3-[(4-cyanopyridin-3-yl)oxy]propyl}carbamate

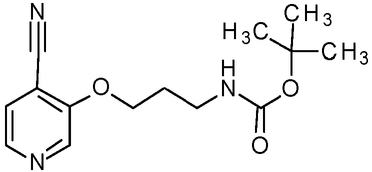

Using an analogous method as described for intermediate 1-1 with 3-chloropyridine-4-carbonitrile (3.95 g, 28.5 mmol, CAS 68325-15-5) and tert-butyl (3-hydroxypropyl)carbamate (5.00 g, 28.5 mmol) as the starting materials and stirring at RT for 16h, a tip of a spatula sodiumhydride was added and stirred at RT for 1.5 h. The reaction mixture was quenched with hydrochloric acid 1mol/l until pH 6, extracted twice into ethyl acetate. The organic layers were concentrated under reduced pressure and purified by column chromatography (silica, gradient DCM/ethanol 0-3%) to give 1.64 g (95 % purity, 20 % yield) of the title compound.

¹H-NMR (400 MHz, DMSO-d6) δ [ppm]: 1.154 (0.66), 1.172 (1.43), 1.190 (0.72), 1.358 (16.00), 1.858 (1.01), 1.875 (1.65), 1.891 (1.07), 1.987 (2.36), 2.518 (0.88), 2.522 (0.54), 3.092 (0.65), 3.109 (1.69), 3.123 (1.63), 3.140 (0.61), 4.017 (0.50), 4.034 (0.48), 4.277 (1.19), 4.291 (2.59), 4.307 (1.19), 5.758 (0.53), 6.937 (0.47), 7.774 (1.50), 7.786 (1.59), 8.369 (2.13), 8.381 (2.05), 8.672 (1.79).

### Intermediate 1-3

### tert-butyl {2-[(4-cyanopyridin-3-yl)oxy]ethyl}methylcarbamate

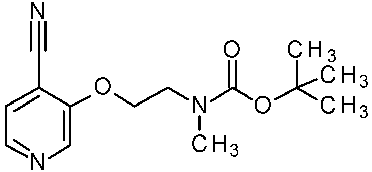

Tert-butyl (2-hydroxyethyl)methylcarbamate (15.2 g, 86.6 mmol) was dissolved in 110 ml DMF. While cooling with an ice bath sodiumhydride (3.32 g, 60 % purity, 83.0 mmol) was added portionwise and stirred for further 10 min. Still cooling 3-chloropyridine-4-carbonitrile (10.0 g, 72.2 mmol) was added and stirring continued for 2h at RT. The mixture was poured into ice water, extracted with ethyl acetate and washed with water several times. The solvents were removed in vacuum, toluene was added and removed in vacuum to give 20g (94% purity, 90 %yield) of the title compound as a brown oil.

1H-NMR (400 MHz, DMSO-d6) delta [ppm]: 1.233 (0.91), 1.307 (16.00), 1.380 (10.58), 1.442 (9.78), 2.298 (0.56), 2.518 (4.43), 2.523 (3.19), 2.673 (0.96), 2.728 (0.49), 2.739 (0.66), 2.889 (6.44), 2.925 (3.85), 3.590 (2.74), 3.602 (2.96), 4.427 (2.43), 4.440 (2.38), 7.795 (1.47), 7.807 (1.15), 8.384 (3.01), 8.396 (2.84), 8.475 (0.42), 8.488 (0.42), 8.709 (0.64), 8.731 (1.54), 8.748 (2.18).

### Intermediate 1-4

### tert-butyl (2S)-2-{[(4-cyanopyridin-3-yl)oxy]methyl}pyrrolidine-1-carboxylate

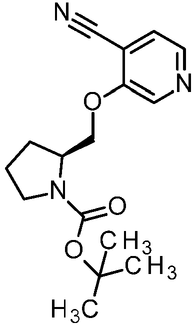

Using an analogous method as described for intermediate 1-1 with 3-chloropyridine-4-carbonitrile (2.50 g, 18.1 mmol) and tert-butyl (2S)-2-(hydroxymethyl)pyrrolidine-1-carboxylate (4.00 g, 19.9 mmol, CAS 69610-40-8) as the starting materials and stirring at RT for 16h. Sat. aqueous ammoniumchloride solution was added, diluted with water and extracted with ethyl acetate. The solvents were removed in vacuum. The residue was trituated with MTBE, the filtrate was purified by column chromatography (silica, gradient hexane/ethyl acetate 0-66%) to give 4.02 g (73.3 % yield) of the title compound.

1H-NMR (400 MHz, DMSO-d6) delta [ppm]: 1.154 (1.08), 1.172 (2.07), 1.190 (0.95), 1.376 (14.20), 1.383 (16.00), 1.808 (0.85), 1.816 (0.68), 1.834 (0.40), 1.839 (0.40), 1.921 (0.85), 1.928 (0.98), 1.941 (0.57), 1.948 (0.53), 1.956 (0.52), 1.988 (4.17), 2.024 (1.33), 2.332 (0.67), 2.518 (3.50), 2.523 (2.43), 2.673 (0.68), 3.273 (1.02), 3.282 (1.48), 3.292 (1.80), 3.300 (1.93), 4.017 (0.85), 4.035 (0.87), 4.053 (0.62), 4.067 (0.93), 4.073 (1.02), 4.079 (0.97), 4.285 (0.43), 4.302 (0.53), 4.337 (1.67), 4.353 (0.82), 4.420 (0.42), 4.440 (0.40), 7.775 (0.85), 7.787 (1.48), 8.390 (1.52), 8.748 (7.37).

### Syntheses of Intermediate 2 Compounds

### Intermediate 2-1

### tert-butyl (2-{[4-(aminomethyl)pyridin-3-yl]oxy}ethyl)carbamate

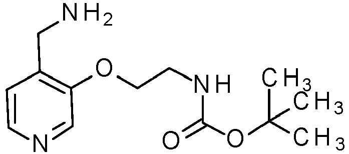

An autoclave was charged with 3 tert-butyl {2-[(4-cyanopyridin-3-yl)oxy]ethyl}carbamate (3.00 g, 11.4 mmol, intermediate 1-1), ammonia solution (40 ml, 7.0 M in methanol, 280 mmol) and Raney-Nickel (CAS 7440-02-0, 1.67 g, 28.5 mmol, 50% wetted) and the mixture was stirred under 25 bar hydrogen atmosphere at RT for 16 h. The mixture was filtered through a PTFE-filter (pore size 0.2 µm), washed with methanol and concentrated under reduced pressure.to give 3.50 g (80 % purity, 92 % yield) of the title compound.

¹H-NMR (400 MHz, DMSO-d6) δ [ppm]: 1.106 (0.66), 1.353 (1.03), 1.377 (16.00), 3.166 (3.04), 3.317 (1.36), 3.329 (1.49), 3.705 (0.44), 4.074 (0.85).

LC-MS (method 1): Rₜ = 0.39 min; MS (ESlpos): m/z = 268 [M+H]⁺

### Intermediate 2-2

### tert-butyl (3-{[4-(aminomethyl)pyridin-3-yl]oxy}propyl)carbamate

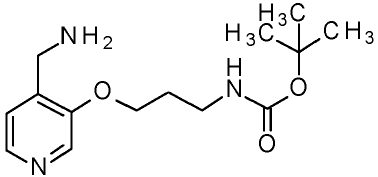

Using an analogous method as described for intermediate 2-1 with tert-butyl {3-[(4-cyanopyridin-3-yl)oxy]propyl}carbamate (1.33 g, 4.80 mmol, intermediate 1-2) as the starting material, 1.61 g (90 % purity) of the title compound were prepared.

1H-NMR (400 MHz, DMSO-d6) delta [ppm]: 1.359 (16.00), 1.826 (0.86), 1.842 (1.32), 1.858 (1.04), 1.874 (0.53), 3.079 (0.44), 3.095 (1.09), 3.110 (1.08), 3.126 (0.43), 3.692 (2.44), 4.072 (0.80), 4.087 (1.62), 4.102 (0.89), 6.926 (0.48), 7.376 (0.73), 7.388 (0.75), 8.150 (0.86), 8.161 (0.86), 8.205 (1.33).

LC-MS (method 1): Rₜ = 0.45 min; MS (ESlpos): m/z = 282 [M+H]⁺

### Intermediate 2-3

### tert-butyl (2-{[4-(aminomethyl)pyridin-3-yl]oxy}ethyl)methylcarbamate

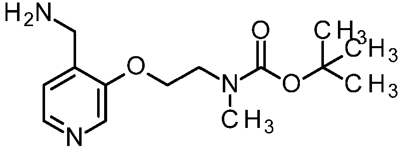

Using an analogous method as described for intermediate 2-1 with tert-butyl {2-[(4-cyanopyridin-3-yl)oxy]ethyl}methylcarbamate (19.1 g, 68.9 mmol, intermediate 1-3) as the starting material 18.8g (90% purity, 96.6% yield) of the title compound were prepared as a dark brown oil

1H-NMR (400 MHz, DMSO-d6) delta [ppm]: 1.233 (0.76), 1.349 (16.00), 1.398 (9.05), 1.889 (0.77), 2.518 (2.35), 2.523 (1.57), 2.739 (0.66), 2.863 (4.11), 2.888 (3.51), 3.558 (2.60), 3.571 (5.32), 3.585 (2.84), 3.678 (3.07), 4.198 (1.79), 4.212 (1.58), 7.394 (1.11), 8.169 (1.53), 8.180 (1.54), 8.255 (1.29).

### Intermediate 2-4

### tert-butyl (2S)-2-({[4-(aminomethyl)pyridin-3-yl]oxy}methyl)pyrrolidine-1-carboxylate

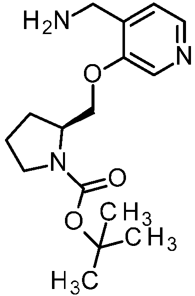

Using an analogous method as described for intermediate 2-1 with tert-butyl (2S)-2-{[(4-cyanopyridin-3-yl)oxy]methyl}pyrrolidine-1-carboxylate (5.00 g, 16.5 mmol, intermediate 1-4) as the starting material, 5.05 g (100 % yield) of the title compound were prepared.

¹H-NMR (400 MHz, DMSO-d6) δ [ppm]: 1.145 (0.40), 1.380 (13.73), 1.387 (11.86), 1.396 (16.00), 1.817 (0.94), 1.921 (1.30), 1.928 (1.57), 1.936 (1.50), 1.942 (1.57), 1.956 (1.64), 2.336 (0.77), 2.518 (8.18), 2.523 (5.61), 2.678 (0.63), 3.159 (3.17), 3.172 (3.31), 3.296 (2.77), 3.381 (0.47), 3.696 (6.08), 4.037 (1.27), 4.054 (1.64), 4.096 (0.87), 4.110 (0.77), 4.123 (0.57), 4.135 (0.94), 4.144 (1.14), 4.157 (1.40), 4.166 (1.17), 4.420 (0.57), 4.440 (0.57), 4.443 (0.53), 4.462 (0.43), 7.396 (1.40), 8.172 (1.74), 8.182 (1.70), 8.274 (2.87).

LC-MS (method 4); MS (ESlpos): m/z = 308 [M+H]⁺

### Syntheses of Intermediate 3 Compounds

### Intermediate 3-1

### 1-chloro-3-isothiocyanato-2-methoxybenzene

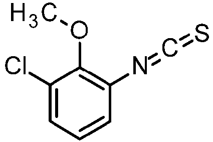

3-chloro-2-methoxyaniline (CAS 51114-68-2, 8.4 ml, 63 mmol) was dissolved in DCM (100 ml) and sat. aqueous sodium bicarbonate solution (100 ml) was added. To the ice cooled mixture was slowly added thiophosgene (5.4 ml, 70 mmol). The reaction was stirred at 0°C for 2 h at RT. The DCM layer was separated and washed with sat. sodium bicarbonate solution, filtered through a hydrophobic filter and concentrated under reduced pressure to give the title compound (12.97 g, 100% yield) which was used directly in the next step.

¹H-NMR (400MHz, DMSO-d₆): δ [ppm]= 7.51 (dd, 1H), 7.35 (dd, 1H), 7.20 (t, 1H), 3.85 - 3.91 (m, 3H).

### Intermediate 3-2

### 1-fluoro-3-isothiocyanato-2-methoxybenzene

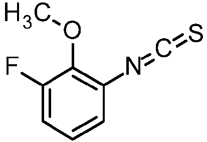

Using an analogous method as described for intermediate 3-1 with 3-fluoro-2-methoxyaniline (CAS 437-83-2, 5.00 g, 35.4 mmol) as the starting material, 6.24 g of the title compound were prepared (96% yield).

¹H-NMR (400MHz, DMSO-d₆): δ [ppm]= 7.32 (m, 1H), 7.10 - 7.19 (m, 2H), 3.96 (d, 3H).

### Intermediate 3-3

### tert-butyl 5-[(3-chloro-2-methoxyphenyl)carbamothioyl]-4-hydroxy-6-oxo-3,6-dihydro-pyridine-1(2H)-carboxylate

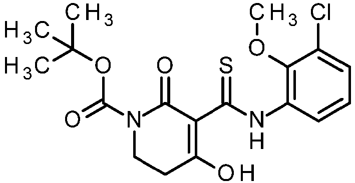

To an ice-cooled solution of 1-chloro-3-isothiocyanato-2-methoxybenzene (intermediate 3-1, 4.00 g, 20.0 mmol) and *tert*-butyl 2,4-dioxopiperidine-1-carboxylate (CAS 845267-78-9, 4.27 g, 20.0 mmol) in acetonitrile (92 ml) was added dropwise DBU (4.5 ml, 30 mmol). The reaction was stirred at RT for 16h. To the reaction mixture was added ice-water (200 mL) and conc. hydrochloric acid (2 mL). The mixture was stirred for 20 min. and extracted with DCM. The organic phase was filtered over a water-repellent filter, concentrated under reduced pressure and purified by flash chromatography (silica, hexane / ethyl acetate gradient 0-50%) to give 6.54 g of the title compound (71 % yield).

¹H-NMR (400MHz, DMSO-*d*₆): δ [ppm]= 13.36 (br s, 1H), 7.73 (d, 1H), 7.47 (dd, 1H), 7.22 (t, 1H), 3.76 - 3.82 (m, 5H), 2.88 (t, 2H), 1.48 (s, 9H).

LC-MS (method 2): Rₜ = 1.49 min; MS (ESlpos): m/z = 413 [M+H]⁺

### Intermediate 3-4

### tert-butyl 5-[(3-fluoro-2-methoxyphenyl)carbamothioyl]-4-hydroxy-6-oxo-3,6-dihydropyridine-1 (2H)-carboxylate

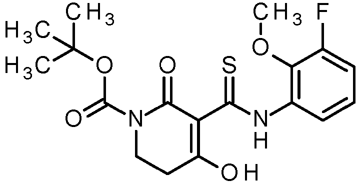

Using an analogous method as described for Intermediate 3-3 with *tert*-butyl 2,4-dioxopiperidine-1-carboxylate (CAS 845267-78-9, 7.26 g, 34.1 mmol) and 1-fluoro-3-isothiocyanato-2-methoxybenzene (intermediate 3-2, 6.24 g, 34.1 mmol) as the starting materials; 9.49 g of the title compound were prepared (67% yield) after stirring the product in MeOH, filtration and drying of the precipitate in vacuum.

¹H-NMR (400 MHz, DMSO-*d*₆): δ [ppm]= 13.37 (br s, 1H), 7.58 (br d, 1H), 7.23 - 7.30 (m, 1H), 7.09 - 7.21 (m, 1H), 4.10 (br s, 1H), 3.78 (t, 2H), 3.17 (s, 3H), 2.88 (br t, 2H), 1.48 (s, 9H).

LC-MS (method 3): Rₜ = 0.66 min; MS (ESlpos): m/z = 397 [M+H]⁺

### Intermediate 3-5

### tert-butyl.5-[(3-chloro-2-methylphenyl)carbamothioyl]-4-hydroxy-6-oxo-3,6-dihydropyridine-1(2H)-carboxylate

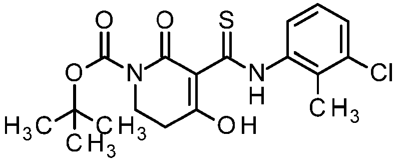

According to the method described for intermediate 3-3 with 1-chloro-3-isothiocyanato-2-methylbenzene (CAS 19241-35-1; 2.50 g, 13.6 mmol) and *tert*-butyl 2,4-dioxopiperidine-1-carboxylate (CAS 845267-78-9, 2.9 g, 13.6 mmol) as the starting materials; 4.68 g of the title compound were prepared (78% yield), after addition of hydrochloric acid ,filtration and drying the precipitate in vacuum at 40°C for 16h.

¹H-NMR (400MHz, DMSO-*d*₆): δ [ppm]= 15.73 (s, 1H), 12.77 (br s, 1H), 7.45 (d, 1H), 7.30 (t, 1H), 7.19 (d, 1H), 3.78 (t, 2H), 2.85 (t, 2H), 2.20 (s, 3H), 1.48 (s, 9H).

LC-MS (method 3): Rₜ = 0.72 min; MS (ESlpos): m/z = 397 [M+H]⁺

### Intermediate 3-6

### tert-butyl 5-[(3-fluoro-2-methylphenyl)carbamothioyl]-4-hydroxy-6-oxo-3,6-dihydropyridine-1(2H)-carboxylate

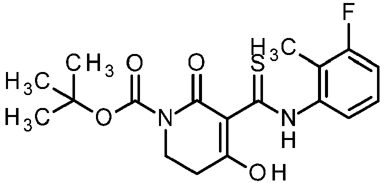

Using an analogous method as described for Intermediate 3-3 with tert-butyl 2,4-dioxopiperidine-1-carboxylate (CAS 845267-78-9, 8.19 g, 38.4 mmol) and 1-fluoro-3-isothiocyanato-2-methylbenzene (CAS 363179-58-2, 6.42 g, 38.4 mmol) as the starting materials; 11.1 g of the title compound were prepared (95% purity, 72% yield) after stirring the product in MeOH, filtration and drying of the precipitate in vacuum.

¹H-NMR (400 MHz, DMSO- d₆): δ [ppm]= 1.479 (16.00), 2.084 (2.80), 2.088 (2.74), 2.834 (0.58), 2.850 (1.12), 2.866 (0.61), 3.768 (0.64), 3.784 (1.16), 3.800 (0.59), 7.073 (0.61), 7.093 (0.71), 7.186 (0.59), 7.299 (0.45), 7.316 (0.42).

### Intermediate 3-7

### N-(4-fluorophenyl)-4-hydroxy-2-oxo-1,2,5,6-tetrahydropyridine-3-carbothioamide

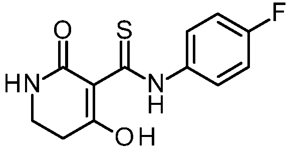

To a mixture of piperidine-2,4-dione (3.69 g, 32.6 mmol) in ACN (20 ml) under argon atmosphere was added TEA (320 µl, 2.3 mmol ) and 1-fluoro-4-isothiocyanatobenzene (1.0 ml, 33 mmol) and stirring continued at 100°C for 2 days. The reaction mixture was extracted 3 times with ethyl acetate, the organic phases werde filtered through a silica plug and the solvents were removed in vacuum. The water phase was extracted with DCM/methanol 10:1. the organic phase was filtered through a silica plug and the solvents were removed in vacuum. The residues were combined and crystallised twice with ethanol to give 2.40 g (28 % yield) of the title compound.

1H-NMR (400 MHz, DMSO-d6) delta [ppm]: 1.037 (1.84), 1.054 (3.57), 1.071 (1.91), 1.173 (0.40), 1.231 (0.44), 1.987 (0.84), 2.323 (0.75), 2.327 (1.09), 2.331 (0.82), 2.596 (7.60), 2.615 (12.96), 2.633 (7.91), 2.665 (2.06), 2.669 (2.15), 2.673 (1.73), 2.748 (7.02), 2.765 (14.29), 2.783 (8.05), 3.232 (1.62), 3.239 (1.90), 3.250 (3.03), 3.257 (3.28), 3.266 (6.18), 3.273 (6.69), 3.283 (9.13), 3.290 (9.35), 3.301 (5.27), 3.308 (5.10), 3.395 (4.50), 3.402 (5.08), 3.414 (7.78), 3.421 (7.98), 3.431 (4.76), 3.438 (4.43), 3.454 (1.35), 3.471 (0.66), 4.330 (0.55), 4.343 (1.06), 4.356 (0.53), 6.853 (4.30), 6.859 (1.84), 6.875 (10.55), 6.891 (2.28), 6.898 (7.93), 6.905 (1.33), 6.934 (6.69), 6.946 (7.27), 6.956 (4.57), 6.963 (2.00), 6.968 (3.85), 7.136 (0.56), 7.158 (0.42), 7.220 (6.07), 7.242 (13.78), 7.253 (9.09), 7.259 (6.63), 7.264 (9.86), 7.275 (16.00), 7.298 (9.11), 7.334 (0.69), 7.346 (0.67), 7.388 (7.49), 7.400 (8.27), 7.410 (7.11), 7.422 (6.34), 7.447 (8.86), 7.459 (9.55), 7.469 (8.04), 7.481 (7.05), 7.531 (2.35), 7.568 (0.62), 7.591 (0.47), 8.143 (5.14), 9.297 (3.92), 13.353 (3.10), 14.184 (7.74), 14.497 (7.23), 16.441 (13.39), 16.505 (8.80).

### Intermediate 3-8

### N-(3-chloro-2-methoxyphenyl)-8-hydroxy-6-oxo-5-azaspiro[3.5]non-7-ene-7-carbothioamide

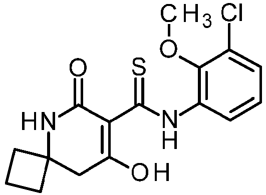

Using an analogous method as described for Intermediate 3-3 with 1-chloro-3-isothiocyanato-2-methoxybenzene (4.47 g, 22.4 mmol, intermediate 3-1) and 5-azaspiro[3.5]nonane-6,8-dione (3.43 g, 22.4 mmol, CAS 1105665-46-0) as the starting materials. Hydrochloric acid (2 mol/l) was added while cooling with an ice bath, until no more precipitate was formed. The mixture was stirred for 30min at RT, the precipitate was filtered off, washed with water and dried in a vacuum oven at 40°C for 16h to give 6.89 g (95 % purity, 83 % yield) of the title compound.

¹H-NMR (400 MHz, DMSO-d6) δ [ppm]: 1.715 (0.55), 1.737 (0.71), 1.761 (0.99), 1.782 (0.90), 1.805 (0.51), 2.024 (0.60), 2.033 (0.77), 2.040 (0.71), 2.055 (1.59), 2.065 (1.21), 2.074 (2.07), 2.085 (0.73), 2.133 (0.42), 2.157 (1.04), 2.162 (0.75), 2.181 (0.72), 2.187 (0.74), 2.205 (0.52), 2.210 (0.50), 2.228 (0.91), 2.234 (0.70), 2.252 (0.68), 2.258 (0.72), 2.518 (1.18), 2.523 (0.80), 2.862 (4.15), 3.001 (4.20), 3.740 (15.84), 3.747 (16.00), 7.153 (0.97), 7.173 (2.14), 7.185 (1.09), 7.193 (1.26), 7.206 (2.23), 7.226 (1.23), 7.379 (1.24), 7.383 (1.32), 7.399 (1.08), 7.403 (1.04), 7.421 (1.31), 7.425 (1.41), 7.441 (1.15), 7.445 (1.11), 7.803 (0.97), 7.806 (1.00), 7.824 (0.93), 7.827 (0.88), 7.860 (1.04), 7.863 (1.04), 7.880 (0.98), 7.883 (0.93), 8.670 (1.36), 9.774 (1.11), 14.249 (1.63), 14.703 (1.41), 16.499 (10.89).

LC-MS (method 1): Rₜ = 1.32 min; MS (ESlpos): m/z = 352 [M+H]⁺

### Intermediate 3-9

### N-(3-chloro-2-methoxyphenyl)-4-hydroxy-6,6-dimethyl-2-oxo-1,2,5,6-tetrahydropyridine-3-carbothioamide

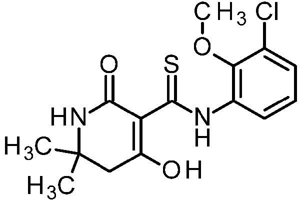

Using an analogous method as described for Intermediate 3-3 with 1-chloro-3-isothiocyanato-2-methoxybenzene (1.40 g, 7.01 mmol, intermediate 3-1) and 6,6-dimethylpiperidine-2,4-dione (990 mg, 7.01 mmol, CAS 5239-39-4) as the starting materials, which were stirred 30 min at 0°C and 1,5 h at RT. The reaction mixture was quenched with 100ml water, 1ml conc. hydrochloric acid was added and stirred at RT for 20min. The mixture was extracted with ethyl acetate twice and purified by column chromatography (silica, hexane / ethyl acetate 5-50%) to give 1.11 g (95 % purity, 44 % yield) of the title compound.

1H-NMR (400 MHz, DMSO-d6) delta [ppm]: 1.254 (11.15), 1.275 (15.43), 1.988 (0.55), 2.518 (1.79), 2.523 (1.25), 2.643 (4.58), 2.788 (3.15), 3.740 (16.00), 3.747 (10.69), 7.154 (0.99), 7.174 (2.19), 7.188 (0.75), 7.195 (1.28), 7.209 (1.47), 7.229 (0.82), 7.377 (1.26), 7.380 (1.35), 7.397 (1.08), 7.400 (1.06), 7.420 (0.87), 7.424 (0.92), 7.440 (0.75), 7.444 (0.73), 7.816 (1.06), 7.819 (1.06), 7.836 (1.01), 7.839 (0.96), 7.874 (0.73), 7.877 (0.74), 7.894 (0.69), 7.898 (0.67), 8.248 (0.97), 9.385 (1.17), 14.274 (1.74), 14.840 (1.02), 16.443 (5.11), 16.509 (3.62)

LC-MS (method 5): Rₜ = 1.3 min; MS (ESlpos): m/z = 340 [M+H]⁺

### Intermediate 3-10

### 1-(difluoromethoxy)-2-isothiocyanatobenzene

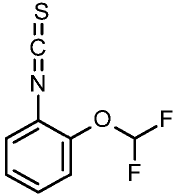

To an ice-cooled biphasic system of 2-(difluoromethoxy)aniline (25.0 g, 157 mmol) in DCM (20 ml) and sat. NaHCO₃ (20 ml) was added slowly dropwise thiophosgene (13 ml, 160 mmol). The reaction was stirred at 0°C for 2h. TLC (ethyl acetate:hexane) showed complete conversion. The DCM layer was separated and washed with sat. sodium hydrogen carbonate , brine, filtered through a hydrophobic filter and concentrated to give the title compound (29.95 g, 95%) which was used directly in the next step.

¹H-NMR (400MHz, DMSO-*d*₆): δ [ppm]= 7.09 - 7.64 (m, 1H).

### Intermediate 3-11

### tert-butyl 5-{[2-(difluoromethoxy)phenyl]carbamothioyl}-4-hydroxy-6-oxo-3,6-dihydropyridine-1 (2H)-carboxylate

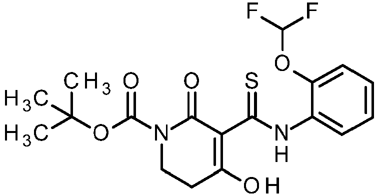

To an ice-cooled mixture of tert-butyl 2,4-dioxopiperidine-1-carboxylate (10.6 g, 49.7 mmol) and 1-(difluoromethoxy)-2-isothiocyanatobenzene (intermediate 3-10, 10.0 g, 49.7 mmol) in MeCN (100 ml) was added DBU (11 ml, 75 mmol) slowly. The reaction was stirred at 0°C for 16h. The reaction mixture was concentrated to half of the volume and the reaction mixture added to 1M hydrochloric acid (500ml) and extracted with ethyl acetate. The organics were combined and concentrated in vacuo. The residue was purified by silica chromatography (ethyl acetate: hexane), to give the title compound 16.4 g (80 % yield).

¹H -NMR (400MHz, DMSO-d6): Shift [ppm]= 13.32 (br s, 1H), 7.67 (d, 1H), 6.99 - 7.47 (m, 4H), 3.75 - 3.77 (m, 1H), 3.72 - 3.88 (m, 1H), 2.89 (br t, 2H), 1.48 (s, 9H).

### Syntheses of intermediate 4 compounds

### Intermediate 4-1

### N-(3-chloro-2-methoxyphenyl)-4-hydroxy-2-oxo-1,2,5,6-tetrahydropyridine-3-carbothioamide

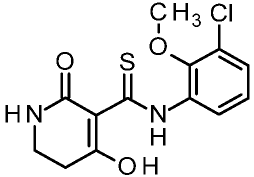

To a solution of *tert*-butyl 5-[(3-chloro-2-methoxyphenyl)carbamothioyl]-4-hydroxy-6-oxo-3,6-dihydropyridine-1 (2H)-carboxylate (intermediate 3-3, 6.54 g, 15.8 mmol) in dichloromethane (94 ml) was added TFA (12 ml, 160 mmol) and the mixture was stirred 1.5 h at RT. The reaction mixture was concentrated under reduced pressure and the residue was dissolved in ethyl acetate and washed with sat. aqueous sodium bicarbonate solution and brine. The organic layer was filtered through a hydrophobic filter and the filtrate was dried to dryness. The residue was purified by flash chromatography (silica, hexane / ethyl acetate gradient 20-100%) to give 4.06 g of the title compound (78% yield).

¹H-NMR (400 MHz, DMSO-*d*₆): δ [ppm]= 16.45 (d, 1H), 14.69 (s, 1H), 14.33 (s, 1H), 9.37 (br s, 1H), 8.18 (br s, 1H), 7.76 - 7.87 (m, 1H), 7.37 - 7.45 (m, 1H), 7.15 - 7.23 (m, 1H), 3.73 - 3.76 (m, 3H), 3.43 (td, 1H), 3.27 - 3.32 (m, 1H), 2.79 (t, 1H), 2.59 - 2.69 (m, 1H).

LC-MS (method 2): Rₜ = 1.19 min; MS (ESlpos): m/z = 313 [M+H]⁺

### Intermediate 4-2

### N-(3-fluoro-2-methoxyphenyl)-4-hydroxy-2-oxo-1,2,5,6-tetrahydropyridine-3-carbothioamide

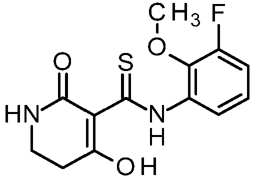

Using an analogous method as described for intermediate 4-1 with *tert*-butyl 5-[(3-fluoro-2-methoxyphenyl)carbamothioyl]-4-hydroxy-6-oxo-3,6-dihydropyridine-1(2*H*)-carboxylate (intermediate 3-4 , 9.49 g, 23.9 mmol) as the starting material, 6.98 g of the title compound was prepared (89% yield) after 15 min of stirring and used in the next steps without further purification.

¹H-NMR (400 MHz, DMSO-*d*₆): δ [ppm]= 16.48 (d, 1H), 14.63 (s, 0.5H), 14.28 (s, 0.5H), 9.34 (br s, 0.5H), 8.16 (br s, 0.5H), 7.65 (t, 1H), 6.97 - 7.37 (m, 2H), 3.79 - 3.85 (m, 3H), 3.35 - 3.46 (m, 1H), 3.26 - 3.32 (m, 1H), 2.78 (t, 1H), 2.63 (t, 1H).

LC-MS (method 3): Rₜ = 0.46 min; MS (ESlpos): m/z = 297 [M+H]⁺

### Intermediate 4-3

### N-(3-chloro-2-methylphenyl)-4-hydroxy-2-oxo-1,2,5,6-tetrahydropyridine-3-carbothioamide

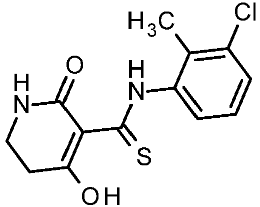

Using an analogous method as described for intermediate 4-1 with *tert*-butyl 5-[(3-chloro-2-methylphenyl)carbamothioyl]-4-hydroxy-6-oxo-3,6-dihydropyridine-1(2*H*)-carboxylate (intermediate 3-5, 4.67 g, 11.8 mmol) as the starting material, 3.54 g of the title compound were prepared (91% yield) after 3 h stirring and used in the next steps without further purification.

¹H-NMR (400 MHz, DMSO-*d*₆): δ [ppm]= 16.42 (d, 1H), 14.01 - 14.37 (m, 1H), 8.14 - 9.40 (m, 1H), 7.43 (br t, 1H), 7.16 - 7.32 (m, 2H), 3.42 - 3.48 (m, 1H), 3.26 - 3.34 (m, 1H), 2.78 (t, 1H), 2.60 - 2.68 (m, 1H), 2.12 - 2.21 (m, 3H).

LC-MS (method 3): Rₜ = 0.60 min; MS (ESlpos): m/z = 297 [M+H]⁺.

### Intermediate 4-4

### N-(3-fluoro-2-methylphenyl)-4-hydroxy-2-oxo-1,2,5,6-tetrahydropyridine-3-carbothioamide

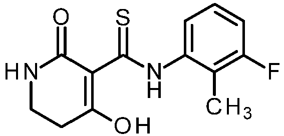

Using an analogous method as described for intermediate 4-1 with *tert*-butyl 5-[(3-fluoro-2-methylphenyl)carbamothioyl]-4-hydroxy-6-oxo-3,6-dihydropyridine-1(2H)-carboxylate (intermediate 3-6, 11.1 g, 29.1 mmol) as the starting material, 7.25 g of the title compound was prepared (84% yield) after 15 min of stirring and used in the next step without further purification.

¹H-NMR (400 MHz, DMSO- *d*₆): δ [ppm]= 1.172 (0.55), 1.987 (1.05), 2.063 (16.00), 2.518 (1.49), 2.523 (1.01), 2.612 (1.94), 2.631 (3.67), 2.649 (2.11), 2.761 (1.99), 2.779 (4.22), 2.798 (2.26), 3.280 (1.36), 3.287 (1.47), 3.298 (2.58), 3.305 (2.57), 3.315 (1.39), 3.322 (1.33), 3.410 (1.40), 3.417 (1.48), 3.428 (2.27), 3.435 (2.22), 3.446 (1.27), 3.454 (1.15), 7.112 (1.56), 7.119 (0.96), 7.132 (2.25), 7.141 (3.30), 7.161 (2.66), 7.168 (2.15), 7.192 (1.12), 7.243 (0.73), 7.262 (1.15), 7.272 (0.99), 7.279 (1.25), 7.292 (1.27), 7.308 (1.21), 7.328 (0.45), 8.150 (1.43), 9.317 (1.16), 14.003 (2.32), 14.321 (2.05), 16.439 (5.35), 16.468 (4.62).

LC-MS (method 3): Rₜ = 0.47 min; MS (ESlpos): m/z = 281 [M+H]⁺.

### Intermediate 4-5

### N-[2-(difluoromethoxy)phenyl]-4-hydroxy-2-oxo-1,2,5,6-tetrahydropyridine-3-carbothioamide

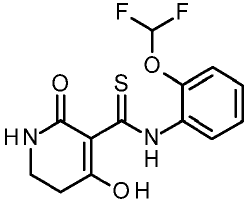

To an ice-cooled solution of tert-butyl 5-{[2-(difluoromethoxy)phenyl]carbamothioyl}-4-hydroxy-6-oxo-3,6-dihydropyridine-1(2H)-carboxylate (intermediate 3-11, 16.4 g, 39.6 mmol) in DCM (30 ml) was added TFA (30 ml) and stirred for 15 min and allowed to warm to RT. The reaction mixture was poured onto a sat. aqueous sodium hydrogen carbonate solution and extracted with DCM. The organics were combined and filtered through a hydrophobic filter and concentrated to give the title compound (12.43g, 95%).

¹H-NMR (400MHz, DMSO-d₆): δ [ppm]= 14.04 - 14.82 (m, 1H), 8.05 - 9.60 (m, 1H), 7.74 (t, 1H), 6.88 - 7.50 (m, 5H), 3.43 (td, 1H), 3.26 - 3.32 (m, 1H), 2.78 (t, 1H), 2.58 - 2.68 (m, 1H).

### Syntheses of intermediate 5 compounds

### Intermediate 5-1

### tert-butyl [2-({4-[({5-[(3-chloro-2-methoxyphenyl)carbamothioyl]-6-oxo-1,2,3,6-tetrahydropyridin-4-yl}amino)methyl]pyridin-3-yl}oxy)ethyl]carbamate

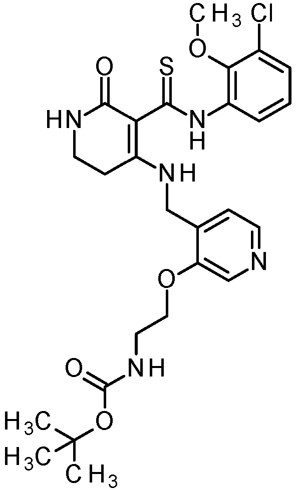

N-(3-chloro-2-methoxyphenyl)-4-hydroxy-2-oxo-1,2,5,6-tetrahydropyridine-3-carbothioamide (400 mg, 1.28 mmol, intermediate 4-1) and tert-butyl (2-{[4-(aminomethyl)pyridin-3-yl]oxy}ethyl)carbamate (444 mg, 1.66 mmol, intermediate 2-1) were mixed in 5.6 ml acetonitrile, *N,O*-bis(trimethylsilyl)acetamide (790 µl, 3.2 mmol, CAS 10416-59-8) was added and the reaction mixture was stirred at 80°C for 16h. After purification by column chromatography (silica, DCM /ethanol gradient 0-10%) 830 mg (80 % purity, 92 % yield) of the title compound were obtained.

¹H-NMR (400 MHz, DMSO-d6) δ [ppm]: 0.000 (2.04), 1.097 (0.66), 1.145 (2.14), 1.163 (4.35), 1.180 (2.19), 1.344 (1.04), 1.362 (16.00), 1.368 (4.52), 1.404 (0.40), 1.741 (7.55), 1.905 (0.68), 1.978 (7.90), 2.317 (0.41), 2.735 (0.49), 2.750 (0.88), 2.767 (0.51), 3.150 (0.73), 3.353 (0.99), 3.705 (7.13), 3.711 (1.22), 3.990 (0.62), 4.007 (1.84), 4.025 (1.80), 4.043 (0.63), 4.116 (0.50), 4.129 (0.95), 4.142 (0.52), 4.677 (0.94), 4.693 (0.95), 7.081 (0.73), 7.101 (1.18), 7.122 (0.66), 7.278 (1.31), 7.282 (1.22), 7.299 (0.81), 7.302 (0.76), 7.732 (0.60), 7.793 (0.69), 7.797 (0.66), 7.814 (0.67), 8.216 (0.89), 8.228 (0.88), 8.354 (1.08), 13.713 (0.41), 14.784 (1.08).

LC-MS (method 1): Rₜ = 1.1 min; MS (ESlpos): m/z = 562 [M+H]⁺.

### Intermediate 5-2

### tert-butyl [2-({4-[({5-[(3-chloro-2-methylphenyl)carbamothioyl]-6-oxo-1,2,3,6-tetrahydropyridin-4-yl}amino)methyl]pyridin-3-yl}oxy)ethyl]carbamate

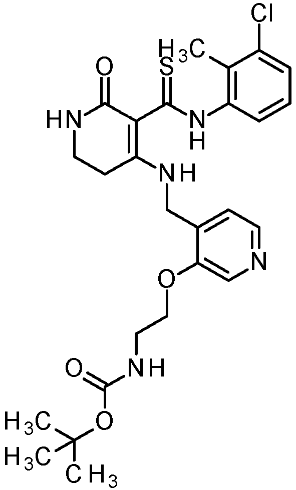

A mixture of N-(3-chloro-2-methylphenyl)-4-hydroxy-2-oxo-1,2,5,6-tetrahydropyridine-3-carbothioamide (400 mg, 1.42 mmol, intermediate 4-3) and tert-butyl (2-{[4-(aminomethyl)pyridin-3-yl]oxy}ethyl)carbamate (493 mg, 1.85 mmol, intermediate 2-1) in ACN (6.2 ml) was treated with *N,O*-bis(trimethylsilyl)acetamide (880 µl, 3.5 mmol, CAS 10416-59-8) and stirred at 80°C for 16h. The formed precipitate was filtered off and dried under reduced pressure to give 534 mg (94 % purity, 65 % yield) of the title compound.

¹H-NMR (400 MHz, DMSO-d6) δ [ppm]: 1.361 (16.00), 2.065 (13.97), 2.151 (5.15), 2.508 (0.76), 2.513 (0.48), 2.723 (0.47), 2.740 (0.89), 2.757 (0.51), 3.154 (0.65), 3.160 (0.62), 3.345 (0.75), 4.108 (0.50), 4.122 (0.98), 4.135 (0.51), 4.658 (1.00), 4.673 (0.99), 7.166 (0.82), 7.170 (0.77), 7.190 (0.60), 7.210 (0.90), 7.262 (0.63), 7.274 (0.66), 7.318 (0.73), 7.321 (0.74), 7.337 (0.60), 7.341 (0.56), 7.687 (0.57), 8.209 (1.18), 8.221 (1.12), 8.346 (1.28), 14.533 (0.87

LC-MS (method 1): Rₜ = 1.12 min; MS (ESlpos): m/z = 546 [M+H]⁺

### Intermediate 5-3

### tert-butyl [2-({4-[({5-[(3-fluoro-2-methylphenyl)carbamothioyl]-6-oxo-1,2,3,6-tetrahydropyridin-4-yl}amino)methyl]pyridin-3-yl}oxy)ethyl]carbamate

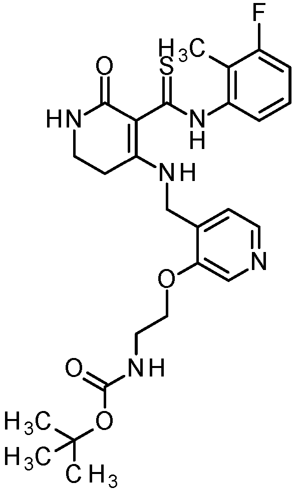

A mixture of N-(3-fluoro-2-methylphenyl)-4-hydroxy-2-oxo-1,2,5,6-tetrahydropyridine-3-carbothioamide (400 mg, 1.43 mmol, intermediate 4-4) tert-butyl (2-{[4-(aminomethyl)pyridin-3-yl]oxy}ethyl)carbamate (496 mg, 1.86 mmol, intermediate 2-1) in ACN (6.2 ml) was treated with *N,O*-bis(trimethylsilyl)acetamide (880 µl, 3.5 mmol, CAS 10416-59-8) and stirred at 80°C for 16h. The formed precipitate was filtered off and dried under reduced pressure to give 362 mg (98 % purity, 47 % yield)of the title compound.

¹H-NMR (400 MHz, DMSO-d6) δ [ppm]: 1.052 (0.65), 1.370 (16.00), 2.043 (2.76), 2.048 (2.73), 2.522 (0.80), 2.731 (0.46), 2.748 (0.90), 2.764 (0.53), 3.161 (0.65), 3.167 (0.62), 3.355 (0.84), 4.118 (0.50), 4.132 (0.99), 4.145 (0.53), 4.669 (1.01), 4.683 (1.02), 7.061 (0.77), 7.075 (0.97), 7.094 (0.94), 7.208 (0.45), 7.224 (0.43), 7.272 (0.64), 7.284 (0.68), 7.696 (0.57), 8.220 (1.23), 8.231 (1.16), 8.355 (1.34), 14.520 (0.84).

LC-MS (method 1): Rₜ = 1.04 min; MS (ESlpos): m/z = 530 [M+H]⁺.

### Intermediate 5-4

### tert-butyl [3-({4-[({5-[(3-chloro-2-methoxyphenyl)carbamothioyl]-6-oxo-1,2,3,6-tetrahydropyridin-4-yl}amino)methyl]pyridin-3-yl}oxy)propyl]carbamate

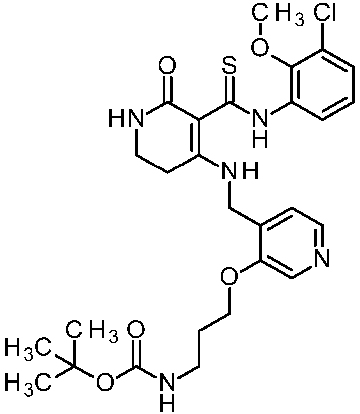

N-(3-chloro-2-methoxyphenyl)-4-hydroxy-2-oxo-1,2,5,6-tetrahydropyridine-3-carbothioamide (400 mg, 1.28 mmol, intermediate 4-1) and tert-butyl (3-{[4-(aminomethyl)pyridin-3-yl]oxy}propyl)carbamate (468 mg, 1.66 mmol) intermediate 2-2) were mixed in ACN (5.6ml), *N,O*-bis(trimethylsilyl)acetamide (790 µl, 3.2 mmol, CAS 10416-59-8) was added and the reaction mixture was stirred at 80°C for 16h. After purification by column chromatography (silica, DCM /ethanol gradient 0-10%) 760 mg (93 % purity, 96 % yield) of the title compound were obtained.

¹H-NMR (400 MHz, DMSO-d6) δ [ppm]: 0.000 (0.62), 1.162 (0.48), 1.330 (16.00), 1.348 (0.95), 1.845 (0.51), 1.861 (0.76), 1.876 (0.53), 1.978 (0.88), 2.064 (1.06), 2.508 (1.61), 2.513 (0.99), 2.743 (0.48), 2.759 (0.99), 2.776 (0.58), 3.113 (0.79), 3.128 (1.05), 3.142 (0.90), 3.703 (10.23), 4.128 (0.57), 4.143 (1.15), 4.158 (0.56), 4.667 (0.86), 4.681 (0.82), 5.748 (12.98), 6.895 (0.43), 7.080 (0.70), 7.100 (1.49), 7.120 (0.88), 7.272 (0.73), 7.277 (1.26), 7.281 (1.41), 7.297 (0.86), 7.301 (0.82), 7.719 (0.70), 7.789 (0.70), 7.792 (0.70), 7.809 (0.68), 7.812 (0.63), 8.206 (1.40), 8.218 (1.39), 8.338 (1.70), 13.702 (0.51), 14.779 (1.25).

LC-MS (method 1): Rₜ = 1.11 min; MS (ESlpos): m/z = 576 [M+H]⁺.

### Intermediate 5-5

### tert-butyl [2-({4-[({5-[(3-chloro-2-methoxyphenyl)carbamothioyl]-6-oxo-1,2,3,6-tetrahydropyridin-4-yl}amino)methyl]pyridin-3-yl}oxy)ethyl]methylcarbamate

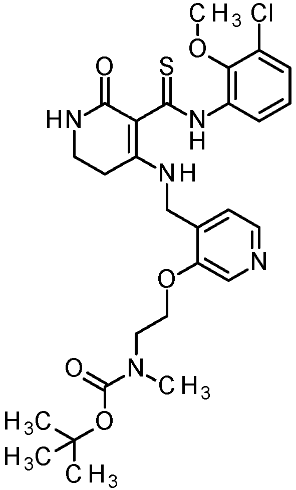

A mixture of N-(3-chloro-2-methoxyphenyl)-4-hydroxy-2-oxo-1,2,5,6-tetrahydropyridine-3-carbothioamide (400 mg, 90 % purity, 1.15 mmol, intermediate 4-1) and tert-butyl (2-{[4-(aminomethyl)pyridin-3-yl]oxy}ethyl)methylcarbamate (421 mg, 1.50 mmol) intermediate 2-3) in ACN (5 ml) was treated with N,O-bis(trimethylsilyl)acetamide (710 µl, 2.9 mmol, CAS 10416-59-8) and stirred at 80°C for 16h. The formed precipitate was filtered off and dried under reduced pressure to give 630 mg (95 % purity, 90 % yield)the title compound.

¹H-NMR (400 MHz, DMSO-d6) δ [ppm]: 1.339 (3.30), 1.383 (3.05), 2.074 (16.00), 2.518 (1.05), 2.522 (0.66), 2.731 (0.63), 2.748 (1.27), 2.765 (0.74), 2.878 (1.46), 2.894 (1.35), 3.143 (0.56), 3.152 (0.87), 3.158 (0.84), 3.579 (0.72), 3.592 (1.42), 3.605 (0.72), 3.712 (12.81), 4.273 (0.67), 4.631 (1.11), 4.645 (1.10), 7.088 (0.96), 7.109 (2.07), 7.129 (1.18), 7.287 (1.39), 7.291 (1.47), 7.307 (1.27), 7.311 (1.24), 7.737 (0.88), 7.799 (0.82), 7.802 (0.82), 7.819 (0.78), 7.823 (0.74), 14.789 (1.46).

LC-MS (method 1): Rₜ = 1.2 min; MS (ESlpos): m/z = 576 [M+H]⁺.

### Intermediate 5-6

### tert-butyl [2-({4-[({5-[(3-fluoro-2-methoxyphenyl)carbamothioyl]-6-oxo-1,2,3,6-tetrahydropyridin-4-yl}amino)methyl]pyridin-3-yl}oxy)ethyl]methylcarbamate

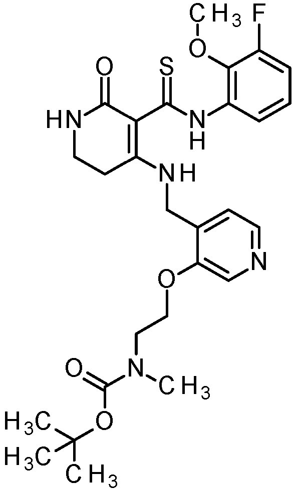

N-(3-fluoro-2-methoxyphenyl)-4-hydroxy-2-oxo-1,2,5,6-tetrahydropyridine-3-carbothioamide (300 mg, 1.01 mmol, intermediate 4-2) and tert-butyl (2-{[4-(aminomethyl)pyridin-3-yl]oxy}ethyl)methylcarbamate (370 mg, 1.32 mmol, intermediate 2-3) were mixed in ACN (4.4ml), *N,O*-bis(trimethylsilyl)acetamide (630 µl, 2.5 mmol, CAS 10416-59-8) was added and the reaction mixture was stirred at 80°C for 16h. After purification by column chromatography (silica, DCM /ethanol gradient 0-10%) 505 mg (98 % purity, 87 % yield) of the title compound were obtained.

¹H-NMR (400 MHz, DMSO-d6) δ [ppm]: 0.253 (0.60), 1.340 (7.50), 1.383 (6.89), 2.074 (0.52), 2.332 (0.47), 2.518 (2.87), 2.522 (1.79), 2.673 (0.53), 2.721 (1.44), 2.737 (2.86), 2.755 (1.65), 2.879 (3.34), 2.895 (3.08), 3.137 (1.27), 3.147 (1.97), 3.153 (1.89), 3.580 (1.63), 3.593 (3.19), 3.606 (1.63), 3.782 (16.00), 3.784 (15.85), 4.271 (1.51), 4.630 (2.86), 4.645 (2.85), 7.029 (0.57), 7.045 (0.78), 7.050 (1.70), 7.066 (1.95), 7.069 (2.33), 7.072 (2.11), 7.078 (2.15), 7.086 (1.79), 7.093 (0.78), 7.100 (1.76), 7.104 (1.85), 7.121 (0.75), 7.125 (0.59), 7.316 (0.79), 7.657 (1.61), 7.677 (1.40), 7.714 (1.97), 8.267 (0.49), 8.431 (0.40), 13.725 (0.82), 14.737 (3.28).

LC-MS (method 1): Rₜ = 1.13 min; MS (ESlpos): m/z = 560 [M+H]⁺

### Intermediate 5-7

### tert-butyl [2-({4-[({5-[(4-fluorophenyl)carbamothioyl]-6-oxo-1,2,3,6-tetrahydropyridin-4-yl}amino)methyl]pyridin-3-yl}oxy)ethyl]methylcarbamate

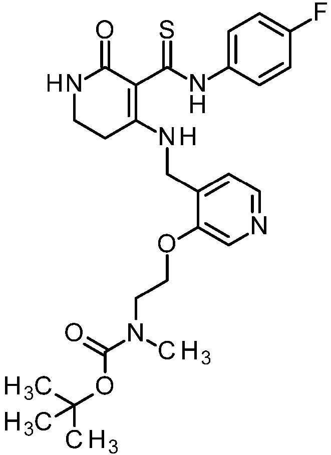

N-(4-fluorophenyl)-4-hydroxy-2-oxo-1,2,5,6-tetrahydropyridine-3-carbothioamide (400 mg, 1.62 mmol, intermediate 3-7) and tert-butyl (2-{[4-(aminomethyl)pyridin-3-yl]oxy}ethyl)methylcarbamate (592 mg, 2.10 mmol, intermediate 2-3) were mixed in ACN (7ml), *N,O*-bis(trimethylsilyl)acetamide (1000 µl, 4.0 mmol, CAS 10416-59-8) was added and the reaction mixture was stirred at 80°C for 16h. After purification by column chromatography (silica, DCM /ethanol gradient 0-5%) 517 mg (85 % purity, 53 % yield) of the title compound were obtained.

¹H-NMR (400 MHz, DMSO-d6) δ [ppm]: 1.035 (1.36), 1.053 (2.71), 1.070 (1.35), 1.340 (2.92), 1.384 (2.74), 1.751 (16.00), 2.518 (1.11), 2.523 (0.73), 2.698 (0.54), 2.715 (1.06), 2.731 (0.62), 2.878 (1.31), 2.893 (1.21), 3.126 (0.49), 3.135 (0.77), 3.141 (0.75), 3.422 (0.65), 3.435 (0.66), 3.440 (0.64), 3.452 (0.66), 3.578 (0.66), 3.591 (1.30), 3.604 (0.67), 4.278 (0.59), 4.343 (0.41), 4.356 (0.77), 4.622 (1.18), 4.637 (1.20), 7.163 (1.19), 7.169 (0.41), 7.180 (0.50), 7.185 (2.39), 7.190 (0.51), 7.202 (0.46), 7.207 (1.44), 7.289 (0.62), 7.385 (1.12), 7.398 (1.19), 7.403 (0.65), 7.407 (1.02), 7.420 (0.91), 7.703 (0.71), 14.702 (1.04).

LC-MS (method 1): Rₜ = 1.1 min; MS (ESlpos): m/z = 530 [M+H]⁺

### Intermediate 5-8

### tert-butyl [2-({4-[({7-[(3-chloro-2-methoxyphenyl)carbamothioyl]-6-oxo-5-azaspiro[3.5]non-7-en-8-yl}amino)methyl]pyridin-3-yl}oxy)ethyl]methylcarbamate

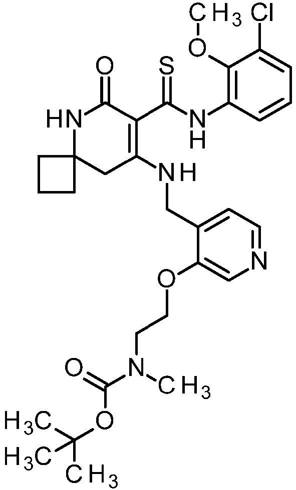

N-(3-chloro-2-methoxyphenyl)-8-hydroxy-6-oxo-5-azaspiro[3.5]non-7-ene-7-carbothioamide (400 mg, 1.13 mmol, intermediate 3-8) and tert-butyl (2-{[4-(aminomethyl)pyridin-3-yl]oxy}ethyl)methylcarbamate (415 mg, 1.47 mmol, intermediate 2-3) were mixed in ACN (4.9m), N,O-bis(trimethylsilyl)acetamide (700 µl, 2.8 mmol, CAS 10416-59-8) was added and the reaction mixture was stirred at 80°C for 16h. The formed precipitate was filtered of, the filtrate was purified by column chromatography (silica, DCM /ethanol gradient 0-5%) to give 731mg (85 % purity, 89 % yield) of the title compound.

H-NMR (400 MHz, DMSO-d6) δ [ppm]: 0.884 (1.20), 0.901 (2.44), 0.920 (1.26), 1.035 (1.24), 1.052 (2.43), 1.070 (1.30), 1.344 (5.27), 1.386 (4.89), 1.583 (0.45), 1.603 (0.47), 1.623 (0.54), 1.874 (0.88), 2.037 (0.41), 2.065 (4.85), 2.091 (1.01), 2.326 (0.44), 2.420 (0.87), 2.438 (0.87), 2.522 (1.66), 2.668 (0.48), 2.891 (2.25), 2.911 (2.47), 2.922 (3.42), 3.422 (0.60), 3.435 (0.65), 3.439 (0.63), 3.452 (0.62), 3.601 (1.21), 3.614 (2.45), 3.627 (1.24), 3.709 (16.00), 4.301 (1.08), 4.344 (0.43), 4.356 (0.79), 4.706 (2.01), 4.721 (2.03), 5.758 (14.52), 7.087 (1.19), 7.107 (2.54), 7.127 (1.46), 7.285 (1.64), 7.289 (1.70), 7.305 (1.37), 7.309 (1.31), 7.362 (0.66), 7.843 (1.30), 7.846 (1.36), 7.863 (1.27), 7.866 (1.22), 8.164 (2.30), 8.251 (1.08), 8.261 (1.03), 8.417 (0.58), 8.437 (0.62), 13.890 (0.53), 14.853 (1.99).

LC-MS (method 1): Rₜ = 1.32 min; MS (ESlpos): m/z = 616 [M+H]⁺

### Intermediate 5-9

### tert-butyl [2-({4-[({5-[(3-chloro-2-methoxyphenyl)carbamothioyl]-2,2-dimethyl-6-oxo-1,2,3,6-tetrahydropyridin-4-yl}amino)methyl]pyridin-3-yl}oxy)ethyl]methylcarbamate

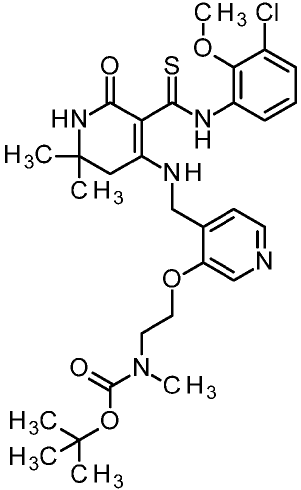

N-(3-chloro-2-methoxyphenyl)-4-hydroxy-6,6-dimethyl-2-oxo-1,2,5,6-tetrahydropyridine-3-carbothioamide (400 mg, 1.17 mmol, intermediate 3-9) and tert-butyl (2-{[4-(aminomethyl)pyridin-3-yl]oxy}ethyl)methylcarbamate (429 mg, 1.53 mmol, intermediate 2-3) were mixed in ACN (5.1ml), *N,O*-bis(trimethylsilyl)acetamide (730 µl, 2.9 mmol, CAS 10416-59-8) was added and the reaction mixture was stirred at 80°C for 16h. Acetonitrile was evaporated in vacuum, the residue was purified by by column chromatography (silica, DCM /ethanol gradient 0-5%) 673 mg (90 % purity, 85 % yield)of the title compound were obtained.

H-NMR (400 MHz, DMSO-d6) δ [ppm]: 0.884 (0.85), 0.901 (1.74), 0.920 (0.98), 1.035 (8.94), 1.052 (16.00), 1.070 (8.98), 1.154 (9.02), 1.341 (3.91), 1.386 (3.68), 2.065 (2.50), 2.420 (0.51), 2.438 (0.54), 2.518 (2.18), 2.522 (1.50), 2.733 (2.18), 2.879 (1.59), 2.898 (1.50), 3.404 (1.49), 3.417 (1.59), 3.422 (3.80), 3.435 (4.11), 3.439 (4.24), 3.452 (4.29), 3.457 (1.43), 3.469 (1.46), 3.588 (0.85), 3.601 (1.69), 3.615 (0.87), 3.711 (15.40), 4.282 (0.77), 4.344 (2.49), 4.357 (4.86), 4.370 (2.47), 4.651 (1.56), 4.666 (1.57), 5.758 (0.63), 7.087 (1.05), 7.108 (2.21), 7.128 (1.30), 7.283 (1.76), 7.287 (1.86), 7.303 (1.69), 7.307 (1.54), 7.730 (1.79), 7.849 (1.08), 7.853 (1.08), 7.870 (1.07), 7.873 (0.97), 8.238 (0.86), 8.249 (0.84), 8.398 (0.45), 8.422 (0.48), 13.921 (0.43), 14.983 (1.83).

LC-MS (method 1): Rₜ = 1.28 min; MS (ESlpos): m/z = 604 [M+H]⁺

### Intermediate 5-10

### tert-butyl (2S)-2-[({4-[({5-[(3-chloro-2-methoxyphenyl)carbamothioyl]-6-oxo-1,2,3,6-tetrahydropyridin-4-yl}amino)methyl]pyridin-3-yl}oxy)methyl]pyrrolidine-1-carboxylate

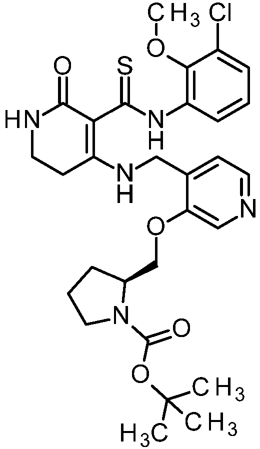

N-(3-chloro-2-methoxyphenyl)-4-hydroxy-2-oxo-1,2,5,6-tetrahydropyridine-3-carbothioamide (400 mg, 90 % purity, 1.15 mmol, intermediate 4-1) and tert-butyl (2S)-2-({[4-(aminomethyl)pyridin-3-yl]oxy}methyl)pyrrolidine-1-carboxylate (460 mg, 1.50 mmol, intermediate 2-4) were heated to 120°C for 90 min. The reaction mixture was purified by column chromatography (silica-aminophase, gradient ethyl acetate/ ethanol 0-1% to give 507 mg (76 % purity, 56 % yield) of the desired compound.

¹H-NMR (400 MHz, DMSO-d6) δ [ppm]: 1.154 (0.60), 1.172 (1.23), 1.190 (0.62), 1.232 (0.48), 1.379 (7.74), 1.393 (9.81), 1.417 (1.01), 1.789 (0.58), 1.798 (0.60), 1.807 (0.60), 1.815 (0.52), 1.820 (0.40), 1.907 (0.56), 1.917 (0.68), 1.921 (0.78), 1.927 (0.94), 1.936 (0.88), 1.941 (1.17), 1.948 (1.17), 1.955 (1.21), 1.987 (2.69), 2.336 (0.44), 2.518 (5.63), 2.522 (3.62), 2.678 (0.42), 2.752 (0.70), 2.768 (0.86), 3.053 (0.52), 3.141 (0.54), 3.148 (0.64), 3.157 (1.05), 3.164 (1.05), 3.181 (0.52), 3.280 (1.51), 3.294 (1.17), 3.425 (0.78), 3.669 (16.00), 3.710 (10.87), 3.806 (0.40), 3.813 (0.46), 4.017 (0.56), 4.035 (0.82), 4.053 (0.84), 4.071 (1.01), 4.135 (0.58), 4.143 (0.54), 4.157 (0.66), 4.166 (0.64), 4.225 (0.54), 4.420 (0.66), 4.440 (0.74), 4.442 (0.72), 4.462 (0.54), 4.654 (1.19), 4.669 (1.23), 5.232 (1.45), 6.526 (1.19), 6.530 (1.33), 6.546 (1.43), 6.550 (1.53), 6.608 (1.19), 6.612 (1.17), 6.628 (1.61), 6.632 (1.39), 6.768 (1.41), 6.788 (2.29), 6.808 (0.96), 7.084 (1.01), 7.105 (2.29), 7.125 (1.27), 7.284 (1.47), 7.288 (1.69), 7.304 (1.83), 7.308 (1.75), 7.733 (0.96), 7.794 (1.05), 7.797 (1.09), 7.814 (1.01), 7.817 (0.98), 8.234 (0.94), 8.246 (0.96), 8.426 (2.65), 13.691 (0.50), 14.787 (1.81).

LC-MS (method 1): Rₜ = 1.23 min; MS (ESlpos): m/z = 606 [M+H]⁺

### Intermediate 5-11

### tert-butyl {2-[(4-{[(5-{[2-(difluoromethoxy)phenyl]carbamothioyl}-6-oxo-1,2,3,6-tetrahydropyridin-4-yl)amino]methyl}pyridin-3-yl)oxy]ethyl}methylcarbamate

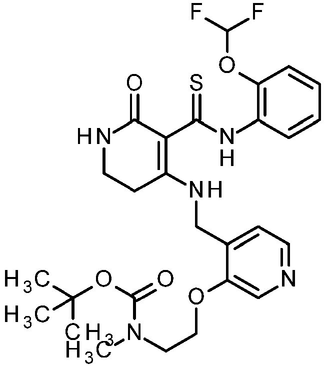

A mixture of N-[2-(difluoromethoxy)phenyl]-4-hydroxy-2-oxo-1,2,5,6-tetrahydropyridine-3-carbothioamide ( 750 mg, 2.39 mmol, intermediate 4-5) and tert-butyl (2-{[4-(aminomethyl)pyridin-3-yl]oxy}ethyl)methylcarbamate (8.06 g, 2.86 mmol, intermediate 2-3) in DMA (9 ml) in a microwave vial under an argon atmosphere was heated at 130°C for 90min. The reaction was concentrated and purified by silica chromatography (Biotage 100g, hexane: ethyl acetate 15-30%) to give the title compound 734 mg (53% yield).

¹H-NMR (400 MHz, DMSO-d6) δ [ppm]: 1.233 (0.55), 1.340 (15.61), 1.385 (16.00), 1.741 (1.67), 1.749 (1.64), 1.753 (1.12), 1.758 (5.24), 1.767 (1.67), 1.774 (1.86), 1.907 (0.58), 1.955 (2.02), 2.085 (1.51), 2.298 (0.61), 2.337 (0.61), 2.518 (8.22), 2.523 (5.59), 2.674 (1.45), 2.679 (0.77), 2.717 (3.21), 2.734 (5.91), 2.751 (3.34), 2.782 (1.96), 2.877 (7.87), 2.894 (6.68), 2.941 (2.83), 3.129 (2.22), 3.136 (2.57), 3.146 (4.14), 3.152 (3.95), 3.162 (2.31), 3.169 (1.90), 3.577 (3.73), 3.582 (4.05), 3.591 (8.32), 3.599 (7.00), 3.603 (5.43), 3.613 (2.12), 3.616 (2.57), 4.268 (3.53), 4.631 (6.52), 4.646 (6.49), 4.772 (0.87), 4.786 (0.84), 5.759 (3.76), 6.933 (4.59), 7.118 (9.25), 7.127 (0.61), 7.207 (1.41), 7.214 (1.54), 7.224 (3.18), 7.230 (5.08), 7.234 (3.24), 7.247 (11.73), 7.252 (6.97), 7.256 (5.88), 7.268 (3.95), 7.272 (1.90), 7.282 (2.18), 7.289 (2.70), 7.293 (2.96), 7.302 (5.94), 7.693 (4.05), 7.719 (3.89), 7.723 (2.92), 7.739 (3.82), 7.743 (2.18), 8.174 (0.45), 8.231 (4.47), 8.243 (4.31), 8.393 (2.02), 8.414 (2.06), 13.706 (1.67), 14.676 (6.39).

### Syntheses of intermediate 6 compounds

### Intermediate 6-1

### tert-butyl [2-({4-[3-(3-chloro-2-methoxyanilino)-4-oxo-4,5,6,7-tetrahydro-1H-pyrrolo[3,2-c]pyridin-2-yl]pyridin-3-yl}oxy)ethyl]carbamate

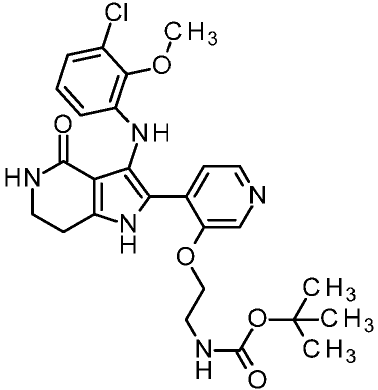

To a suspension of tert-butyl [2-({4-[({5-[(3-chloro-2-methoxyphenyl)carbamothioyl]-6-oxo-1,2,3,6-tetrahydropyridin-4-yl}amino)methyl]pyridin-3-yl}oxy)ethyl]carbamate (830 mg, 80 % purity, 1.18 mmol, intermediate 5-1) in MeOH (10 mL) was added TFA (270 µl, 3.5 mmol) followed by hydrogen peroxide (240 µl, 30 % in water, 2.4 mmol). The mixture was heated to 60°C and stirred for 3.5 h. Additional hydrogen peroxide (50 µl, 30 % in water,0,5 mmol) was added. The suspension was stirred at 60°C for further 0.5h. Aqueous sodiumsulfate was added and stirred at RT for 15min. The mixture was basified with aqueous sodium hydrogencarbonte and extracted twice with DCM/methanol. followed by column chromatography (silica, DCM /ethanol gradient 0-10%) to give 340 mg (95 % purity, 52 % yield) of the title compound.

¹H-NMR (400 MHz, DMSO-d6) δ [ppm]: 1.154 (2.24), 1.172 (4.69), 1.189 (2.33), 1.368 (16.00), 1.752 (0.63), 1.987 (8.06), 2.518 (1.19), 2.522 (0.85), 2.953 (0.70), 3.405 (0.42), 3.416 (0.70), 3.421 (0.71), 3.438 (0.43), 3.459 (0.54), 3.471 (0.53), 3.892 (4.27), 3.999 (0.60), 4.016 (1.80), 4.034 (1.74), 4.053 (0.56), 4.176 (0.54), 4.189 (0.96), 4.201 (0.55), 6.100 (0.47), 6.112 (0.69), 6.124 (0.48), 6.682 (1.12), 6.693 (1.38), 7.165 (0.55), 7.343 (0.62), 7.357 (0.62), 7.541 (0.96), 7.981 (0.75), 7.994 (0.73), 8.379 (1.46), 11.118 (0.56).

LC-MS (method 1): Rₜ = 0.84 min; m/z = 528.3 (M +H)⁺

### Intermediate 6-2

### tert-butyl [2-({4-[3-(3-chloro-2-methylanilino)-4-oxo-4,5,6,7-tetrahydro-1H-pyrrolo[3,2-c]pyridin-2-yl]pyridin-3-yl}oxy)ethyl]carbamate

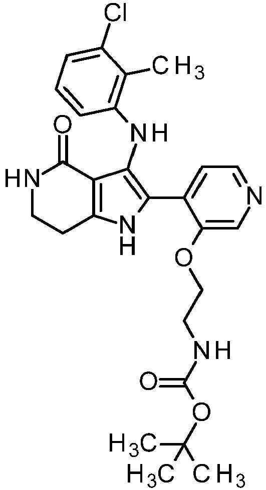

Using an analogous method as described for intermediate 6-1 with tert-butyl [2-({4-[({5-[(3-chloro-2-methylphenyl)carbamothioyl]-6-oxo-1,2,3,6-tetrahydropyridin-4-yl}amino)methyl]pyridin-3-yl}oxy)ethyl]carbamate (534 mg, 978 µmol, intermediate 5-2) as the starting material, stirring at 60°C for 5.5h and followed by stirring at RT for 16h. 151 mg (97 % purity, 29 % yield) of the title compound were prepared after purification by column chromatography (silica, DCM / ethanol gradient 0-10%).

¹H-NMR (400 MHz, DMSO-d6) δ [ppm]: 1.035 (0.59), 1.053 (1.43), 1.071 (0.67), 1.369 (16.00), 2.363 (3.45), 2.518 (0.86), 2.523 (0.59), 2.963 (0.65), 3.409 (0.40), 3.420 (0.72), 3.425 (0.72), 3.440 (0.57), 3.457 (0.54), 3.469 (0.52), 4.161 (0.45), 4.173 (0.77), 4.185 (0.44), 6.155 (0.45), 6.159 (0.45), 6.175 (0.51), 6.179 (0.47), 6.745 (0.72), 6.748 (0.65), 6.757 (0.52), 6.777 (0.50), 7.192 (0.51), 7.302 (0.72), 7.315 (0.91), 7.361 (1.00), 7.945 (0.72), 7.958 (0.69), 8.356 (1.75), 11.094 (0.49).

LC-MS (method 1): Rₜ = 0.86 min; MS (ESlpos): m/z = 512 [M+H]⁺

### Intermediate 6-3

### tert-butyl [2-({4-[3-(3-fluoro-2-methylanilino)-4-oxo-4,5,6,7-tetrahydro-1H-pyrrolo[3,2-c]pyridin-2-yl]pyridin-3-yl}oxy)ethyl]carbamate

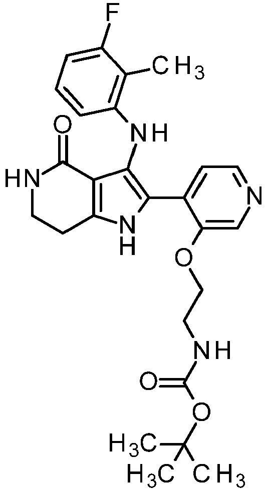

Using an analogous method as described for intermediate 6-1 with tert-butyl [2-({4-[({5-[(3-fluoro-2-methylphenyl)carbamothioyl]-6-oxo-1,2,3,6-tetrahydropyridin-4-yl}amino)methyl]pyridin-3-yl}oxy)ethyl]carbamate (362 mg, 684 µmol, intermediate 5-3) as the starting material wich was stirred at 60°C for 16h. Aqueous sodiumsulfate was added. The mixture was neutralised with aqueous sodiumhydrogencarbonte and extracted twice with DCM. followed by column chromatography (silica, DCM /ethanol gradient 0-10%) to give 96.2 mg (98 % purity, 28 % yield) of the title compound.

¹H-NMR (400 MHz, DMSO-d6) δ [ppm]: 1.035 (0.76), 1.052 (1.67), 1.070 (0.72), 1.369 (16.00), 2.195 (2.36), 2.518 (0.96), 2.522 (0.61), 2.960 (0.69), 3.404 (0.47), 3.409 (0.42), 3.422 (1.01), 3.426 (0.74), 3.435 (0.80), 3.440 (0.83), 3.452 (0.81), 3.457 (0.65), 3.469 (0.63), 4.158 (0.48), 4.169 (0.80), 4.181 (0.45), 4.357 (0.51), 5.759 (2.65), 6.019 (0.61), 6.039 (0.64), 6.467 (0.45), 7.193 (0.54), 7.302 (0.76), 7.315 (0.95), 7.342 (1.23), 7.936 (0.73), 7.949 (0.69), 8.351 (1.76), 11.075 (0.53).

LC-MS (method 1): Rₜ = 0.81 min; MS (ESlpos): m/z = 496 [M+H]⁺

### Intermediate 6-4

### tert-butyl [3-({4-[3-(3-chloro-2-methoxyanilino)-4-oxo-4,5,6,7-tetrahydro-1H-pyrrolo[3,2-c]pyridin-2-yl]pyridin-3-yl}oxy)propyl]carbamate

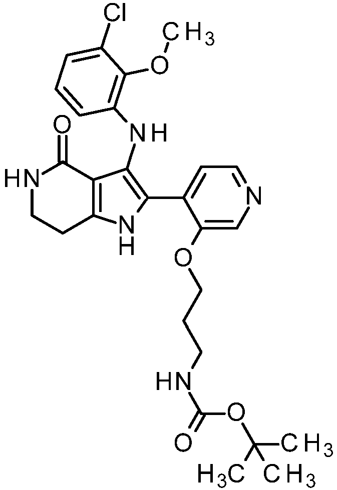

Using an analogous method as described for intermediate 6-1 with tert-butyl [3-({4-[({5-[(3-chloro-2-methoxyphenyl)carbamothioyl]-6-oxo-1,2,3,6-tetrahydropyridin-4-yl}amino)methyl]pyridin-3-yl}oxy)propyl]carbamate (700 mg, 1.22 mmol, intermediate 5-4) as the starting material, which was stirred at 60°C for 3h. 73.5 mg (90% purity, 47% yield) of the title compound were prepared.

¹H-NMR (400MHz, DMSO-d₆): δ [ppm]= 1H-NMR (400 MHz, DMSO-d6) delta [ppm]: 1.365 (16.00), 1.875 (0.76), 1.891 (1.14), 1.907 (0.80), 2.850 (0.70), 2.867 (1.38), 2.884 (0.75), 3.085 (0.43), 3.101 (0.98), 3.116 (0.95), 3.397 (0.70), 3.407 (1.21), 3.413 (1.20), 3.424 (0.64), 3.850 (7.44), 4.163 (0.78), 4.179 (1.52), 4.194 (0.77), 6.138 (0.79), 6.144 (0.84), 6.156 (0.79), 6.162 (0.84), 6.627 (0.44), 6.642 (1.75), 6.646 (2.11), 6.664 (0.88), 6.992 (0.65), 7.090 (1.12), 7.257 (1.46), 7.269 (1.50), 7.425 (1.75), 8.010 (1.14), 8.023 (1.11), 8.370 (2.48), 11.162 (1.09).

LC-MS (method 1): Rₜ = 0.87 min; MS (ESlpos): m/z = 542 [M+H]⁺

### Intermediate 6-5

### tert-butyl [2-({4-[3-(3-chloro-2-methoxyanilino)-4-oxo-4,5,6,7-tetrahydro-1H-pyrrolo[3,2-c]pyridin-2-yl]pyridin-3-yl}oxy)ethyl]methylcarbamate

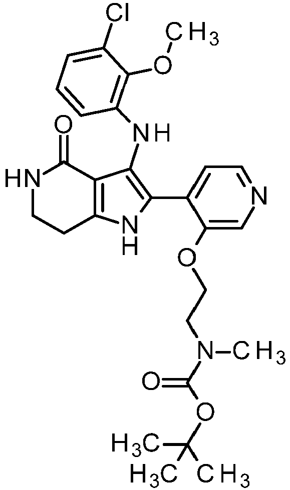

Using an analogous method as described for intermediate 6-1 with tert-butyl [2-({4-[({5-[(3-chloro-2-methoxyphenyl)carbamothioyl]-6-oxo-1,2,3,6-tetrahydropyridin-4-yl}amino)methyl]pyridin-3-yl}oxy)ethyl]methylcarbamate (630 mg, 90 % purity, 984 µmol, intermediate 5-5) as the starting material, 322 mg (60 % yield) of the title compound were prepared after purification by preparative HPLC (method 7, gradient: 0.00-0.50 min 30% B, 0.50-6.00 min 30-70% B, 250 nm).

¹H-NMR (400 MHz, DMSO-d₆): δ [ppm]= 1H-NMR (400 MHz, DMSO-d6) delta [ppm]: 1.154 (3.43), 1.171 (6.97), 1.189 (3.54), 1.230 (1.09), 1.290 (4.48), 1.368 (16.00), 1.986 (10.72), 2.518 (1.67), 2.522 (1.06), 2.777 (2.06), 2.890 (3.09), 2.949 (1.20), 3.294 (0.62), 3.299 (0.63), 3.394 (1.73), 3.405 (2.83), 3.410 (2.81), 3.422 (1.52), 3.499 (0.50), 3.512 (1.06), 3.526 (0.58), 3.628 (0.95), 3.689 (1.20), 3.713 (1.22), 3.885 (5.14), 3.999 (0.85), 4.017 (2.56), 4.034 (2.57), 4.052 (0.85), 4.311 (1.60), 4.722 (0.72), 4.735 (0.69), 6.092 (1.20), 6.100 (1.28), 6.107 (1.39), 6.115 (1.27), 6.679 (1.89), 7.162 (0.85), 7.250 (0.68), 7.271 (0.81), 7.289 (0.69), 7.362 (0.88), 7.416 (0.78), 7.428 (0.82), 7.522 (0.87), 7.625 (0.99), 7.646 (0.90), 8.002 (0.94), 8.272 (1.13), 8.283 (1.03), 8.390 (6.52), 11.176 (3.37).

LC-MS (method 1): Rₜ = 0.94 min; MS (ESlpos): m/z = 542 [M+H]⁺.

### Intermediate 6-6

### tert-butyl [2-({4-[3-(3-fluoro-2-methoxyanilino)-4-oxo-4,5,6,7-tetrahydro-1H-pyrrolo[3,2-c]pyridin-2-yl]pyridin-3-yl}oxy)ethyl]methylcarbamate

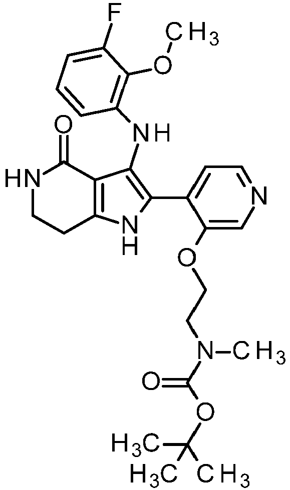

Using an analogous method as described for intermediate 6-1 with tert-butyl [2-({4-[({5-[(3-fluoro-2-methoxyphenyl)carbamothioyl]-6-oxo-1,2,3,6-tetrahydropyridin-4-yl}amino)methyl]pyridin-3-yl}oxy)ethyl]methylcarbamate (500 mg, 893 µmol, intermediate 5-6) as the starting material, which was stirred at 60°C for 3.5h 280 mg (90 % purity, 54 % yield) of the title compound were prepared after purification by column chromatography (silica, DCM /ethanol gradient 0-10%)

H-NMR (400 MHz, DMSO-d6) δ [ppm]: 1.298 (8.15), 1.368 (16.00), 2.074 (0.50), 2.327 (0.91), 2.539 (3.86), 2.669 (0.96), 2.785 (2.05), 2.893 (3.32), 2.945 (1.34), 3.290 (0.50), 3.390 (1.90), 3.403 (2.98), 3.518 (0.69), 3.532 (0.41), 3.605 (0.44), 3.717 (1.45), 3.911 (6.46), 4.310 (1.68), 4.717 (0.50), 4.731 (0.48), 5.950 (1.90), 5.970 (1.99), 6.497 (0.77), 6.623 (0.75), 7.161 (1.10), 7.264 (0.42), 7.369 (0.91), 7.440 (0.53), 7.452 (0.54), 7.519 (1.01), 7.995 (1.01), 8.278 (0.58), 8.290 (0.54), 8.385 (6.22), 11.152 (3.55).

LC-MS (method 1): Rₜ = 0.89 min; MS (ESlpos): m/z = 526 [M+H]⁺

### Intermediate 6-7

### tert-butyl [2-({4-[3-(4-fluoroanilino)-4-oxo-4,5,6,7-tetrahydro-1H-pyrrolo[3,2-c]pyridin-2-yl]pyridin-3-yl}oxy)ethyl]methylcarbamate

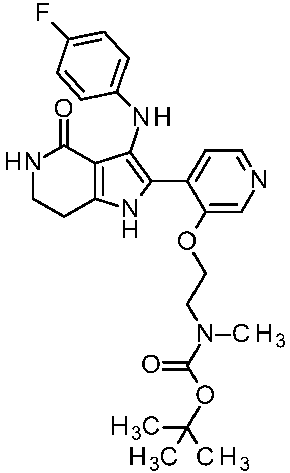

Using an analogous method as described for intermediate 6-1 with tert-butyl [2-({4-[({5-[(4-fluorophenyl)carbamothioyl]-6-oxo-1,2,3,6-tetrahydropyridin-4-yl}amino)methyl]pyridin-3-yl}oxy)ethyl]methylcarbamate (517 mg, 85 % purity, 830 µmol, intermediate 5-7) as the starting material, which was stirred at 60°C for 16h, 206 mg (97 % purity, 49 % yield) of the title compound were prepared after purification by flash chromatography (silica, DCM / ethanol gradient 0-10%).

¹H-NMR (400 MHz, DMSO-d6) δ [ppm]: 0.798 (0.42), 0.815 (0.44), 0.822 (0.45), 0.905 (0.48), 1.035 (7.91), 1.053 (16.00), 1.070 (7.62), 1.373 (9.61), 2.327 (0.49), 2.518 (1.78), 2.523 (1.17), 2.669 (0.51), 2.893 (1.66), 2.945 (0.69), 3.384 (0.96), 3.396 (1.58), 3.400 (1.58), 3.405 (1.85), 3.418 (1.69), 3.422 (3.03), 3.435 (2.85), 3.440 (2.77), 3.452 (2.76), 3.457 (0.92), 3.469 (0.85), 3.717 (0.69), 4.291 (0.80), 4.343 (1.60), 4.356 (3.06), 4.369 (1.48), 6.526 (1.04), 6.538 (1.24), 6.548 (1.37), 6.559 (1.18), 6.843 (0.87), 7.118 (0.41), 7.136 (0.51), 7.418 (0.90), 7.958 (0.57), 8.350 (5.19), 11.083 (0.83).

LC-MS (method 1): Rₜ = 0.82 min; MS (ESlpos): m/z = 496 [M-H]⁺

### Intermediate 6-8

### tert-butyl [2-({4-[3'-(3-chloro-2-methoxyanilino)-4'-oxo-1',4',5',7'-tetrahydrospiro[cyclobutane-1,6'-pyrrolo[3,2-c]pyridin]-2'-yl]pyridin-3-yl}oxy)ethyl]methylcarbamate

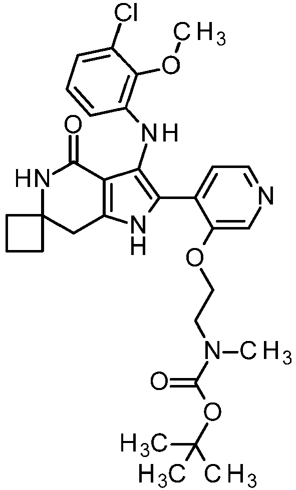

Using an analogous method as described for intermediate 6-2 with tert-butyl [2-({4-[({7-[(3-chloro-2-methoxyphenyl)carbamothioyl]-6-oxo-5-azaspiro[3.5]non-7-en-8-yl}amino)methyl]pyridin-3-yl}oxy)ethyl]methylcarbamate (731 mg, 1.19 mmol, intermediate 5-8) as the starting material, which was stirred at 60°C for 16h, 319 mg (95 % purity, 44 % yield) of the title compound were prepared after purification by preparative HPLC (method 7, gradient: 0.00-0.50 min 50% B, 0.50-4.65 min 50-70% B, 254nm) under basic conditions.

¹H-NMR (400 MHz, DMSO-d6) δ [ppm]: 1.404 (16.00), 1.678 (0.82), 1.703 (1.42), 1.732 (1.94), 1.755 (1.34), 1.998 (1.56), 2.020 (3.19), 2.041 (1.97), 2.074 (1.94), 2.118 (1.20), 2.140 (2.68), 2.166 (2.18), 2.322 (0.87), 2.326 (1.20), 2.332 (0.96), 2.518 (6.14), 2.522 (3.60), 2.665 (0.90), 2.669 (1.28), 2.673 (0.93), 2.789 (0.66), 2.909 (4.53), 3.010 (0.46), 3.156 (2.62), 3.604 (0.44), 3.734 (2.02), 3.887 (6.85), 4.338 (2.29), 6.072 (2.51), 6.081 (2.43), 6.088 (2.70), 6.096 (2.62), 6.685 (2.81), 7.381 (1.50), 7.467 (1.75), 7.558 (1.69), 7.994 (1.47), 8.394 (8.76), 11.258 (2.02).

LC-MS (method 1): Rt = 1.06 min; MS (ESlpos): m/z = 582 [M+H]⁺

### Intermediate 6-9

### tert-butyl [2-({4-[3-(3-chloro-2-methoxyanilino)-6,6-dimethyl-4-oxo-4,5,6,7-tetrahydro-1H-pyrrolo[3,2-c]pyridin-2-yl]pyridin-3-yl}oxy)ethyl]methylcarbamate

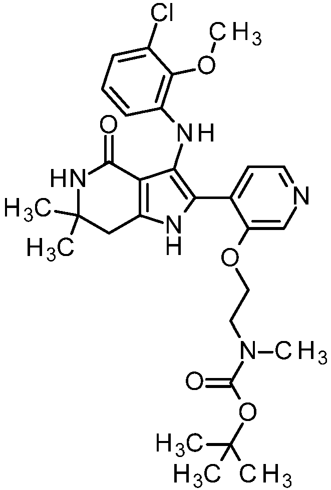

Using an analogous method as described for intermediate 6-2 with tert-butyl [2-({4-[({5-[(3-chloro-2-methoxyphenyl)carbamothioyl]-2,2-dimethyl-6-oxo-1,2,3,6-tetrahydropyridin-4-yl}amino)methyl]pyridin-3-yl}oxy)ethyl]methylcarbamate (673 mg, 90 % purity, 1.00 mmol, intermediate 5-9) as the starting material, which was stirred at 60°C for 16h, 411 mg (97 % purity, 70% yield) of the title compound were prepared after purification by column chromatography (silica, DCM / ethanol gradient 0-10%).

H-NMR (400 MHz, DMSO-d6) δ [ppm]: 0.884 (1.93), 0.902 (4.08), 0.920 (1.87), 1.035 (2.36), 1.053 (5.00), 1.070 (2.21), 1.259 (16.00), 1.375 (7.89), 2.066 (5.14), 2.327 (0.47), 2.420 (1.10), 2.438 (1.13), 2.518 (1.54), 2.523 (0.93), 2.669 (0.49), 2.807 (0.41), 2.890 (1.67), 2.924 (0.97), 3.316 (0.52), 3.422 (1.03), 3.435 (1.04), 3.440 (1.06), 3.452 (1.10), 3.712 (0.69), 3.887 (2.28), 4.319 (0.81), 4.343 (0.98), 4.355 (1.46), 4.368 (0.71), 5.758 (1.76), 6.093 (0.93), 6.107 (1.17), 6.117 (0.94), 6.680 (0.99), 7.086 (0.49), 7.381 (0.53), 7.480 (0.49), 7.999 (0.51), 8.386 (3.31), 11.192 (1.09).

LC-MS (method 1): Rt = 1.0 min; MS (ESlpos): m/z = 570 [M+H]+

### Intermediate 6-10

### tert-butyl (2S)-2-[({4-[3-(3-chloro-2-methoxyanilino)-4-oxo-4,5,6,7-tetrahydro-1H-pyrrolo[3,2-c]pyridin-2-yl]pyridin-3-yl}oxy)methyl]pyrrolidine-1-carboxylate

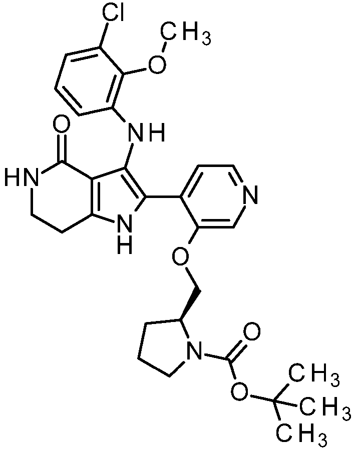

Tert-butyl (2S)-2-[({4-[({5-[(3-chloro-2-methoxyphenyl)carbamothioyl]-6-oxo-1,2,3,6-tetrahydropyridin-4-yl}amino)methyl]pyridin-3-yl}oxy)methyl]pyrrolidine-1-carboxylate (507 mg, 76 % purity, 640 µmol, intermediate 5-10) was taken up in 6ml methanol, TFA (99 µl, 1.3 mmol) was added, the mixture was stirred for 5min at RT. Meta-chloroperbenzoic acid (221 mg, 1.28 mmol) was added and stirred at 50°C for 1h. The reaction was quenched with aqueous sodiumthiosulfate solution and stirred at RT for 0.5h. Then TEA (187.2µl, 1.343mmol) was added and extracted twice with DCM. The crude product was purified by column chromatography (silica - amino-phase, ethyl acetate/ ethanol gradient 0-1%). The product rich fractions were pooled and evaporated to give the desired product as a yellow crystalline solid (241.3mg, 77% purity).

¹H-NMR (400 MHz, DMSO-d6) δ [ppm]: 1.154 (4.77), 1.172 (9.54), 1.190 (4.64), 1.232 (1.54), 1.406 (14.27), 1.824 (1.22), 1.988 (16.00), 2.332 (1.34), 2.336 (0.61), 2.518 (8.22), 2.523 (5.18), 2.673 (1.41), 2.678 (0.64), 2.841 (0.42), 2.932 (1.41), 3.413 (2.46), 3.762 (0.61), 3.883 (4.86), 4.000 (1.31), 4.017 (3.81), 4.035 (4.06), 4.053 (2.34), 4.068 (1.95), 4.086 (0.64), 4.331 (1.41), 6.107 (1.28), 6.119 (2.11), 6.131 (1.38), 6.673 (2.05), 7.151 (1.06), 7.349 (1.31), 7.486 (1.31), 7.998 (0.99), 8.417 (2.18), 11.433 (0.90).

LC-MS (method 1): Rₜ = 0.98 min; MS (ESlpos): m/z = 568 [M+H]⁺

### Intermediate 6-11

### tert-butyl (2-{[4-(3-{[2-(difluoromethoxy)phenyl]amino}-4-oxo-4,5,6,7-tetrahydro-1H-pyrrolo[3,2-c]pyridin-2-yl)pyridin-3-yl]oxy}ethyl)methylcarbamate

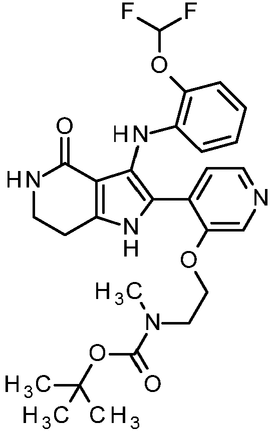

Using an analogous method as described for Intermediate 6-1with tert-butyl {2-[(4-{[(5-{[2-(difluoromethoxy)phenyl]carbamothioyl}-6-oxo-1,2,3,6-tetrahydropyridin-4-yl)amino]methyl}pyridin-3-yl)oxy]ethyl}methylcarbamate (730 mg, 1.26 mmol, intermediate 5-11) as the starting material the title compound was prepared 273 mg (36% yield) after purification with column chromatography (silica, DCM/MeOH 10-35%).

¹H-NMR (400MHz, DMSO-*d*₆): δ [ppm]= 11.16 (s, 1H), 8.38 (s, 1H), 7.96 (br s, 1H), 6.98 - 7.50 (m, 6H), 6.79 (br s, 1H), 6.66 (br s, 1H), 6.26 (br d, 1H), 4.30 (br s, 2H), 3.71 (br s, 2H), 3.36 - 3.47 (m, 2H), 2.75 - 3.02 (m, 5H), 1.37 (br s, 9H).

### Syntheses of intermediate 7 compounds

### Intermediate 7-1

### 2-[3-(2-aminoethoxy)pyridin-4-yl]-3-(3-chloro-2-methoxyanilino)-1,5,6,7-tetrahydro-4H-pyrrolo[3,2-c]pyridin-4-one

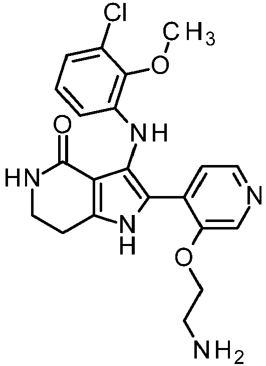

To a solution of tert-butyl [2-({4-[3-(3-chloro-2-methoxyanilino)-4-oxo-4,5,6,7-tetrahydro-1H-pyrrolo[3,2-c]pyridin-2-yl]pyridin-3-yl}oxy)ethyl]carbamate (340 mg, 644 µmol, intermediate 6-1) in dichloromethane (6.2 ml) TFA (500 µl, 6.4 mmol) was added and the mixture was stirred for 16h. The solvents were evaporated and the residue was purified by preparative HPLC (method 7, gradient: 0.00-0.50 min 20% B, 0.50-6.00 min 20-40% B, 277nm) under basic pH conditions to give 210 mg (97 % purity, 74 % yield) of the title compound.

¹H-NMR (400 MHz, DMSO-d6) δ [ppm]: 20.518 (1.26), 2.522 (0.89), 2.539 (16.00), 2.852 (0.84), 2.869 (0.46), 3.024 (0.43), 3.036 (0.70), 3.049 (0.45), 3.402 (0.60), 3.409 (0.58), 3.888 (5.40), 4.255 (0.46), 4.267 (0.75), 4.279 (0.45), 6.160 (0.49), 6.171 (0.46), 6.173 (0.54), 6.184 (0.49), 6.675 (2.00), 6.685 (1.12), 6.689 (0.98), 7.124 (0.54), 7.256 (0.87), 7.269 (0.87), 7.556 (1.18), 7.981 (1.14), 7.993 (1.03), 8.418 (1.59).

LC-MS (method 1): Rₜ = 0.48 min; MS (ESlpos): m/z = 428 [M+H]⁺

### Intermediate 7-2

### 2-[3-(2-aminoethoxy)pyridin-4-yl]-3-(3-chloro-2-methylanilino)-1,5,6,7-tetrahydro-4H-pyrrolo[3,2-c]pyridin-4-one

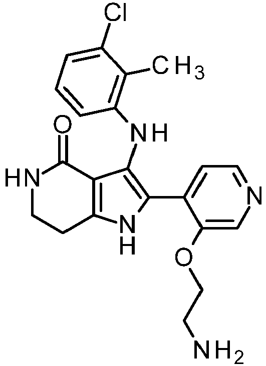

Using an analogous method as described for intermediate 5-1 with tert-butyl [2-({4-[3-(3-chloro-2-methylanilino)-4-oxo-4,5,6,7-tetrahydro-1H-pyrrolo[3,2-c]pyridin-2-yl]pyridin-3-yl}oxy)ethyl]carbamate (233 mg, 454 µmol, intermediate 6-2) as the starting material 81.5 mg (97 % purity, 42 % yield) of the title compound were prepared after purification by preparative HPLC (method 7, gradient: 0.00-0.50 min 15% B, 0.50-6.00 min 15-35% B, 254nm ) under basic pH conditions

¹H-NMR (400 MHz, DMSO-d6) δ [ppm]: 2.359 (16.00), 2.523 (1.81), 2.539 (4.11), 2.640 (0.44), 2.838 (1.75), 2.855 (3.72), 2.872 (2.05), 2.987 (1.96), 2.999 (3.20), 3.012 (2.10), 3.388 (1.49), 3.393 (1.59), 3.405 (2.64), 3.410 (2.56), 3.421 (1.38), 3.428 (1.24), 4.236 (2.08), 4.248 (3.30), 4.260 (2.03), 6.233 (1.90), 6.236 (1.92), 6.252 (2.09), 6.256 (1.99), 6.716 (1.23), 6.720 (1.46), 6.736 (3.23), 6.739 (2.76), 6.759 (2.26), 6.779 (2.71), 6.799 (0.96), 7.141 (2.34), 7.201 (3.81), 7.214 (3.83), 7.351 (4.51), 7.930 (5.02), 7.943 (4.74), 8.397 (6.80).

LC-MS (method 1): Rₜ = 0.49 min; MS (ESlpos): m/z = 412 [M+H]⁺

### Intermediate 7-3

### 2-[3-(2-aminoethoxy)pyridin-4-yl]-3-(3-fluoro-2-methylanilino)-1,5,6,7-tetrahydro-4H-pyrrolo[3,2-c]pyridin-4-one

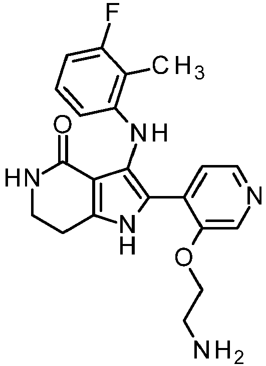

To a solution of tert-butyl [2-({4-[3-(3-fluoro-2-methylanilino)-4-oxo-4,5,6,7-tetrahydro-1H-pyrrolo[3,2-c]pyridin-2-yl]pyridin-3-yl}oxy)ethyl]carbamate (161 mg, 324 µmol, intermediate 6-3) in dichloromethane (2.5 ml) TFA (250 µl, 3.2 mmol) was added and the mixture was stirred for 16h. The solvents were evaporated, the residue was neutralized with a few drops of aqueous sodium hydroxide solution and TEA and purified by preparative HPLC (method 7, gradient: 0.00-0.50 min 10% B, 0.50-6.00 min 10-30% B, 332nm) under basic pH conditions to give 35.3 mg (97 % purity, 27 % yield) of the title compound.

¹H-NMR (400 MHz, DMSO-d6) δ [ppm]: 2.190 (15.92), 2.193 (16.00), 2.332 (1.59), 2.336 (0.69), 2.518 (7.72), 2.523 (4.91), 2.673 (1.58), 2.678 (0.68), 2.835 (3.08), 2.852 (6.63), 2.869 (3.49), 2.992 (3.22), 3.005 (5.32), 3.017 (3.41), 3.388 (2.33), 3.394 (2.44), 3.405 (4.34), 3.411 (4.23), 3.422 (2.12), 3.428 (1.91), 4.235 (3.54), 4.247 (5.67), 4.259 (3.50), 6.097 (3.92), 6.117 (4.06), 6.435 (1.78), 6.458 (3.33), 6.480 (2.08), 6.739 (1.26), 6.760 (2.60), 6.777 (2.49), 6.798 (1.07), 7.140 (4.01), 7.203 (7.18), 7.216 (7.20), 7.331 (8.05), 7.925 (10.53), 7.938 (9.63), 8.393 (13.28).

LC-MS (method 1): Rₜ = 0.44 min; MS (ESlpos): m/z = 396 [M+H]⁺

### Intermediate 7-4

### 2-[3-(3-aminopropoxy)pyridin-4-yl]-3-(3-chloro-2-methoxyanilino)-1,5,6,7-tetrahydro-4H-pyrrolo[3,2-c]pyridin-4-one

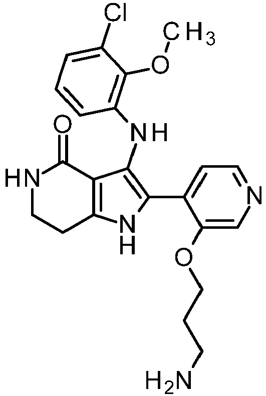

To a solution of tert-butyl [3-({4-[3-(3-chloro-2-methoxyanilino)-4-oxo-4,5,6,7-tetrahydro-1H-pyrrolo[3,2-c]pyridin-2-yl]pyridin-3-yl}oxy)propyl]carbamate (300 mg, 553 µmol, intermediate 6-4) in dichloromethane (5.9 ml), TFA (430 µl, 5.5 mmol) was added and the mixture was stirred for 16h. Further 100 µl TFA were added and stirred for 4h at RT. A small amount of aqueous sodium hydrogen carbonate solution was added until the reaction mixture became slightly basic, it was then extracted twice with DCM/methanol. The solvents were evaporated and purified by preparative HPLC (method 7, gradient: 0.00-0.50 min 25% B, 0.50-6.00 min 25-45% B, 250nm) under basic pH conditions to give 220 mg (66% purity, 59% yield) of the title compound.

1H-NMR (400 MHz, DMSO-d6) delta [ppm]: 2.518 (0.16), 2.539 (16.00), 2.827 (0.27), 2.836 (0.23), 2.844 (0.21), 2.850 (0.16), 3.338 (0.30), 3.373 (0.24), 3.379 (0.23), 3.390 (0.28), 3.396 (0.26), 3.406 (0.20), 3.852 (0.57), 4.282 (0.21), 6.138 (0.16), 6.668 (0.57), 6.677 (0.25), 6.682 (0.23), 7.312 (0.23), 7.324 (0.23), 7.503 (0.30), 7.976 (0.28), 7.988 (0.26), 8.355 (0.36), 8.381 (0.16).

LC-MS (method 1): Rₜ = 0.49 min; MS (ESlpos): m/z = 442.2 [M+H]⁺

### Intermediate 7-5

### 3-(3-chloro-2-methoxyanilino)-2-{3-[2-(methylamino)ethoxy]pyridin-4-yl}-1,5,6,7-tetrahydro-4H-pyrrolo[3,2-c]pyridin-4-one

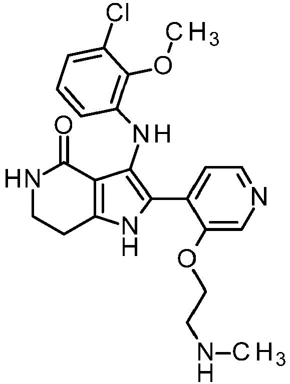

To a solution of tert-butyl [2-({4-[3-(3-chloro-2-methoxyanilino)-4-oxo-4,5,6,7-tetrahydro-1H-pyrrolo[3,2-c]pyridin-2-yl]pyridin-3-yl}oxy)ethyl]methylcarbamate (300 mg, 553 µmol, intermediate 6-5) in dichloromethane (5.9 ml), TFA (430 µl, 5.5 mmol) was added and the mixture was stirred 4h at RT. The solvents were evaporated and the residue was stored in the deep freezer over the weekend. The solvents were removed in vacuo, aqueos sodiumhydrogencarbonate was added until ph 8, the mixture was extracted twice with DCM/methanol and then purified by preparative HPLC (method 7, gradient: 0.00-0.50 min 30% B, 0.50-6.00 min 30-50% B, 252nm) under basic pH conditions to give 220 mg (94 % purity, 85 % yield) of the title compound.

¹H-NMR (400 MHz, DMSO-d6) δ [ppm]: 2.359 (16.00), 2.523 (1.81), 2.539 (4.11), 2.640 (0.44), 2.838 (1.75), 2.855 (3.72), 2.872 (2.05), 2.987 (1.96), 2.999 (3.20), 3.012 (2.10), 3.388 (1.49), 3.393 (1.59), 3.405 (2.64), 3.410 (2.56), 3.421 (1.38), 3.428 (1.24), 4.236 (2.08), 4.248 (3.30), 4.260 (2.03), 6.233 (1.90), 6.236 (1.92), 6.252 (2.09), 6.256 (1.99), 6.716 (1.23), 6.720 (1.46), 6.736 (3.23), 6.739 (2.76), 6.759 (2.26), 6.779 (2.71), 6.799 (0.96), 7.141 (2.34), 7.201 (3.81), 7.214 (3.83), 7.351 (4.51), 7.930 (5.02), 7.943 (4.74), 8.397 (6.80).

LC-MS (method 1): Rₜ = 0.51 min; MS (ESlpos): m/z = 442 [M+H]⁺

### Intermediate 7-6

### 3-(3-fluoro-2-methoxyanilino)-2-{3-[2-(methylamino)ethoxy]pyridin-4-yl}-1,5,6,7-tetrahydro-4H-pyrrolo[3,2-c]pyridin-4-one

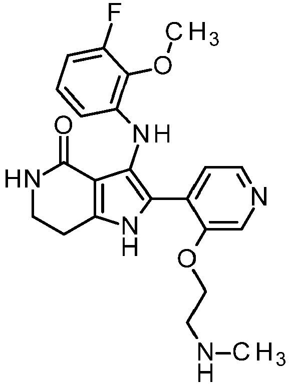

To a solution of tert-butyl [2-({4-[3-(3-fluoro-2-methoxyanilino)-4-oxo-4,5,6,7-tetrahydro-1H-pyrrolo[3,2-c]pyridin-2-yl]pyridin-3-yl}oxy)ethyl]methylcarbamate (275 mg, 523 µmol, intermediate 6-6) in dichloromethane (5.0 ml), TFA (400 µl, 5.2 mmol) was added and the mixture was stirred for 16h. Another 100 µl TFA were added an stirred for 16h again. The solvents were removed, aqueous sodiumhydrogencarbonate was added until ph 8, the mixture was extracted twice with DCM/methanol and then purified by preparative HPLC (method 7, gradient: 0.00-0.50 min 25% B, 0.50-6.00 min 25-45% B, 275nm) under basic pH conditions to give 200 mg (97 % purity, 87 % yield) of the title compound.

¹H-NMR (400 MHz, DMSO-d6) δ [ppm]: 1.225 (0.48), 2.084 (2.54), 2.422 (14.90), 2.434 (1.14), 2.518 (1.86), 2.522 (1.13), 2.539 (1.15), 2.834 (1.57), 2.852 (3.40), 2.869 (1.90), 2.955 (1.41), 2.967 (2.17), 2.978 (1.49), 3.399 (1.22), 3.405 (1.33), 3.416 (2.29), 3.422 (2.27), 3.433 (1.17), 3.438 (1.06), 3.758 (1.04), 3.920 (16.00), 4.349 (1.78), 4.361 (2.64), 4.373 (1.78), 5.758 (1.78), 6.011 (1.83), 6.031 (1.92), 6.471 (0.82), 6.475 (0.90), 6.492 (1.21), 6.496 (1.26), 6.499 (1.12), 6.502 (0.96), 6.519 (1.15), 6.523 (1.08), 6.611 (0.86), 6.627 (0.98), 6.632 (1.56), 6.647 (1.52), 6.653 (0.81), 6.668 (0.65), 7.137 (2.18), 7.268 (3.36), 7.281 (3.45), 7.567 (4.68), 7.974 (4.10), 7.986 (3.83), 8.428 (5.99).

LC-MS (method 1): Rₜ = 0.45 min; MS (ESlpos): m/z = 426 [M+H]⁺

### Intermediate 7-7

### 3-(4-fluoroanilino)-2-{3-[2-(methylamino)ethoxy]pyridin-4-yl}-1,5,6,7-tetrahydro-4H-pyrrolo[3,2-c]pyridin-4-one

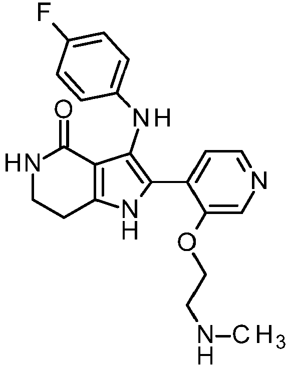

Using an analogous method as described for intermediate 7-2 with tert-butyl [2-({4-[3-(4-fluoroanilino)-4-oxo-4,5,6,7-tetrahydro-1H-pyrrolo[3,2-c]pyridin-2-yl]pyridin-3-yl}oxy)ethyl]methylcarbamate (206 mg, 416 µmol, intermediate 6-7) as the starting material, with adding another 200 µl TFA and stirring at RT for 24h, the title compound was prepared after purification by preparative HPLC (method 7, gradient: 0.00-0.50 min 15% B, 0.50-6.00 min 15-35% B, 346nm) under basic pH conditions 99.7 mg (97 % purity, 59 % yield).

¹H-NMR (400 MHz, DMSO-d6) δ [ppm]: 2.327 (0.64), 2.392 (16.00), 2.669 (0.55), 2.825 (2.43), 2.843 (5.19), 2.859 (2.82), 2.892 (2.39), 2.903 (3.55), 2.914 (2.45), 3.391 (1.77), 3.397 (1.94), 3.408 (3.49), 3.414 (3.40), 3.424 (1.77), 4.330 (2.73), 4.343 (4.01), 4.355 (2.69), 6.570 (3.19), 6.581 (3.41), 6.587 (2.32), 6.592 (4.14), 6.604 (3.88), 6.833 (3.79), 6.855 (6.65), 6.877 (3.19), 7.101 (3.25), 7.303 (4.84), 7.316 (4.97), 7.432 (6.28), 7.923 (5.59), 7.935 (5.13), 8.401 (8.48).

LC-MS (method 1): Rₜ = 0.39 min; MS (ESlpos): m/z = 396 [M+H]⁺

### Intermediate 7-8

### 3'-(3-chloro-2-methoxyanilino)-2'-{3-[2-(methylamino)ethoxy]pyridin-4-yl}-1',7'-dihydrospiro[cyclobutane-1,6'-pyrrolo[3,2-c]pyridin]-4'(5'H)-one

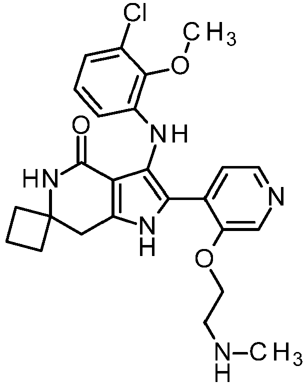

Using an analogous method as described for intermediate 7-7 with tert-butyl [2-({4-[3'-(3-chloro-2-methoxyanilino)-4'-oxo-1',4',5',7'-tetrahydrospiro[cyclobutane-1,6'-pyrrolo[3,2-c]pyridin]-2'-yl]pyridin-3-yl}oxy)ethyl]methylcarbamate (319 mg, 548 µmol, intermediate 6-8) as the starting material, 259 mg (98 % purity, 96 % yield) of the title compound were prepared after purification by preparative HPLC (method 7, gradient: 0.00-0.50 min 30% B, 0.50-6.00 min 30-50% B, 243nm DAD) under basic pH conditions.

¹H-NMR (400 MHz, DMSO-d6) δ [ppm]: 1.735 (1.02), 1.750 (1.44), 1.772 (1.06), 2.041 (1.40), 2.052 (1.37), 2.074 (1.28), 2.104 (0.76), 2.128 (1.68), 2.156 (1.24), 2.180 (0.40), 2.327 (0.57), 2.411 (10.66), 2.669 (0.52), 2.933 (2.27), 3.024 (5.62), 3.892 (16.00), 4.359 (1.64), 4.371 (2.44), 4.382 (1.64), 6.143 (1.47), 6.156 (1.76), 6.167 (1.54), 6.683 (5.87), 6.694 (3.56), 6.697 (3.27), 7.243 (2.70), 7.256 (2.74), 7.547 (5.47), 7.962 (2.77), 7.975 (2.60), 8.443 (4.31).

LC-MS (method 1): Rₜ = 0.6 min; MS (ESlpos): m/z = 482 [M+H]⁺

### Intermediate 7-9

### 3-(3-chloro-2-methoxyanilino)-6,6-dimethyl-2-{3-[2-(methylamino)ethoxy]pyridin-4-yl}-1,5,6,7-tetrahydro-4H-pyrrolo[3,2-c]pyridin-4-one

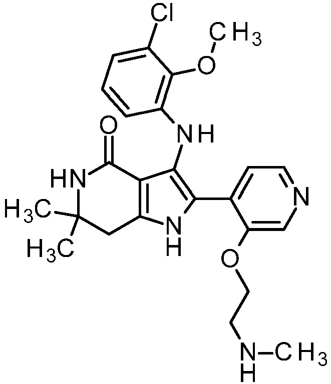

Using an analogous method as described for intermediate 7-7 with tert-butyl [2-({4-[3-(3-chloro-2-methoxyanilino)-6,6-dimethyl-4-oxo-4,5,6,7-tetrahydro-1H-pyrrolo[3,2-c]pyridin-2-yl]pyridin-3-yl}oxy)ethyl]methylcarbamate (411 mg, 720 µmol, intermediate 6-9) as the starting material, 257 mg (96 % purity, 73 % yield) of the title compound were prepared after purification by preparative HPLC (method 7, gradient: 0.00-0.50 min 28% B, 0.50-6.00 min 28-48% B, 242nm DAD) under basic conditions

¹H-NMR (400 MHz, DMSO-d6) δ [ppm]: 1.277 (16.00), 2.327 (0.43), 2.442 (6.82), 2.518 (1.66), 2.523 (1.06), 2.539 (15.46), 2.669 (0.43), 2.817 (4.96), 3.027 (1.05), 3.892 (14.97), 4.352 (1.23), 4.366 (1.84), 4.377 (1.22), 6.150 (1.26), 6.162 (1.86), 6.174 (1.33), 6.677 (4.94), 6.688 (3.19), 6.691 (2.94), 7.064 (2.66), 7.271 (2.31), 7.284 (2.32), 7.556 (3.48), 7.989 (2.17), 8.002 (2.03), 8.430 (3.73).

LC-MS (method 1): Rₜ = 0.56 min; MS (ESlpos): m/z = 470 [M+H]⁺

### Intermediate 7-10

### 3-(3-chloro-2-methoxyanilino)-2-(3-{[(2S)-pyrrolidin-2-yl]methoxy}pyridin-4-yl)-1,5,6,7-tetrahydro-4H-pyrrolo[3,2-c]pyridin-4-one

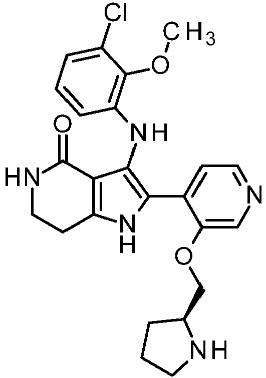

To a solution of tert-butyl (2S)-2-[({4-[3-(3-chloro-2-methoxyanilino)-4-oxo-4,5,6,7-tetrahydro-1H-pyrrolo[3,2-c]pyridin-2-yl]pyridin-3-yl}oxy)methyl]pyrrolidine-1-carboxylate (224 mg, 77 % purity, 304 µmol, intermediate 6-10) in dichloromethane (10.0 ml) TFA (230 µl, 3.0 mmol) was added and the mixture was stirred for 16h. Because of incomplete conversion another 230 µl ( 3.0 mmol) TFA were added and stirred at RT for 16h again. The solvents were removed and the residue was purified by preparative HPLC (method 6, gradient: 0.00-0.50 min 25% B, 0.50-6.00 min 25-45% B, 277nm DAD) under acidic conditions. The product rich fractions were combined, ACN was removed, the aqueous phase was basified with 1N sodium hydroxide to pH 10, extracted twice with ethyl acetate and evaporated to give 72.5 mg (98 % purity, 50 % yield) of the title compound.

¹H-NMR (400 MHz, DMSO-d6) δ [ppm]: 1.683 (0.43), 1.768 (0.41), 1.780 (0.41), 1.853 (0.40), 1.904 (0.43), 2.518 (3.67), 2.523 (2.51), 2.792 (0.48), 2.801 (0.86), 2.820 (0.92), 2.842 (1.10), 2.860 (0.51), 2.881 (0.46), 2.896 (1.10), 2.912 (0.56), 2.936 (0.73), 2.952 (0.65), 2.964 (0.44), 3.397 (0.84), 3.403 (0.87), 3.418 (1.46), 3.431 (0.76), 3.437 (0.70), 3.604 (0.41), 3.655 (0.60), 3.871 (0.51), 3.898 (16.00), 3.913 (0.52), 4.353 (0.83), 4.362 (0.87), 4.377 (0.83), 4.387 (0.73), 6.164 (1.37), 6.174 (1.21), 6.178 (1.38), 6.188 (1.38), 6.683 (0.49), 6.693 (5.70), 6.703 (2.84), 6.707 (2.59), 7.140 (1.54), 7.270 (2.56), 7.283 (2.61), 7.577 (3.37), 7.970 (3.18), 7.983 (2.81), 8.442 (4.73).

LC-MS (method 1): Rₜ = 0.53 min; MS (ESlpos): m/z = 468 [M+H]⁺

### Intermediate 7-11

### 3-{[2-(difluoromethoxy)phenyl]amino}-2-{3-[2-(methylamino)ethoxy]pyridin-4-yl}-1,5,6,7-tetrahydro-4H-pyrrolo[3,2-c]pyridin-4-one

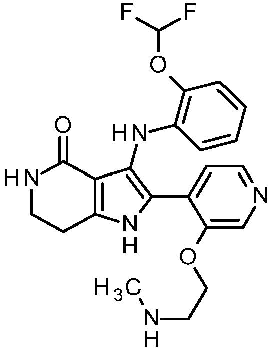

To an ice-cooled solution of tert-butyl (2-{[4-(3-{[2-(difluoromethoxy)phenyl]amino}-4-oxo-4,5,6,7-tetrahydro-1H-pyrrolo[3,2-c]pyridin-2-yl)pyridin-3-yl]oxy}ethyl)methylcarbamate (250 mg, 460 µmol, intermediate 6-11) in DCM (8ml) was added slowly TFA (708 µl). The reaction mixture was stirred at 50°C for 3h. The precipitate was filtered off, dissolved in water, sat. sodium hydrogen carbonate was added, the mixture was extracted three times into ethyl acetate, filtered and evaporated in vacuum to give (135 mg, 66% yield) of the title compound.

¹H-NMR (400MHz, DMSO-*d*₆): δ [ppm]= 8.42 (s, 1H), 7.92 (d, 1H), 7.47 (s, 1H), 7.00 - 7.42 (m, 4H), 6.81 (t, 1H), 6.67 (td, 1H), 6.33 (dd, 1H), 4.36 (t, 2H), 3.37 - 3.45 (m, 2H), 2.82 - 2.95 (m, 4H), 2.35 - 2.43 (m, 3H).

### Syntheses of examples

### Example 1

### N-[2-({4-[3-(3-chloro-2-methoxyanilino)-4-oxo-4,5,6,7-tetrahydro-1H-pyrrolo[3,2-c]pyridin-2-yl]pyridin-3-yl}oxy)ethyl]prop-2-enamide

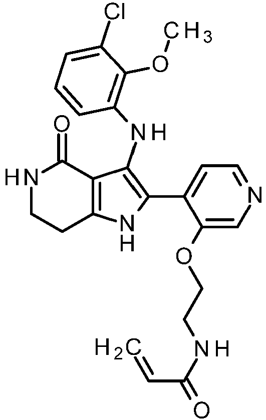

To a solution of 2-[3-(2-aminoethoxy)pyridin-4-yl]-3-(3-chloro-2-methoxyanilino)-1,5,6,7-tetrahydro-4H-pyrrolo[3,2-c]pyridin-4-one (105 mg, 245 µmol, intermediate 7-1) in dry DMF (3.3 ml) was added prop-2-enoic acid (25 µl, 370 µmol) followed by N,N-diisopropylethylamine (260 µl, 1.5 mmol) and propanephosphonic acid anhydride (220 µl, 50 % purity, solution in DMF, 370 µmol; CAS-RN:[68957-94-8]) and the mixture was stirred under argon at RT for 1h. The reaction mixture was quenched with 100µl water and the solution then purified by preparative HPLC (method 8, gradient: 0.00-0.50 min 25% B, 0.50-6.00 min 25-45% B, 277nm) under neutral pH conditions to give 60.0 mg (97 % purity, 49 % yield) of the title compound.

¹H-NMR (400 MHz, DMSO-d6) δ [ppm]: 2.327 (0.42), 2.518 (1.43), 2.523 (0.95), 2.669 (0.42), 2.990 (1.07), 3.007 (2.35), 3.024 (1.25), 3.398 (0.89), 3.404 (0.94), 3.415 (1.60), 3.421 (1.56), 3.432 (0.78), 3.438 (0.72), 3.675 (0.59), 3.689 (1.39), 3.701 (1.45), 3.715 (0.72), 3.898 (16.00), 3.920 (0.43), 4.245 (1.26), 4.257 (2.13), 4.270 (1.18), 5.652 (1.37), 5.658 (1.24), 5.677 (1.36), 5.683 (1.56), 6.109 (1.49), 6.119 (1.48), 6.123 (1.30), 6.133 (1.47), 6.147 (0.85), 6.152 (0.92), 6.190 (1.90), 6.195 (1.81), 6.255 (1.82), 6.280 (1.72), 6.298 (0.87), 6.323 (0.79), 6.688 (2.89), 6.692 (3.22), 6.702 (5.71), 6.712 (0.44), 7.162 (1.55), 7.349 (2.67), 7.362 (2.70), 7.564 (3.56), 7.980 (3.19), 7.993 (2.94), 8.388 (3.91), 8.609 (0.52), 8.624 (1.06), 8.639 (0.52), 11.125 (1.65).

LC-MS (method 1): Rₜ = 0.68 min; MS (ESlpos): m/z = 482 [M+H]⁺

### Example 2

### N-[2-({4-[3-(3-fluoro-2-methylanilino)-4-oxo-4,5,6,7-tetrahydro-1H-pyrrolo[3,2-c]pyridin-2-yl]pyridin-3-yl}oxy)ethyl]prop-2-enamide

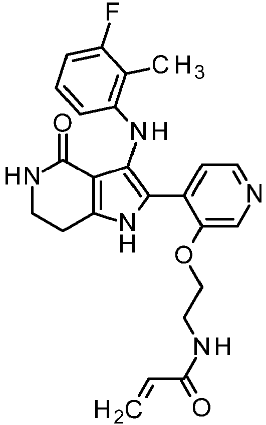

To a solution of 2-[3-(2-aminoethoxy)pyridin-4-yl]-3-(3-fluoro-2-methylanilino)-1,5,6,7-tetrahydro-4H-pyrrolo[3,2-c]pyridin-4-one (35.3 mg, 89.3 µmol, intermediate 7-3) in dry DMF (1,2 ml) was added prop-2-enoic acid (9.2 µl, 130 µmol) followed by N,N-diisopropylethylamine (93 µl, 540 µmol; CAS-RN:[7087-68-5]) and propanephosphonic acid anhydride (80 µl, 50 % purity, solution in DMF, 130 µmol; CAS-RN:[68957-94-8]) and the mixture was stirred under argon at RT for 0,5h. The reaction mixture was quenched with 100µl water and the solution then purified by preparative HPLC (method 8, gradient: 0.00-0.50 min 25% B, 0.50-6.00 min 25-45% B, 277nm) under neutral pH conditions to give 8.80 mg (98 % purity, 21 % yield) of the title compound.

¹H-NMR (400 MHz, DMSO-d6) δ [ppm]: 1.232 (0.68), 2.074 (2.07), 2.201 (16.00), 2.888 (0.44), 3.000 (2.79), 3.017 (6.16), 3.034 (3.32), 3.409 (2.50), 3.419 (4.20), 3.425 (4.16), 3.436 (2.17), 3.688 (3.74), 3.700 (3.92), 3.713 (1.79), 4.227 (3.35), 4.239 (5.60), 4.252 (3.15), 5.656 (3.15), 5.662 (2.85), 5.681 (3.03), 5.687 (3.48), 6.026 (3.78), 6.046 (3.93), 6.152 (1.83), 6.157 (1.97), 6.194 (4.41), 6.200 (4.09), 6.257 (4.00), 6.282 (3.89), 6.300 (1.90), 6.324 (1.70), 6.456 (1.71), 6.478 (3.22), 6.499 (2.01), 6.746 (1.16), 6.766 (2.49), 6.784 (2.40), 6.804 (1.00), 7.189 (3.92), 7.309 (5.97), 7.322 (5.95), 7.369 (7.45), 7.933 (6.41), 7.947 (5.85), 8.360 (9.10), 8.619 (1.37), 8.634 (2.78), 8.648 (1.36), 11.082 (4.24).

LC-MS (method 1): Rₜ = 0.64 min; MS (ESlpos): m/z = 450 [M+H]⁺

### Example 3

### N-[2-({4-[3-(3-chloro-2-methylanilino)-4-oxo-4,5,6,7-tetrahydro-1H-pyrrolo[3,2-c]pyridin-2-yl]pyridin-3-yl}oxy)ethyl]prop-2-enamide

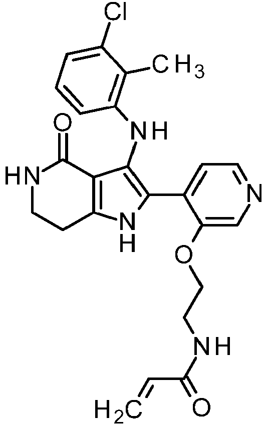

Using an analogous method as described for example 2 with 2-[3-(2-aminoethoxy)pyridin-4-yl]-3-(3-chloro-2-methylanilino)-1,5,6,7-tetrahydro-4H-pyrrolo[3,2-c]pyridin-4-one (81.5 mg, 198 µmol, intermediate 7-2) and prop-2-enoic acid (20 µl, 300 µmol) as the starting materials 47.7 mg (98 % purity, 51 % yield) of the title compound were prepared after purification by preparative HPLC (method 8, gradient: 0.00-0.50 min 28% B, 0.50-6.00 min 28-48% B, 279nm ) under neutral conditions

¹H-NMR (400 MHz, DMSO-d6) δ [ppm]: 2.074 (1.59), 2.367 (16.00), 3.002 (1.72), 3.019 (3.68), 3.036 (2.03), 3.407 (1.64), 3.419 (2.61), 3.424 (2.62), 3.436 (1.35), 3.689 (2.29), 3.702 (2.41), 4.230 (2.09), 4.243 (3.45), 4.255 (2.01), 5.656 (1.86), 5.662 (1.78), 5.681 (1.81), 5.687 (2.14), 6.151 (1.21), 6.161 (2.05), 6.180 (2.05), 6.184 (2.08), 6.193 (2.66), 6.199 (2.56), 6.257 (2.39), 6.281 (2.33), 6.299 (1.14), 6.324 (1.06), 6.737 (1.22), 6.753 (3.39), 6.757 (3.10), 6.765 (2.83), 6.785 (2.65), 6.804 (0.88), 7.188 (2.45), 7.306 (3.69), 7.319 (3.68), 7.382 (4.70), 7.938 (4.04), 7.952 (3.70), 8.363 (5.70), 8.619 (0.83), 8.634 (1.71), 8.649 (0.87), 11.097 (2.70).

LC-MS (method 1): Rₜ = 0.7 min; MS (ESlpos): m/z = 466 [M+H]⁺

### Example 4

### N-[3-({4-[3-(3-chloro-2-methoxyanilino)-4-oxo-4,5,6,7-tetrahydro-1H-pyrrolo[3,2-c]pyridin-2-yl]pyridin-3-yl}oxy)propyl]prop-2-enamide

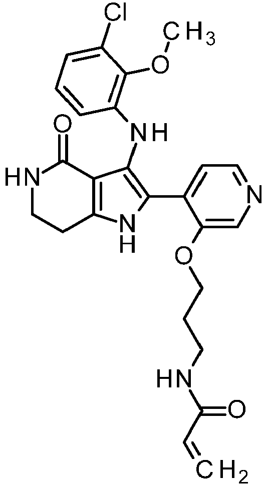

To a solution of 2-[3-(3-aminopropoxy)pyridin-4-yl]-3-(3-chloro-2-methoxyanilino)-1,5,6,7-tetrahydro-4H-pyrrolo[3,2-c]pyridin-4-one (100 mg, 226 µmol, intermediate 7-4) in dry DMF (6 ml) was added prop-2-enoic acid (23 µl, 340 µmol) followed by N,N-diisopropylethylamine (300 µl, 1.7 mmol; CAS-RN:[7087-68-5]) and propanephosphonic acid anhydride (200 µl, 50 % purity, 340 µmol; CAS-RN:[68957-94-8]) and the mixture was stirred under argon at RT for 20min. The reaction mixture was quenched with a few drops of water and the solution then purified by preparative HPLC (method 7, gradient: 0.00-0.50 min 25% B, 0.50-6.00 min 25-45% B, 277nm) under basisc pH conditions followed by flash chromatography (silica, DCM / ethanol gradient 0-10 %, DCM / ethanol gradient 10 % + 1 promille TEA ) to give 41.5 mg (93 % purity, 34 % yield) of the title compound.

¹H-NMR (400 MHz, DMSO-d6) δ [ppm]: 1.035 (1.19), 1.052 (2.40), 1.070 (1.27), 1.154 (0.73), 1.172 (1.50), 1.189 (0.81), 1.936 (1.23), 1.952 (1.88), 1.968 (1.31), 1.987 (2.34), 2.326 (0.63), 2.668 (0.66), 2.863 (1.29), 2.880 (2.75), 2.897 (1.51), 3.353 (2.47), 3.370 (0.93), 3.397 (1.16), 3.407 (2.01), 3.413 (1.96), 3.422 (1.42), 3.434 (1.04), 3.439 (0.73), 3.452 (0.67), 3.856 (16.00), 4.017 (0.50), 4.035 (0.56), 4.177 (1.36), 4.192 (2.82), 4.207 (1.40), 4.356 (0.73), 5.594 (1.46), 5.600 (1.39), 5.618 (1.46), 5.624 (1.66), 5.758 (7.10), 6.075 (0.91), 6.081 (1.04), 6.118 (2.09), 6.124 (2.09), 6.139 (1.58), 6.145 (1.55), 6.157 (1.58), 6.163 (1.64), 6.191 (1.97), 6.216 (1.86), 6.233 (0.99), 6.259 (0.88), 6.633 (0.77), 6.646 (3.74), 6.649 (3.85), 6.652 (3.00), 6.666 (2.28), 6.687 (0.61), 7.095 (1.83), 7.272 (2.82), 7.284 (2.88), 7.447 (3.81), 8.007 (3.32), 8.019 (3.18), 8.295 (0.59), 8.309 (1.18), 8.324 (0.64), 8.379 (4.69), 11.352 (2.05).

LC-MS (method 1): Rₜ = 0.67 min; MS (ESlpos): m/z = 496 [M+H]⁺

### Example 5

### N-[2-({4-[3-(3-chloro-2-methoxyanilino)-4-oxo-4,5,6,7-tetrahydro-1H-pyrrolo[3,2-c]pyridin-2-yl]pyridin-3-yl}oxy)ethyl]-N-methylprop-2-enamide

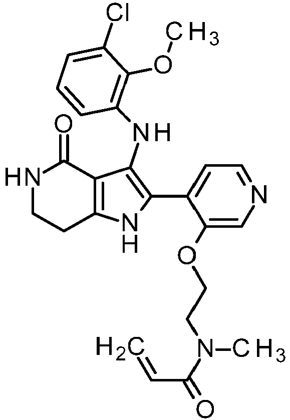

Using an analogous method as described for example 4 with 3-(3-chloro-2-methoxyanilino)-2-{3-[2-(methylamino)ethoxy]pyridin-4-yl}-1,5,6,7-tetrahydro-4H-pyrrolo[3,2-c]pyridin-4-one (100 mg, 226 µmol, intermediate 7-5) and prop-2-enoic acid (23 µl, 340 µmol) as the starting materials, which were stirred for 45 min at RT. The reaction mixture was quenched with 100µl water and the solution then purified by preparative HPLC (method 7, gradient: 0.00-0.50 min 28% B, 0.50-6.00 min 28-48% B, 275nm) under basisc pH conditions followed by flash chromatography (silica, DCM / ethanol gradient 0-5 % to give 60.0 mg (98 % purity, 52 % yield) of the titel compound.

¹H-NMR (400 MHz, DMSO-d6) δ [ppm]: 1.154 (1.54), 1.172 (3.21), 1.190 (1.62), 1.988 (5.57), 2.323 (0.42), 2.327 (0.55), 2.331 (0.41), 2.523 (2.91), 2.665 (0.43), 2.669 (0.60), 2.673 (0.44), 2.817 (0.42), 2.834 (0.91), 2.851 (0.52), 2.903 (4.42), 3.002 (1.23), 3.020 (2.70), 3.036 (1.51), 3.157 (13.97), 3.400 (1.78), 3.410 (2.30), 3.416 (2.25), 3.842 (4.89), 3.856 (1.09), 3.870 (0.68), 3.896 (16.00), 3.911 (1.73), 3.923 (2.50), 3.934 (1.66), 3.999 (0.44), 4.017 (1.28), 4.035 (1.24), 4.213 (0.49), 4.226 (0.92), 4.239 (0.45), 4.364 (1.57), 4.376 (2.43), 4.387 (1.48), 5.501 (0.43), 5.508 (0.42), 5.527 (0.42), 5.534 (0.45), 5.729 (1.48), 5.734 (1.35), 5.755 (1.36), 5.760 (1.66), 6.034 (0.44), 6.039 (0.45), 6.068 (0.44), 6.075 (0.46), 6.084 (0.54), 6.093 (1.75), 6.101 (1.51), 6.110 (1.39), 6.117 (1.50), 6.174 (1.29), 6.180 (1.34), 6.215 (1.50), 6.221 (1.50), 6.597 (0.74), 6.607 (0.90), 6.614 (1.60), 6.661 (0.53), 6.682 (2.56), 6.690 (2.98), 6.698 (5.77), 6.710 (0.69), 6.782 (0.42), 6.792 (1.44), 6.818 (1.44), 6.834 (1.37), 6.860 (1.18), 7.087 (0.60), 7.164 (1.83), 7.263 (0.82), 7.275 (0.82), 7.359 (2.79), 7.372 (2.81), 7.443 (1.17), 7.538 (3.78), 7.975 (3.34), 7.988 (3.13), 8.055 (0.93), 8.068 (0.85), 8.358 (1.41), 8.380 (4.43), 11.137 (1.99), 11.171 (0.73).

LC-MS (method 1): Rₜ = 0.71 min; MS (ESlpos): m/z = 496 [M+H]⁺

### Example 6

### N-[2-({4-[3-(3-fluoro-2-methoxyanilino)-4-oxo-4,5,6,7-tetrahydro-1H-pyrrolo[3,2-c]pyridin-2-yl]pyridin-3-yl}oxy)ethyl]-N-methylprop-2-enamide

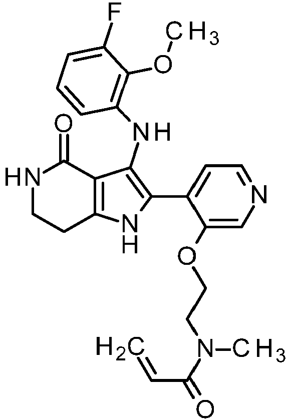

Using an analogous method as described for example 1 with 3-(3-fluoro-2-methoxyanilino)-2-{3-[2-(methylamino)ethoxy]pyridin-4-yl}-1,5,6,7-tetrahydro-4H-pyrrolo[3,2-c]pyridin-4-one (100 mg, 235 µmol, intermediate 7-6) and prop-2-enoic acid (24 µl, 350 µmol) as the starting materials The reaction mixture was quenched with 100µl water and the solution then purified by preparative HPLC under basic pH conditions (method 7, gradient: 0.00-0.50 min 28% B, 0.50-6.00 min 28-48% B, 244nm) followed by flash chromatography (silica, DCM / ethanol gradient 0-5 %) to give 45.0 mg (97 % purity, 39 % yield) of the title compound.

¹H-NMR (400 MHz, DMSO-d6) δ [ppm]: 0.884 (0.68), 0.901 (1.36), 0.920 (0.74), 1.053 (0.49), 2.065 (2.02), 2.327 (0.50), 2.419 (0.47), 2.437 (0.49), 2.522 (1.61), 2.669 (0.52), 2.813 (0.45), 2.831 (0.96), 2.847 (0.52), 2.903 (4.52), 3.002 (1.36), 3.019 (2.96), 3.036 (1.61), 3.159 (14.68), 3.399 (1.88), 3.410 (2.52), 3.415 (2.43), 3.843 (0.49), 3.857 (1.04), 3.876 (4.40), 3.924 (16.00), 3.937 (1.95), 4.191 (0.54), 4.204 (1.00), 4.217 (0.50), 4.362 (1.73), 4.374 (2.67), 4.386 (1.61), 5.497 (0.43), 5.503 (0.41), 5.523 (0.43), 5.529 (0.48), 5.729 (1.52), 5.734 (1.43), 5.755 (1.53), 5.758 (3.18), 5.920 (0.54), 5.941 (0.61), 5.953 (1.67), 5.974 (1.72), 5.988 (0.46), 5.995 (0.44), 6.030 (0.46), 6.036 (0.46), 6.174 (1.37), 6.179 (1.39), 6.216 (1.56), 6.221 (1.59), 6.487 (0.69), 6.490 (0.71), 6.507 (1.04), 6.511 (1.16), 6.518 (0.92), 6.535 (1.22), 6.538 (1.20), 6.616 (0.74), 6.631 (0.89), 6.636 (1.32), 6.651 (1.25), 6.657 (0.70), 6.672 (0.53), 6.770 (0.42), 6.793 (1.53), 6.819 (1.51), 6.834 (1.37), 6.860 (1.22), 7.090 (0.65), 7.164 (1.97), 7.267 (0.84), 7.279 (0.85), 7.371 (2.90), 7.383 (2.93), 7.476 (1.22), 7.537 (3.97), 7.972 (3.39), 7.984 (3.14), 8.057 (0.95), 8.069 (0.88), 8.345 (1.45), 8.375 (4.69), 11.119 (2.13), 11.151 (0.76).

LC-MS (method 1): Rₜ = 0.66 min; MS (ESlpos): m/z = 480 [M+H]⁺

### Example 7

### N-[2-({4-[3-(4-fluoroanilino)-4-oxo-4,5,6,7-tetrahydro-1H-pyrrolo[3,2-c]pyridin-2-yl]pyridin-3-yl}oxy)ethyl]-N-methylprop-2-enamide

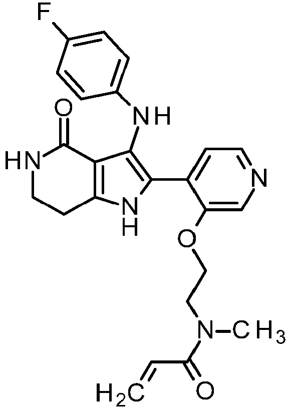

Using an analogous method as described for example 4 with 3-(4-fluoroanilino)-2-{3-[2-(methylamino)ethoxy]pyridin-4-yl}-1,5,6,7-tetrahydro-4H-pyrrolo[3,2-c]pyridin-4-one (99.7 mg, 252 µmol, intermediate 7-7) and prop-2-enoic acid (26 µl, 380 µmol) as the starting materials, which were stirred for 30 min at RT. The reaction mixture was quenched with 100µl water and the solution then purified by preparative HPLC (method 8, gradient: 0.00-0.50 min 20% B, 0.50-6.00 min 20-40% B, 277nm) under neutral pH conditions to give 40.6 mg (99 % purity, 35 % yield) of the title compound.

¹H-NMR (400 MHz, DMSO-d6) δ [ppm]: 2.075 (0.61), 2.322 (1.16), 2.327 (1.65), 2.332 (1.19), 2.518 (8.22), 2.522 (4.95), 2.665 (1.19), 2.669 (1.68), 2.673 (1.24), 2.802 (0.49), 2.819 (0.98), 2.836 (0.52), 2.905 (4.80), 3.001 (1.48), 3.018 (3.21), 3.035 (1.76), 3.161 (16.00), 3.391 (2.14), 3.403 (2.66), 3.408 (2.63), 3.418 (1.30), 3.855 (1.01), 3.868 (0.58), 3.915 (1.65), 3.927 (2.72), 3.938 (1.82), 4.148 (0.55), 4.161 (1.01), 4.174 (0.52), 4.343 (1.85), 4.355 (2.81), 4.366 (1.74), 5.495 (0.43), 5.501 (0.43), 5.522 (0.43), 5.527 (0.49), 5.732 (1.68), 5.738 (1.53), 5.758 (1.56), 5.764 (1.85), 5.996 (0.41), 6.002 (0.43), 6.038 (0.49), 6.044 (0.46), 6.177 (1.48), 6.183 (1.50), 6.218 (1.68), 6.224 (1.71), 6.485 (0.58), 6.497 (0.64), 6.507 (0.78), 6.519 (0.78), 6.537 (2.05), 6.548 (2.20), 6.554 (1.53), 6.559 (2.60), 6.571 (2.46), 6.743 (0.67), 6.764 (1.27), 6.786 (0.78), 6.800 (2.03), 6.826 (2.23), 6.833 (2.58), 6.841 (2.03), 6.855 (4.25), 6.867 (1.76), 6.877 (2.11), 7.059 (0.69), 7.136 (2.11), 7.234 (0.87), 7.246 (0.87), 7.357 (1.22), 7.425 (3.33), 7.437 (4.98), 7.931 (3.41), 7.944 (3.15), 8.021 (0.93), 8.033 (0.90), 8.293 (1.39), 8.343 (4.69), 11.058 (2.69).

LC-MS (method 1): Rₜ = 0.61 min; MS (ESlpos): m/z = 450 [M+H]⁺

### Example 8

### (2E)-4-(dimethylamino)-N-[2-({4-[3-(3-fluoro-2-methoxyanilino)-4-oxo-4,5,6,7-tetrahydro-1H-pyrrolo[3,2-c]pyridin-2-yl]pyridin-3-yl}oxy)ethyl]-N-methylbut-2-enamide

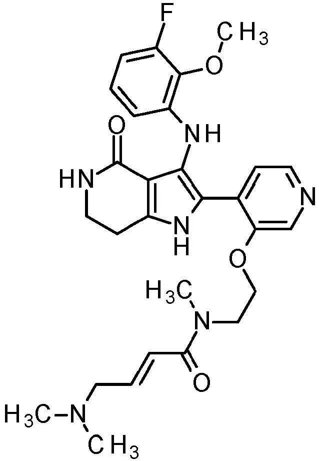

Using an analogous method as described for example 4 with 3-(3-fluoro-2-methoxyanilino)-2-{3-[2-(methylamino)ethoxy]pyridin-4-yl}-1,5,6,7-tetrahydro-4H-pyrrolo[3,2-c]pyridin-4-one (100 mg, 235 µmol, intermediate 7-6) and (2E)-4-(dimethylamino)but-2-enoic acid-hydrogen chloride (1/1) (58.4 mg, 353 µmol; CAS-RN:[848133-35-7]) as the starting materials, which were stirred for 40 min at RT. The reaction mixture was quenched with a few drops of water and the solution then purified by preparative HPLC (method 8, gradient: 0.00-0.50 min 28% B, 0.50-6.00 min 28-48% B, 245nm ) under neutral pH conditions to give 40.0 mg (96 % purity, 30 % yield) of the title compound.

¹H-NMR (400 MHz, DMSO-d6) δ [ppm]: 2.037 (3.29), 2.115 (16.00), 2.326 (0.45), 2.668 (0.43), 2.834 (0.44), 2.868 (0.58), 2.901 (1.63), 3.003 (0.94), 3.021 (3.56), 3.036 (2.85), 3.145 (7.14), 3.410 (1.70), 3.825 (0.43), 3.882 (1.74), 3.922 (9.26), 4.227 (0.42), 4.356 (1.02), 4.367 (1.57), 5.947 (1.07), 5.967 (1.10), 6.489 (0.45), 6.518 (1.14), 6.535 (0.74), 6.587 (0.64), 6.625 (1.68), 6.649 (0.78), 6.661 (0.66), 6.676 (1.10), 6.690 (0.51), 6.714 (0.45), 7.167 (1.25), 7.374 (1.51), 7.386 (1.55), 7.505 (0.53), 7.534 (2.30), 7.966 (1.60), 7.979 (1.55), 8.370 (2.82), 11.096 (0.41), 11.118 (1.41).

LC-MS (method 1): Rₜ = 0.48 min; MS (ESlpos): m/z = 537 [M+H]⁺

### Example 9

### (2E)-N-[2-({4-[3-(3-chloro-2-methoxyanilino)-4-oxo-4,5,6,7-tetrahydro-1H-pyrrolo[3,2-c]pyridin-2-yl]pyridin-3-yl}oxy)ethyl]-4-(dimethylamino)-N-methylbut-2-enamide

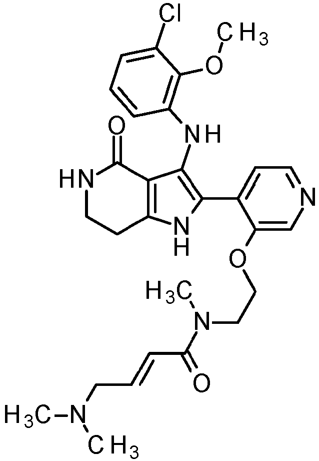

Using an analogous method as described for example 4 with 3-(3-chloro-2-methoxyanilino)-2-{3-[2-(methylamino)ethoxy]pyridin-4-yl}-1,5,6,7-tetrahydro-4H-pyrrolo[3,2-c]pyridin-4-one (50.0 mg, 113 µmol, intermediate 7-5) and (2E)-4-(dimethylamino)but-2-enoic acid-hydrogen chloride (1/1) (28.1 mg, 170 µmol; CAS-RN:[848133-35-7]) as the starting materials, which were stirred for 20 min at RT. The reaction mixture was quenched with a few drops of water and the solution then purified by preparative HPLC (method 7, gradient: 0.00-0.50 min 26% B, 0.50-6.00 min 26-46% B, 279nm) under basisc pH conditions to give 40.0 mg (95 % purity, 61 % yield) of the title compound.

¹H-NMR (400 MHz, DMSO-d6) δ [ppm]: 2.032 (3.44), 2.111 (16.00), 2.665 (0.41), 2.834 (0.48), 2.860 (0.69), 2.898 (1.68), 3.017 (3.80), 3.031 (2.90), 3.139 (7.11), 3.408 (1.85), 3.821 (0.53), 3.845 (1.87), 3.893 (8.43), 3.913 (1.88), 4.247 (0.47), 4.365 (1.71), 6.084 (1.13), 6.091 (1.15), 6.101 (1.17), 6.108 (1.18), 6.514 (0.81), 6.581 (0.69), 6.619 (2.05), 6.656 (0.84), 6.675 (1.86), 6.686 (2.29), 6.692 (3.15), 6.708 (0.67), 7.162 (1.33), 7.359 (1.50), 7.372 (1.49), 7.473 (0.52), 7.533 (2.29), 7.967 (1.62), 7.979 (1.51), 8.372 (3.43), 11.107 (0.45), 11.133 (1.49).

LC-MS (method 1): Rₜ = 0.53 min; MS (ESlpos): m/z = 553 [M+H]⁺

### Example 10

### (2E)-N-[2-({4-[3'-(3-chloro-2-methoxyanilino)-4'-oxo-1',4',5',7'-tetrahydrospiro[cyclobutane-1,6'-pyrrolo[3,2-c]pyridin]-2'-yl]pyridin-3-yl}oxy)ethyl]-4-(dimethylamino)-N-methylbut-2-enamide

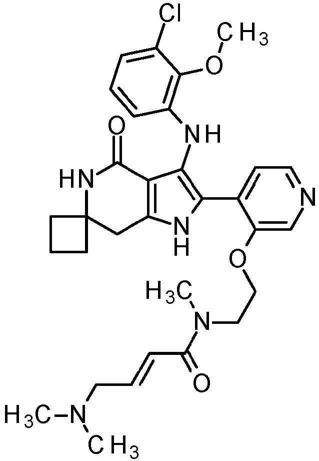

Using an analogous method as described for example 4 with 3'-(3-chloro-2-methoxyanilino)-2'-{3-[2-(methylamino)ethoxy]pyridin-4-yl}-1',7'-dihydrospiro[cyclobutane-1,6'-pyrrolo[3,2-c]pyridin]-4'(5'H)-one (54.4 mg, 113 µmol, intermediate 7-8) and (2E)-4-(dimethylamino)but-2-enoic acid-hydrogen chloride (1/1) (28.0 mg, 169 µmol; CAS-RN:[848133-35-7]) as the starting materials, which were stirred for 25 min at RT. The reaction mixture was quenched with 100µl water and the solution then purified by preparative HPLC (method 8, gradient: 0.00-0.50 min 35% B, 0.50-6.00 min 35-55% B, 280nm) under neutral pH conditions to give 34.2 mg (99 % purity, 51 % yield) of the title compound.

¹H-NMR (400 MHz, DMSO-d6) δ [ppm]: 1.720 (0.48), 1.740 (0.82), 1.758 (0.71), 2.019 (0.85), 2.059 (2.63), 2.121 (16.00), 2.137 (1.22), 2.163 (0.75), 2.327 (0.40), 2.898 (1.43), 3.006 (0.59), 3.022 (1.60), 3.037 (1.58), 3.152 (6.89), 3.224 (2.66), 3.845 (1.62), 3.896 (8.50), 3.940 (1.25), 4.370 (0.84), 4.382 (1.27), 6.071 (1.00), 6.078 (1.06), 6.088 (1.01), 6.095 (1.03), 6.532 (0.54), 6.614 (0.75), 6.623 (0.54), 6.651 (1.24), 6.678 (1.27), 6.690 (1.75), 6.697 (2.73), 6.710 (0.54), 6.718 (1.05), 6.733 (0.42), 6.756 (0.47), 7.362 (1.46), 7.375 (1.45), 7.507 (2.23), 7.574 (1.67), 7.965 (1.66), 7.978 (1.53), 8.375 (3.08), 11.183 (1.23).

LC-MS (method 1): Rₜ = 0.62 min; MS (ESlpos): m/z = 593[M+H]⁺

### Example 11

### N-[2-({4-[3'-(3-chloro-2-methoxyanilino)-4'-oxo-1',4',5',7'-tetrahydrospiro[cyclobutane-1,6'-pyrrolo[3,2-c]pyridin]-2'-yl]pyridin-3-yl}oxy)ethyl]-N-methylprop-2-enamide

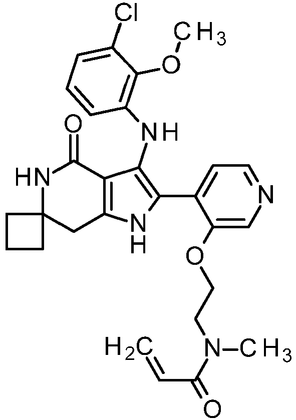

Using an analogous method as described for example 4 with 3'-(3-chloro-2-methoxyanilino)-2'-{3-[2-(methylamino)ethoxy]pyridin-4-yl}-1',7'-dihydrospiro[cyclobutane-1,6'-pyrrolo[3,2-c]pyridin]-4'(5'H)-one (70.0 mg, 145 µmol, intermediate 7-8) and prop-2-enoic acid (15 µl, 220 µmol) as the starting materials, which were stirred for 30 min at RT. The reaction mixture was quenched with 100µl water and the solution then purified by preparative HPLC (method 8, gradient: 0.00-0.50 min 35% B, 0.50-6.00 min 35-55% B, 245nm) under neutral pH conditions to give 45.9 mg (98 % purity, 58 % yield) of the title compound.

¹H-NMR (400 MHz, DMSO-d6) δ [ppm]: 0.967 (0.44), 1.692 (0.52), 1.714 (1.02), 1.738 (1.28), 1.751 (0.91), 1.998 (0.69), 2.021 (1.55), 2.041 (1.21), 2.074 (0.75), 2.109 (0.74), 2.133 (1.75), 2.157 (1.34), 2.182 (0.43), 2.322 (0.53), 2.326 (0.70), 2.331 (0.51), 2.518 (4.37), 2.522 (2.87), 2.664 (0.51), 2.669 (0.69), 2.673 (0.49), 2.727 (0.47), 2.888 (0.68), 2.900 (3.68), 3.004 (1.44), 3.164 (13.92), 3.216 (4.96), 3.841 (3.79), 3.873 (0.90), 3.895 (16.00), 3.939 (1.42), 3.951 (2.24), 3.963 (1.54), 4.222 (0.42), 4.236 (0.79), 4.377 (1.54), 4.389 (2.32), 4.401 (1.45), 5.744 (1.44), 5.750 (1.32), 5.770 (1.33), 5.776 (1.63), 6.052 (0.49), 6.058 (0.48), 6.070 (1.87), 6.077 (1.86), 6.086 (1.49), 6.094 (1.52), 6.197 (1.26), 6.203 (1.29), 6.238 (1.45), 6.245 (1.48), 6.595 (0.58), 6.609 (0.83), 6.614 (1.06), 6.659 (0.59), 6.680 (2.48), 6.690 (3.18), 6.697 (5.57), 6.710 (0.73), 6.822 (1.48), 6.848 (1.45), 6.864 (1.35), 6.889 (1.16), 7.263 (0.68), 7.276 (0.69), 7.358 (2.80), 7.371 (2.81), 7.436 (1.00), 7.508 (4.13), 7.569 (3.03), 7.969 (3.36), 7.982 (3.10), 8.051 (0.79), 8.063 (0.73), 8.361 (1.22), 8.380 (4.47), 11.149 (2.08), 11.198 (0.65).

LC-MS (method 1): Rₜ = 0.85 min; MS (ESlpos): m/z = 536 [M+H]⁺

### Example 12

### N-[2-({4-[3-(3-chloro-2-methoxyanilino)-6,6-dimethyl-4-oxo-4,5,6,7-tetrahydro-1H-pyrrolo[3,2-c]pyridin-2-yl]pyridin-3-yl}oxy)ethyl]-N-methylprop-2-enamide

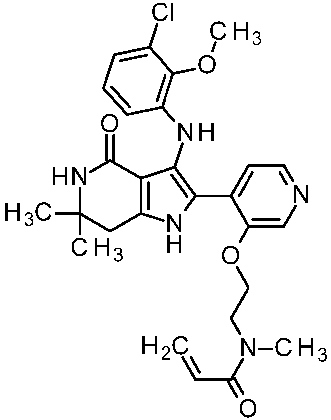

Using an analogous method as described for example 4 with 3-(3-chloro-2-methoxyanilino)-6,6-dimethyl-2-{3-[2-(methylamino)ethoxy]pyridin-4-yl}-1,5,6,7-tetrahydro-4H-pyrrolo[3,2-c]pyridin-4-one (128 mg, 273 µmol, intermediate 7-9) and prop-2-enoic acid (28 µl, 410 µmol) as the starting materials, which were stirred for 30 min at RT. The reaction mixture was quenched with 100µl water and the solution then purified by preparative HPLC (method 8, gradient: 0.00-0.50 min 33% B, 0.50-6.00 min 33-35% B, 245nm) under neutral pH conditions to give 50.0 mg (99 % purity, 35 % yield) of the title compound.

¹H-NMR (400 MHz, DMSO-d6) δ [ppm]: 1.239 (0.49), 1.264 (16.00), 2.318 (0.40), 2.322 (0.89), 2.327 (1.31), 2.332 (0.96), 2.336 (0.42), 2.518 (5.06), 2.523 (3.31), 2.660 (0.42), 2.665 (0.94), 2.669 (1.36), 2.673 (0.96), 2.678 (0.42), 2.796 (1.11), 2.891 (2.84), 2.992 (4.05), 3.152 (10.52), 3.843 (3.01), 3.864 (0.64), 3.877 (0.42), 3.898 (12.25), 3.918 (1.06), 3.931 (1.65), 3.941 (1.11), 4.220 (0.57), 4.365 (1.11), 4.378 (1.68), 4.388 (1.04), 5.732 (1.11), 5.738 (0.99), 5.758 (0.99), 5.763 (1.21), 6.091 (1.26), 6.100 (1.28), 6.107 (1.06), 6.115 (1.14), 6.165 (0.96), 6.171 (0.99), 6.207 (1.09), 6.212 (1.14), 6.603 (0.49), 6.610 (0.57), 6.619 (1.06), 6.682 (1.93), 6.689 (2.35), 6.698 (4.44), 6.710 (0.44), 6.795 (1.31), 6.821 (1.28), 6.837 (1.01), 6.862 (0.91), 7.011 (0.62), 7.088 (2.22), 7.275 (0.49), 7.287 (0.49), 7.366 (1.98), 7.379 (2.02), 7.449 (0.77), 7.514 (2.81), 7.970 (2.15), 7.983 (2.00), 8.055 (0.52), 8.067 (0.52), 8.349 (0.81), 8.373 (2.96), 11.100 (1.48), 11.159 (0.44).

LC-MS (method 1): Rₜ = 0.79 min; MS (ESlpos): m/z = 534 [M+H]⁺

### Example 13

### 3-(3-chloro-2-methoxyanilino)-2-(3-{[(2S)-1-(prop-2-enoyl)pyrrolidin-2-yl]methoxy}pyridin-4-yl)-1,5,6,7-tetrahydro-4H-pyrrolo[3,2-c]pyridin-4-one

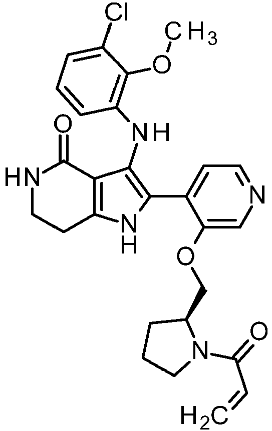

Using an analogous method as described for example 4 with 3-(3-chloro-2-methoxyanilino)-2-(3-{[(2S)-pyrrolidin-2-yl]methoxy}pyridin-4-yl)-1,5,6,7-tetrahydro-4H-pyrrolo[3,2-c]pyridin-4-one (36.0 mg, 76.9 µmol, intermediate 7-10) and prop-2-enoic acid (7.9 µl, 120 µmol) as the starting materials, which were stirred at RT for 16h. The reaction mixture was purified by preparative HPLC (method 7, gradient: 0.00-0.50 min 30% B, 0.50-6.00 min 30-50% B, 249 nm). The product rich fractions were evaporated to remove ACN, the remaining water was extracted twice with ethyl acetate, the solvent was removed to give 4.00 mg (90 % purity, 9% yield) of the title compound.

H-NMR (400 MHz, DMSO-d6) δ [ppm]: 0.697 (0.49), 0.828 (0.43), 0.848 (0.77), 0.867 (0.46), 1.232 (3.13), 1.256 (0.60), 1.295 (0.44), 1.513 (6.49), 1.886 (0.99), 1.901 (1.12), 1.910 (1.00), 1.930 (0.95), 1.944 (0.70), 1.963 (0.78), 1.983 (0.78), 1.998 (0.97), 2.013 (0.99), 2.038 (0.41), 2.518 (4.12), 2.523 (2.62), 2.843 (0.56), 2.956 (1.16), 2.972 (2.31), 2.990 (1.31), 3.164 (0.73), 3.246 (0.82), 3.383 (1.07), 3.396 (1.82), 3.404 (1.86), 3.413 (2.59), 3.419 (2.72), 3.431 (1.26), 3.572 (0.70), 3.636 (1.40), 3.645 (1.11), 3.829 (1.91), 3.836 (2.64), 3.881 (16.00), 3.886 (3.42), 4.068 (0.41), 4.146 (0.73), 4.157 (0.92), 4.171 (0.97), 4.182 (0.95), 4.342 (0.82), 4.360 (0.97), 4.367 (0.75), 4.385 (0.71), 4.657 (0.60), 5.728 (1.34), 5.734 (1.14), 5.754 (1.16), 5.760 (1.45), 6.101 (0.54), 6.107 (0.51), 6.112 (1.67), 6.124 (1.87), 6.136 (1.46), 6.209 (1.14), 6.215 (1.19), 6.251 (1.40), 6.257 (1.40), 6.605 (1.28), 6.613 (0.44), 6.619 (1.50), 6.645 (1.60), 6.661 (1.60), 6.666 (3.49), 6.668 (3.76), 6.679 (5.40), 6.687 (1.96), 7.136 (1.62), 7.281 (0.68), 7.293 (0.51), 7.339 (2.72), 7.351 (3.08), 7.363 (0.70), 7.425 (0.66), 7.459 (3.73), 7.981 (3.90), 7.994 (3.51), 8.066 (0.56), 8.070 (0.41), 8.079 (0.48), 8.283 (0.44), 8.337 (0.71), 8.370 (0.41), 8.386 (0.53), 8.418 (4.19), 8.553 (2.89), 11.562 (0.66).

LC-MS (method 5): Rₜ = 0.82 min; MS (ESlpos): m/z = 522 [M+H]⁺

### Example 14

### N-(2-{[4-(3-{[2-(difluoromethoxy)phenyl]amino}-4-oxo-4,5,6,7-tetrahydro-1H-pyrrolo[3,2-c]pyridin-2-yl)pyridin-3-yl]oxy}ethyl)-N-methylprop-2-enamide

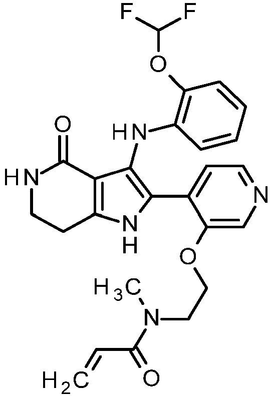

Using an analogous method as described for Example 4 with 3-{[2-(difluoromethoxy)phenyl]-amino}-2-{3-[2-(methylamino)ethoxy]pyridin-4-yl}-1,5,6,7-tetrahydro-4H-pyrrolo[3,2-c]pyridin-4-one (50.0 mg, 113 µmol, intermediate 7-11) and prop-2-enoic acid (12 µl, 170 µmol) as the starting materials, the title compound was prepared (28 mg, 47%) after basic preparative HPLC.

¹H-NMR (400MHz, DMSO-d₆): δ [ppm]= 11.06 - 11.27 (m, 1H), 8.32 - 8.37 (m, 1H), 7.93 - 8.05 (m, 1H), 7.33 - 7.45 (m, 2H), 6.56 - 7.27 (m, 6H), 5.95 - 6.30 (m, 2H), 5.46 - 5.80 (m, 1H), 4.12 - 4.41 (m, 2H), 3.83 - 3.96 (m, 2H), 3.38 - 3.45 (m, 2H), 2.79 - 3.21 (m, 5H).

### EXPERIMENTAL SECTION - BIOLOGICAL ASSAYS

The pharmacological activity of the compounds according to the invention can be assessed using *in vitro-* and/or *in vivo*-assays, as known to the person skilled in the art. The following examples describe the biological activity of the compounds according to the invention, without the invention being limited to said examples.

Example compounds according to the invention were tested in selected biological assays one or more times. When tested more than once, data are reported as either average values or as median values, wherein
- the average value, also referred to as the arithmetic mean value, represents the sum of the values obtained divided by the number of times tested, and
- the median value represents the middle number of the group of values when ranked in ascending or descending order. If the number of values in the data set is odd, the median is the middle value. If the number of values in the data set is even, the median is the arithmetic mean of the two middle values.

Examples were synthesized one or more times. When synthesized more than once, data from biological assays represent average values or median values calculated utilizing data sets obtained from testing of one or more synthetic batch.

The *in vitro* activity of the compounds of the present invention can be demonstrated in the following assays:

### Expression and purification of the EGFR proteins used in the biochemical kinase assays

The different EGFR proteins used in the biochemical kinase activity inhibition assays were generated inhouse by expression in insect cells using Baculo Virus system and subsequent purification as described in the following paragraphs.

Expression constructs:
The cDNAs encoding the various protein sequences from human EGFR human (P00533) were optimized for expression in eukaryotic cells and synthesized by the GeneArt Technology at Life Technologies.

These DNA sequences encoded the following sequence:
Construct EGFR #1 amino acid R669 to A1210
Construct EGFR #2 amino acid R669 to A1210 and the insertion of the amino acids sequence ASV between V769 and D770
Construct EGFR #3 amino acid R669 to A1210 and the insertion of the amino acids sequence SVD between D770 and N771
Additionally all constructs EGFR #1 to #3 encoded: at the N-terminus a TEV (Tobacco etch virus) protease cleavage site (DYDIPTTENLYFQG), at the C-terminus two stop codons and additionally 5' and 3' att-DNA sequences for Gateway Cloning.

Each of the four EFGR constructs was subcloned using the Gateway Technology into the Destination vector pD-Ins1. The vector pD-Ins1 is a Baculovirus transfer vector (based on vector pVL1393, Pharmingen) which provides a N-terminal fusion of a GST-tag to the integrated gene construct. The respective transfer vectors were termed pD-Ins1_ EGFR #1, pD-Ins1_ EGFR #2, pD-Ins1_ EGFR #3.

### EGFR amino acid sequences:

GST-EGFR #1 (Wild Type) 
MSPILGYWKIKGLVQPTRLLLEYLEEKYEEHLYERDEGDKWRNKKFELGLEFPNLPYYIDGDV
KLTQSMAIIRYIADKHNMLGGCPKERAEISMLEGAVLDIRYGVSRIAYSKDFETLKVDFLSKLPE
MLKMFEDRLCHKTYLNGDHVTHPDFMLYDALDVVLYMDPMCLDAFPKLVCFKKRIEAIPQIDK
YLKSSKYIAWPLQGWQATFGGGDHPPKSDPITSLYKKAGSDYDIPTTTENLYFQGRRRHIVRK
RTLRRLLQERELVEPLTPSGEAPNQALLRILKETEFKKIKVLGSGAFGTVYKGLWIPEGEKVKIP
VAIKELREATSPKANKEILDEAYVMASVDNPHVCRLLGICLTSTVQLITQLMPFGCLLDYVREH
KDNIGSQYLLNWCVQIAKGMNYLEDRRLVHRDLAARNVLVKTPQHVKITDFGLAKLLGAEEKE
YHAEGGKVPIKWMALESILHRIYTHQSDVWSYGVTVWELMTFGSKPYDGIPASEISSILEKGE
RLPQPPICTIDVYMIMVKCWMIDADSRPKFRELIIEFSKMARDPQRYLVIQGDERMHLPSPTDS
NFYRALMDEEDMDDVVDADEYLIPQQGFFSSPSTSRTPLLSSLSATSNNSTVACIDRNGLQSC
PIKEDSFLQRYSSDPTGALTEDSIDDTFLPVPEYINQSVPKRPAGSVQNPVYHNQPLNPAPSR
DPHYQDPHSTAVGNPEYLNTVQPTCVNSTFDSPAHWAQKGSHQISLDNPDYQQDFFPKEAK
PNGIFKGSTAENAEYLRVAPQSSEFIGA
GST-EGFR #2 (ASV between V769 and D770) 
MSPILGYWKIKGLVQPTRLLLEYLEEKYEEHLYERDEGDKWRNKKFELGLEFPNLPYYIDGDV
KLTQSMAIIRYIADKHNMLGGCPKERAEISMLEGAVLDIRYGVSRIAYSKDFETLKVDFLSKLPE
MLKMFEDRLCHKTYLNGDHVTHPDFMLYDALDVVLYMDPMCLDAFPKLVCFKKRIEAIPQIDK
YLKSSKYIAWPLQGWQATFGGGDHPPKSDPITSLYKKAGSDYDIPTTTENLYFQGRRRHIVRK
RTLRRLLQERELVEPLTPSGEAPNQALLRILKETEFKKIKVLGSGAFGTVYKGLWIPEGEKVKIP
 
VAIKELREATSPKANKEILDEAYVMASVDASVNPHVCRLLGICLTSTVQLITQLMPFGCLLDYVR
EHKDNIGSQYLLNWCVQIAKGMNYLEDRRLVHRDLAARNVLVKTPQHVKITDFGLAKLLGAEE
KEYHAEGGKVPIKWMALESILHRIYTHQSDVWSYGVTVWELMTFGSKPYDGIPASEISSILEKG
ERLPQPPICTIDVYMIMVKCWMIDADSRPKFRELIIEFSKMARDPQRYLVIQGDERMHLPSPTD
SNFYRALMDEEDMDDVVDADEYLIPQQGFFSSPSTSRTPLLSSLSATSNNSTVACIDRNGLQS
CPIKEDSFLQRYSSDPTGALTEDSIDDTFLPVPEYINQSVPKRPAGSVQNPVYHNQPLNPAPS
RDPHYQDPHSTAVGNPEYLNTVQPTCVNSTFDSPAHWAQKGSHQISLDNPDYQQDFFPKEA
KPNGIFKGSTAENAEYLRVAPQSSEFIGA
GST-EGFR #3 (SVD between D770 and N771)

MSPILGYWKIKGLVQPTRLLLEYLEEKYEEHLYERDEGDKWRNKKFELGLEFPNLPYYIDGDV
KLTQSMAIIRYIADKHNMLGGCPKERAEISMLEGAVLDIRYGVSRIAYSKDFETLKVDFLSKLPE
MLKMFEDRLCHKTYLNGDHVTHPDFMLYDALDVVLYMDPMCLDAFPKLVCFKKRIEAIPQIDK
YLKSSKYIAWPLQGWQATFGGGDHPPKSDPITSLYKKAGSDYDIPTTTENLYFQGRRRHIVRK
RTLRRLLQERELVEPLTPSGEAPNQALLRILKETEFKKIKVLGSGAFGTVYKGLWIPEGEKVKIP
VAIKELREATSPKANKEILDEAYVMASVDSVDNPHVCRLLGICLTSTVQLITQLMPFGCLLDYV
REHKDNIGSQYLLNWCVQIAKGMNYLEDRRLVHRDLAARNVLVKTPQHVKITDFGLAKLLGAE
EKEYHAEGGKVPIKWMALESILHRIYTHQSDVWSYGVTVWELMTFGSKPYDGIPASEISSILEK
GERLPQPPICTIDVYMIMVKCWMIDADSRPKFRELIIEFSKMARDPQRYLVIQGDERMHLPSPT
DSNFYRALMDEEDMDDVVDADEYLIPQQGFFSSPSTSRTPLLSSLSATSNNSTVACIDRNGLQ
SCPIKEDSFLQRYSSDPTGALTEDSIDDTFLPVPEYINQSVPKRPAGSVQNPVYHNQPLNPAP
SRDPHYQDPHSTAVGNPEYLNTVQPTCVNSTFDSPAHWAQKGSHQISLDNPDYQQDFFPKE
AKPNGIFKGSTAENAEYLRVAPQSSEFIGA

### Generation of recombinant Baculovirus:

In separate approaches each of the three transfer vectors was co-transfected in Sf9 cells with Baculovirus DNA (Flashbac Gold DNA, Oxford Expression Technologies) using Fugene HD (Roche). After 5 days the supernatant of the transfected cells containing the recombinant Baculovirus encoding the various EGFR proteins was used for further infection of Sf9 cells for virus amplification whereby the virus titer was monitored using qPCR.

### EGFR expression in Sf9 cells using bioreactor:

Sf9 cells cultured (Insect-xpress medium, Lonza, 27 °C) in a Wave-bioreactor with a disposable culture bag were infected at a cell density of 106 cells/ml with one of the recombinant baculovirus stocks at a multiplicity of infection of 1 and incubated for 48 h. Subsequently the cells were harvested by centrifugation and the cell pellet frozen at -80 °C.

### Purification of the GST-EGFR fusion proteins:

Purification of the GST-EGFR fusion proteins was achieved by affinity chromatography using Glutathion Sepharose 4B matrix (GE Healthcare Life Sciences).

The pelleted cells (from 4 I cell culture) were resuspended in Lysis-Buffer (50 mM HEPES pH 7.4, 150 mM NaCl, 5% Glycerol, 1 mM MgCl2, 1 mM MnCl2, 0.5 mM Na3VO4) and lysed by a freeze-thaw cycle followed by an incubation on ice for 60 min. The supernatant was centrifuged at 4000 x g for 30 min. at 4 °C. The supernatant was than incubated with Glutathion Sepharose 4B matrix (in a glass bottle rotating for 16 h, at 4 °C) for binding of the GST EGFR fusion protein, rinsed with Wash-Buffer and finally the bound protein was eluted using Elusion-Buffer (Lysis Buffer plus 25 mM Glutathione) and shock frozen with liquid nitrogen.

### WT-EGFR kinase assay

Inhibitory activity of compounds of the present invention against wild-type Epidermal Growth Factor Receptor (EGFR) was quantified employing the TR-FRET based EGFR assay as described in the following paragraphs.

Recombinant fusion protein of N-terminal Glutathion-S-Transferase (GST) and a fragment of human EGFR (amino acids R669 to A1210), expressed in Sf9 insect cells and purified via affinity chromatography using Glutathion Sepharose as described above, was used as a kinase. As substrate for the kinase reaction the biotinylated peptide biotin-Ahx-AEEEEYFELVAKKK (C-terminus in amide form) was used, which can be purchased e.g. form the company Biosynthan GmbH (Berlin-Buch, Germany).

For the assay 50 nl of a 100 fold concentrated solution of the test compound in DMSO was pipetted into either a black low volume 384 well microtiter plate or a black 1536 well microtiter plate (both Greiner Bio-One, Frickenhausen, Germany), 2 µl of a solution of EGFR in aqueous assay buffer [50 mM Hepes pH 7.0, 10 mM MgCl2, 1mM dithiothreitol, 0.5 mM EGTA, 0.3 mM activated sodium ortho-vanadate, 0.005% (w/v) bovine serum albumin, 0.005% (v/v) Tween-20] were added and the mixture was incubated for 15 min at 22°C to allow pre binding of the test compounds to the enzyme before the start of the kinase reaction. Then the kinase reaction was started by the addition of 3 µL of a solution of adenosine tri phosphate (ATP, 3.33 mM => final conc. in the 5 µL assay volume is 2 mM) and substrate (1.67 µM => final conc. in the 5 µL assay volume is 1 µM) in assay buffer and the resulting mixture was incubated for a reaction time of 30 min at 22°C. The concentration of EGFR was adjusted depending of the activity of the enzyme lot and was chosen appropriate to have the assay in the linear range, typical concentration was 7.6 pg/µl. The reaction was stopped by the addition of 3 µl of a solution of HTRF detection reagents (83.3 nM streptavidine-XL665 [Cisbio Bioassays, Codolet, France] and 1.67 nM PT66-Tb-Cryptate, an terbium-cryptate labelled anti-phospho-tyrosine antibody from Cisbio Bioassays [instead of the PT66 Tb cryptate PT66 Eu Chelate from Perkin Elmer can also be used]) in an aqueous EDTA-solution (133.3 mM EDTA, 0.2% (w/v) bovine serum albumin in 50 mM HEPES pH 7.5).

The resulting mixture was incubated 1 h at 22°C to allow the binding of the biotinylated phosphorylated peptide to the streptavidine-XL665 and the PT66-Tb-Cryptate. Subsequently the amount of phosphorylated substrate was evaluated by measurement of the resonance energy transfer from the PT66-Tb-Cryptate to the streptavidine-XL665. Therefore, the fluorescence emissions at 620 nm and 665 nm after excitation at 337 nm were measured in a HTRF reader, *e.g*. a Pherastar (BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0% inhibition, all other assay components but no enzyme = 100% inhibition). Usually the test compounds were tested on the same microtiterplate in 11 different concentrations in the range of 20 µM to 0.07 nM (20 µM, 5.7 µM, 1.6 µM, 0.47 µM, 0.13 µM, 38 nM, 11 nM, 3.1 nM, 0.9 nM, 0.25 nM and 0.07 nM, the dilution series prepared separately before the assay on the level of the 100-fold concentrated solutions in DMSO by serial dilutions, exact concentrations may vary depending pipettors used) in duplicate values for each concentration and IC₅₀ values were calculated using Genedata Screener^{™} software.

### Exon20-mutant-EGFR(D770_N771insSVD) kinase assay

Inhibitory activity of compounds of the present invention against an Epidermal Growth Factor Receptor (EGFR) with an insertion of the amino acids sequence SVD between D770 and N771 was quantified employing the TR-FRET based kinase activity assay as described in the following paragraphs.

A recombinant fusion protein of N-terminal Glutathion-S-Transferase (GST) and a fragment of human EGFR variant (amino acids R669 to A1210 with insertion of the amino acids sequence SVD between D770 and N771 ("**EGFR ins SVD**"), expressed in Sf9 insect cells and purified via affinity chromatography using Glutathion Sepharose as described above, was used as a kinase. As substrate for the kinase reaction the biotinylated peptide biotin-Ahx-AEEEEYFELVAKKK (C-terminus in amide form) was used which can be purchased e.g. form the company Biosynthan GmbH (Berlin-Buch, Germany).

For the assay 50 nl of a 100-fold concentrated solution of the test compound in DMSO was pipetted into either a black low volume 384 well microtiter plate or a black 1536 well microtiter plate (both Greiner Bio-One, Frickenhausen, Germany), 2 µl of a solution of EGFR in aqueous assay buffer [50 mM Hepes pH 7.0, 10 mM MgCl2, 1 mM dithiothreitol, 0.5 mM EGTA, 0.3 mM activated sodium ortho-vanadate, 0.005% (w/v) bovine serum albumin, 0.005% (v/v) Tween-20] were added and the mixture was incubated for 15 min at 22°C to allow pre binding of the test compounds to the enzyme before the start of the kinase reaction. Then the kinase reaction was started by the addition of 3 µL of a solution of adenosine tri phosphate (ATP, 3.33 mM => final conc. in the 5 µL assay volume is 2 mM) and substrate (1.67 µM => final conc. in the 5 µL assay volume is 1 µM) in assay buffer and the resulting mixture was incubated for a reaction time of 30 min at 22°C. The concentration of EGFR was adjusted depending of the activity of the enzyme lot and was chosen appropriate to have the assay in the linear range, typical concentration was 15 pg/µl. The reaction was stopped by the addition of 3 µl of a solution of HTRF detection reagents (83.3 nM streptavidine-XL665 [Cisbio Bioassays, Codolet, France] and 1.67 nM PT66-Tb-Cryptate, a terbium-cryptate labelled anti-phospho-tyrosine antibody from Cisbio Bioassays [instead of the PT66 Tb cryptate PT66 Eu Chelate from Perkin Elmer can also be used]) in an aqueous EDTA-solution (133.3 mM EDTA, 0.2% (w/v) bovine serum albumin in 50 mM HEPES pH 7.5).

The resulting mixture was incubated 1 h at 22°C to allow the binding of the biotinylated phosphorylated peptide to the streptavidine-XL665 and the PT66-Tb-Cryptate. Subsequently the amount of phosphorylated substrate was evaluated by measurement of the resonance energy transfer from the PT66-Tb-Cryptate to the streptavidine-XL665. Therefore, the fluorescence emissions at 620 nm and 665 nm after excitation at 337 nm were measured in a HTRF reader, *e.g*. a Pherastar (BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0% inhibition, all other assay components but no enzyme = 100% inhibition). Usually the test compounds were tested on the same microtiterplate in 11 different concentrations in the range of 20 µM to 0.07 nM (20 µM, 5.7 µM, 1.6 µM, 0.47 µM, 0.13 µM, 38 nM, 11 nM, 3.1 nM, 0.9 nM, 0.25 nM and 0.07 nM, the dilution series prepared separately before the assay on the level of the 100fold concentrated solutions in DMSO by serial dilutions, exact concentrations may vary depending pipettors used) in duplicate values for each concentration and IC₅₀ values were calculated using Genedata Screener^{™} software.

### Exon20-mutant-EGFR(V769_D770insASV) kinase assay

Inhibitory activity of compounds of the present invention against an Epidermal Growth Factor Receptor (EGFR) with an insertion of the amino acids sequence ASV between V769 and D770 was quantified employing the TR-FRET based kinase activity assay as described in the following paragraphs.

A recombinant fusion protein of N-terminal Glutathion-S-Transferase (GST) and a fragment of human EGFR variant (amino acids R669 to A1210 with insertion of the amino acids sequence ASV between V769 and D770; ("**EGFR ins ASV**"), expressed in Sf9 insect cells and purified via affinity chromatography using Glutathion Sepharose as described above, was used as kinase. As substrate for the kinase reaction the biotinylated peptide biotin-Ahx-AEEEEYFELVAKKK (C-terminus in amide form) was used which can be purchased e.g. form the company Biosynthan GmbH (Berlin-Buch, Germany).

For the assay 50 nl of a 100-fold concentrated solution of the test compound in DMSO was pipetted into either a black low volume 384well microtiter plate or a black 1536 well microtiter plate (both Greiner Bio-One, Frickenhausen, Germany), 2 µl of a solution of EGFR in aqueous assay buffer [50 mM Hepes pH 7.0, 10 mM MgCl2, 1mM dithiothreitol, 0.5 mM EGTA, 0.3 mM activated sodium ortho-vanadate, 0.005% (w/v) bovine serum albumin, 0.005% (v/v) Tween-20] were added and the mixture was incubated for 15 min at 22°C to allow pre binding of the test compounds to the enzyme before the start of the kinase reaction. Then the kinase reaction was started by the addition of 3 µL of a solution of adenosine tri phosphate (ATP, 3.33 mM => final conc. in the 5 µL assay volume is 2 mM) and substrate (1.67 µM => final conc. in the 5 µL assay volume is 1 µM) in assay buffer and the resulting mixture was incubated for a reaction time of 30 min at 22°C. The concentration of EGFR was adjusted depending of the activity of the enzyme lot and was chosen appropriate to have the assay in the linear range, typical concentration was 2.5 pg/µl. The reaction was stopped by the addition of 3 µl of a solution of HTRF detection reagents (83.3 nM streptavidine-XL665 [Cisbio Bioassays, Codolet, France] and 1.67 nM PT66-Tb-Cryptate, an terbium-cryptate labelled anti-phospho-tyrosine antibody from Cisbio Bioassays [instead of the PT66 Tb cryptate PT66 Eu Chelate from Perkin Elmer can also be used]) in an aqueous EDTA-solution (133.3 mM EDTA, 0.2% (w/v) bovine serum albumin in 50 mM HEPES pH 7.5).

The resulting mixture was incubated 1 h at 22°C to allow the binding of the biotinylated phosphorylated peptide to the streptavidine-XL665 and the PT66-Tb-Cryptate. Subsequently the amount of phosphorylated substrate was evaluated by measurement of the resonance energy transfer from the PT66-Tb-Cryptate to the streptavidine-XL665. Therefore, the fluorescence emissions at 620 nm and 665 nm after excitation at 337 nm were measured in a HTRF reader, *e.g*. a Pherastar (BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0% inhibition, all other assay components but no enzyme = 100% inhibition). Usually the test compounds were tested on the same microtiterplate in 11 different concentrations in the range of 20 µM to 0.07 nM (20 µM, 5.7 µM, 1.6 µM, 0.47 µM, 0.13 µM, 38 nM, 11 nM, 3.1 nM, 0.9 nM, 0.25 nM and 0.07 nM, the dilution series prepared separately before the assay on the level of the 100-fold concentrated solutions in DMSO by serial dilutions, exact concentrations may vary depending pipettors used) in duplicate values for each concentration and IC₅₀ values were calculated using Genedata Screener^{™} software. Table 2 shows the results of the inhibition in mutant EGFR biochemical assay.

**Table 2:**

| **Example No.** | **mutEGFR (D770_N771insSVD) kinase assay** |
|---|---|
| | **IC₅₀** |
| | **[mol/l]** |
| **1** | 1.55 E-10 |
| **2** | 3.86 E-10 |
| **3** | 2.65 E-10 |
| **4** | 3.05 E-10 |
| **5** | 2.31 E-10 |
| **6** | 1.71 E-10 |
| **7** | 1.62 E-9 |
| **8** | 5.54 E-10 |
| **9** | 2.48 E-9 |
| **10** | 3.55 E-9 |
| **11** | 4.64 E-10 |
| **12** | 5.83 E-10 |
| **13** | 2.06 E-10 |
| **14** | 5.71 E-10 |

### Cellular Data Description (WT, insSVD, insSVD T790M)

293T cells from ATCC were transfected with pBABEpuro expression constructs for WT EGFR or EGFR-insSVD, or EGFR-insSVD T790M, and pCL-Eco packaging vector using Fugene-6 transfection reagent from Promega. Plates were incubated at at 37°C for 48 h. Retrovirus was harvested by filtering the media supernatant through a 0.45 µm filter.

Ba/F3 cells purchased from DSMZ were grown in RPMI + 10% FBS + 10 ng/mL IL-3 and infected with filtered retroviral supernatant at a 1:2 dilution. Polybrene was added to a concentration of 8 µg/mL, plates were spun for 90 min, and incubated for 16h at 37°C. 2 µg/mL puromycin was added to the infected cells 24 h after infection and cells were continually grown in the presence of puromycin and 10 ng/mL IL-3. Following stably expressing Ba/F3 cell lines were generated: Ba/F3-EGFR-WT, Ba/F3-EGFR-insSVD, Ba/F3-EGFR-insSVD T790M, (Ba/F3- vector-control).

For cell survival assays, Ba/F3 cells were grown to a density of 1-2 million cells per mL, spun down and resuspended in media without IL-3, and replated at a concentration 200,000-500,000 cells per mL. The cells ectopically expressing WT EGFR, EGFR-insSVD, or EGFR-insSVD T790M were plated with 10 ng/mL Millipore Culture grade EGF. The cells ectopically expressing pBABEpuro empty vector were plated with 10 ng/mL IL-3.

2 days later, cells were plated in 50 µL in a 384 well plate at a concentration of 4000 cells per well for cells assayed in the absence of IL-3 and 2000 cells per well for cells assayed in the presence of IL-3. 100 nL of compound was added to each well using a 100 nL pin head, and plates were incubated at 37°C for 48 h.

Cell viability was measured by adding 20 µL of Cell Titer-Glo Luminescent Cell Viability Reagent diluted 1:3 in PBS. Plates were sealed with Perkin Elmer Top-Seal, inverted several times to mix, and immediately centrifuged at 1000 rpm for 2 min. Plates were incubated in low light conditions for 8-10 min and luminescence was measured. The IC₅₀ values for the examples are shown in Table 3.

**Table 3:**

| **Example No.** | **BA/F3 (insSVD) IC₅₀ [mol/l]** | **BA/F3 (wild type) IC₅₀ [mol/l]** |
|---|---|---|
| **1** | 3.76 E-7 | 3.03 E-6 |
| **2** | 5.79 E-8 | 9.07 E-7 |
| **3** | 4.02 E-8 | 5.66 E-7 |
| **4** | 5.19 E-7 | 4.42 E-6 |
| **5** | 5.77 E-9 | 6.80 E-8 |
| **6** | 8.94 E-9 | 1.09 E-7 |
| **7** | 1.46 E-7 | 3.66 E-7 |
| **8** | 2.40 E-7 | 4.70 E-6 |
| **9** | 2.63 E-7 | 4.52 E-6 |
| **10** | 2.05 E-7 | 1.66 E-6 |
| **11** | 1.22 E-8 | 3.09 E-7 |
| **12** | 1.77 E-8 | 3.30 E-7 |
| **13** | 6.69 E-9 | 5.12 E-8 |
| **14** | 3.54 E-8 | 6.52 E-7 |

### Cellular Data Description (L858R, E746_A750del, L858R T790M, E746_A750del T790M)

293T cells from ATCC were transfected with pBABEpuro expression constructs for EGFR-L858R, EGFR-E746_A750del, EGFR-L858R T790M, or EGFR-E746_A750del T790M, and pCL-Eco packaging vector using Fugene-6 transfection reagent from Promega. Plates were incubated at at 37°C for 48 h. Retrovirus was harvested by filtering the media supernatant through a 0.45 µm filter.

Ba/F3 cells purchased from DSMZ were grown in RPMI + 10% FBS + 10 ng/mL IL-3 and infected with filtered retroviral supernatant at a 1:2 dilution. Polybrene was added to a concentration of 8 µg/mL, plates were spun for 90 min, and incubated for 16h at 37°C. 2 µg/mL puromycin was added to the infected cells 24 h after infection and cells were continually grown in the presence of puromycin and 10 ng/mL IL-3. Following stably expressing Ba/F3 cell lines were generated: Ba/F3-EGFR-L858R, Ba/F3-EGFR-E746_A750del, Ba/F3-EGFR-L858R T790M, or Ba/F3-EGFR-E746_A750del T790M.

For cell survival assays, Ba/F3 cells were grown to a density of 1-2 million cells per mL, spun down and resuspended in media without IL-3, and replated at a concentration 200,000-500,000 cells per mL. The cells ectopically expressing EGFR-L858R, EGFR-E746_A750del, EGFR-L858R T790M, or EGFR-E746_A750del T790M were plated with 10 ng/mL Millipore Culture grade EGF. The cells ectopically expressing pBABEpuro empty vector were plated with 10 ng/mL IL-3.

2 days later, cells were plated in 50 µL in a 384 well plate at a concentration of 4000 cells per well for cells assayed in the absence of IL-3 and 2000 cells per well for cells assayed in the presence of IL-3. 100 nL of compound was added to each well using a 100 nL pin head, and plates were incubated at 37°C for 48 h.

Cell viability was measured by adding 20 µL of Cell Titer-Glo Luminescent Cell Viability Reagent diluted 1:3 in PBS. Plates were sealed with Perkin Elmer Top-Seal, inverted several times to mix, and immediately centrifuged at 1000 rpm for 2 min. Plates were incubated in low light conditions for 8-10 min and luminescence was measured. The IC₅₀ values for the examples are shown in Table 4.

**Table 4:**

| **Example No.** | **BA/F3 (L858R T790M) IC₅₀ [mol/l]** | **BA/F3 (E746_A750del T790M) IC₅₀ [mol/l]** |
|---|---|---|
| **1** | >1.00 E-6 | > 1.00 E-6 |
| **2** | 2.15 E-7 | 2.52 E-8 |
| **3** | 2.01 E-7 | 3.26 E-8 |
| **4** | > 1.00 E-6 | > 1.00 E-6 |
| **5** | 1.29 E-8 | 3.39 E-9 |
| **6** | 2.64 E-8 | 4.96 E-9 |
| **7** | 2.74 E-7 | 2.19 E-8 |
| **8** | > 1.00 E-6 | 3.70 E-7 |
| **9** | 7.31 E-7 > 1.00 E-6 | 1.83 E-7 |
| **10** | !>1.00 E-6 | 6.51 E-7 |
| **11** | 1.98 E-7 | 2.58 E-8 |
| **12** | 2.76 E-7 | 4.74 E-8 |
| **13** | | |
| **14** | 3.97 E-7 | 291E-8 |

### References:

Arcila et al., 2012: Arcila et al., Clin Cancer Res. 2012 Sep 15;18(18):4910-8.
Chen *et al.*, 2016: Chen et al., Onco Targets Ther. 2016 Jul 8;9:4181-6
Chiu et al., 2015: Chiu et al., J Thorac Oncol. 2015;10: 793-799
Doebele et al., 2018 : Doebele et al.. Poster 338, presented at the 54th Annual Meeting of the American Society of Clinical Oncology, June 1-5, 2018, Chicago, Illinois
Floc'h et al., 2018 : Floc'h et al., Mol Cancer Ther. 2018 May 17(5) : 885-896
Hasako et al., 2018: Hasako et al., Mol Cancer Ther. 2018 Aug ;17(8):1648-1658
Jang et al., 2018: Jang et al., Angew Chem Int Ed Engl. 2018 Sep 3; 57(36): 11629-11633
Mok *et al.*, 2009 : Mok et al., N Engl J Med. 2009 Sep 3;361(10):947-57
Mok *et al.*, 2017: Mok et al., N Engl J Med. 2017 Feb 16;376(7):629-640
Oxnard *et al.*, 2013: Oxnard et al., J Thorac Oncol. 2013 Feb; 8(2): 179-184
Oxnard *et al.*, 2018: Oxnard et al., JAMA Oncol. 2018;4(11):1527-1534
Paez *et al.*, 2004 : Paez et al., Science. 2004 Jun 4;304(5676):1497-500
Pao *et al.*, 2005: Pao et al., PLoS Med. 2005 Mar;2(3):e73
Pao *et al.*, 2010: Pao and Chmielecki, Nat Rev Cancer. 2010 Nov;10(11):760-74
Ramalingam et al.,2018a: Ramalingam et al., J Clin Oncol. 2018 Mar 20;36(9):841-849.
Ramalingam et al., 2018b: Ramalingam et al., ESMO 2018; Annals Oncol. 2018 Oct : 29 (Suppl 8)
Robichaux et al., 2018 : Robichaux et al., Nat Med. 2018 May;24(5):638-646
Sequist *et al.*, 2013: Sequist et al., J Clin Oncol. 2013 Sep 20;31(27):3327-34
Soria et al., 2018: Soria et al., N Engl J Med. 2018 Jan 11;378(2):113-125.
Thress et al., 2015: Thress et al., Nat Med. 2015 Jun; 21(6): 560-562.
Yang *et al.*, 2015: Yang et al., Lancet Oncol. 2015 Jul;16(7):830-8
Yasuda, 2013: Yasuda, Sci Transl Med. 2013 Dec 18;5(216):216ra17.

## Claims

1. A compound of formula (I) 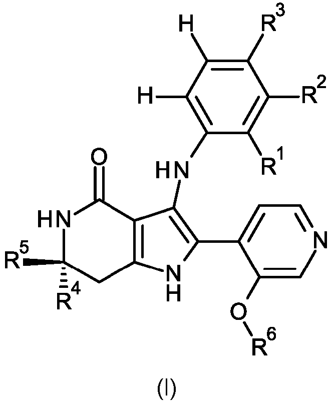 in which:
R¹ represents hydrogen, methyl, ethyl, trifluoromethyl, 2,2-difluoroethyl, cyano, chloro, bromo, methoxy, or difluoromethoxy;
R² represents hydrogen, methyl, ethyl, fluoro, chloro, or bromo;
R³ represents hydrogen or fluoro;
R⁴ represents hydrogen, methyl, or trifluoromethyl;
R⁵ represents hydrogen, methyl, or trifluoromethyl, or
R⁴ and R⁵, together with the carbon atom to which they are attached, represent a C₃-C₆-cycloalkyl group;
R⁶ represents a group selected from the group: 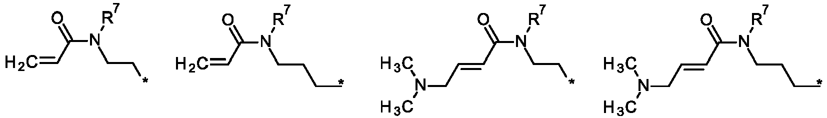 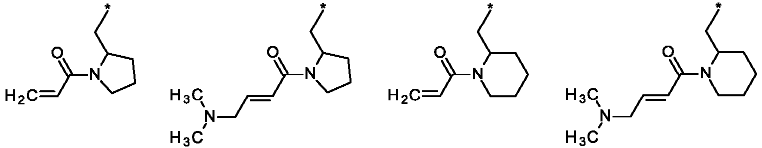 wherein * indicates the point of attachment of said group to the oxygen atom of the compound of formula (I);
R⁷ represents hydrogen or methyl;
or an N-oxide, a salt or a tautomer of said compound, or a salt of said N-oxide or tautomer.

2. The compound of formula (I) according to claim 1, wherein:
R¹ represents hydrogen, methyl, methoxy, or difluoromethoxy;
R² represents hydrogen, fluoro, or chloro;
R³ represents hydrogen or fluoro;
R⁴ represents hydrogen or methyl;
R⁵ represents hydrogen or methyl, or
R⁴ and R⁵, together with the carbon atom to which they are attached, represent a C₃-C₅-cycloalkyl group;
R⁶ represents a group selected from the group: 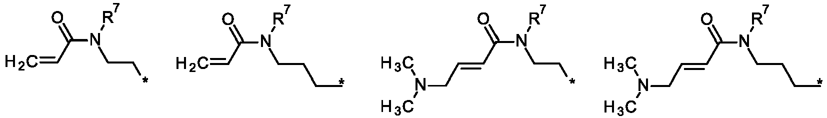 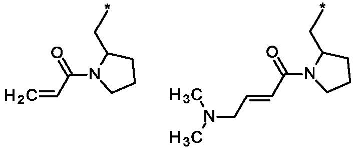 wherein * indicates the point of attachment of said group to the oxygen atom of the compound of formula (I);
R⁷ represents hydrogen or methyl;
or an N-oxide, a salt or a tautomer of said compound, or a salt of said N-oxide or tautomer.

3. The compound of formula (I) according to claim 1 or 2, wherein:
R¹ represents methyl, methoxy, or difluoromethoxy;
R² represents hydrogen, fluoro, or chloro;
R³ represents hydrogen;
R⁴ represents hydrogen or methyl;
R⁵ represents hydrogen or methyl, or
R⁴ and R⁵, together with the carbon atom to which they are attached, represent a cyclobutyl group;
R⁶ represents a group selected from the group: 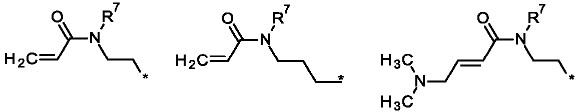 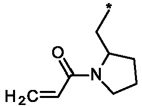 wherein * indicates the point of attachment of said group to the oxygen atom of the compound of formula (I);
R⁷ represents hydrogen or methyl;
or an N-oxide, a salt or a tautomer of said compound, or a salt of said N-oxide or tautomer.

4. The compound of formula (I) according to any of claims 1 to 3, which is selected from the group consisting of:
N-[2-({4-[3-(3-chloro-2-methoxyanilino)-4-oxo-4,5,6,7-tetrahydro-1H-pyrrolo[3,2-c]pyridin-2-yl]pyridin-3-yl}oxy)ethyl]prop-2-enamide
N-[2-({4-[3-(3-fluoro-2-methylanilino)-4-oxo-4,5,6,7-tetrahydro-1H-pyrrolo[3,2-c]pyridin-2-yl]pyridin-3-yl}oxy)ethyl]prop-2-enamide
N-[2-({4-[3-(3-chloro-2-methylanilino)-4-oxo-4,5,6,7-tetrahydro-1H-pyrrolo[3,2-c]pyridin-2-yl]pyridin-3-yl}oxy)ethyl]prop-2-enamide
N-[3-({4-[3-(3-chloro-2-methoxyanilino)-4-oxo-4,5,6,7-tetrahydro-1H-pyrrolo[3,2-c]pyridin-2-yl]pyridin-3-yl}oxy)propyl]prop-2-enamide
N-[2-({4-[3-(3-chloro-2-methoxyanilino)-4-oxo-4,5,6,7-tetrahydro-1H-pyrrolo[3,2-c]pyridin-2-yl]pyridin-3-yl}oxy)ethyl]-N-methylprop-2-enamide
N-[2-({4-[3-(3-fluoro-2-methoxyanilino)-4-oxo-4,5,6,7-tetrahydro-1H-pyrrolo[3,2-c]pyridin-2-yl]pyridin-3-yl}oxy)ethyl]-N-methylprop-2-enamide
N-[2-({4-[3-(4-fluoroanilino)-4-oxo-4,5,6,7-tetrahydro-1H-pyrrolo[3,2-c]pyridin-2-yl]pyridin-3-yl}oxy)ethyl]-N-methylprop-2-enamide
(2E)-4-(dimethylamino)-N-[2-({4-[3-(3-fluoro-2-methoxyanilino)-4-oxo-4,5,6,7-tetrahydro-1H-pyrrolo[3,2-c]pyridin-2-yl]pyridin-3-yl}oxy)ethyl]-N-methylbut-2-enamide
(2E)-N-[2-({4-[3-(3-chloro-2-methoxyanilino)-4-oxo-4,5,6,7-tetrahydro-1H-pyrrolo[3,2-c]pyridin-2-yl]pyridin-3-yl}oxy)ethyl]-4-(dimethylamino)-N-methylbut-2-enamide
(2E)-N-[2-({4-[3'-(3-chloro-2-methoxyanilino)-4'-oxo-1',4',5',7'-tetrahydrospiro[cyclobutane-1,6'-pyrrolo[3,2-c]pyridin]-2'-yl]pyridin-3-yl}oxy)ethyl]-4-(dimethylamino)-N-methylbut-2-enamide
N-[2-({4-[3'-(3-chloro-2-methoxyanilino)-4'-oxo-1',4',5',7'-tetrahydrospiro[cyclobutane-1,6'-pyrrolo[3,2-c]pyridin]-2'-yl]pyridin-3-yl}oxy)ethyl]-N-methylprop-2-enamide
N-[2-({4-[3-(3-chloro-2-methoxyanilino)-6,6-dimethyl-4-oxo-4,5,6,7-tetrahydro-1H-pyrrolo[3,2-c]pyridin-2-yl]pyridin-3-yl}oxy)ethyl]-N-methylprop-2-enamide
3-(3-chloro-2-methoxyanilino)-2-(3-{[(2S)-1-(prop-2-enoyl)pyrrolidin-2-yl]methoxy}pyridin-4-yl)-1,5,6,7-tetrahydro-4H-pyrrolo[3,2-c]pyridin-4-one
N-(2-{[4-(3-{[2-(difluoromethoxy)phenyl]amino}-4-oxo-4,5,6,7-tetrahydro-1H-pyrrolo[3,2-c]pyridin-2-yl)pyridin-3-yl]oxy}ethyl)-N-methylprop-2-enamide

5. A compound of general formula (I) according to any of claims 1 to 4 for use in the treatment or prophylaxis of diseases.

6. The compound for use according to claim 5, wherein the diseases are hyperproliferative diseases and/or disorders responsive to induction of cell death.

7. The compound for use according to claim 6, wherein the hyperproliferative diseases and/or disorders responsive to induction of cell death are haematological tumours, solid tumours and/or metastases thereof.

8. The compound for use according to claim 7, wherein the tumour harbors a mutant EGFR and/or metastases thereof.

9. The compound for use according to claim 7, wherein the tumour is lung cancer, particularly lung cancer harboring a mutant EGFR with exon 20 insertion mutation, and/or metastases thereof.

10. The compound for use according to claim 7, wherein the tumour is lung cancer, particularly lung cancer harboring a mutant EGFR with in-frame deletions in exon 19 (such as EGFR E746_A750del) or point mutations in exon 21 (e.g. L858R), and/or metastases thereof.

11. The compound for use according to claim 7, wherein the tumour is lung cancer, particularly lung cancer harboring a mutant EGFR with an exon 20 insertion and a T790M mutation, e.g. a D770_N771insSVD T790M mutation, and/or metastases thereof.

12. The compound for use according to claim 7, wherein the tumour is lung cancer, particularly lung cancer harboring a mutant EGFR with inframe deletion in exon 19 such as E746_A750del and a T790M mutation, and/or metastases thereof.

13. The compound for use according to claim 7, wherein the tumour is lung cancer, particularly lung cancer harboring a mutant EGFR with a point mutation in exon 21 such as L858R and a T790M mutation, and/or metastases thereof.

14. The compound for use according to claim 7, wherein the tumour is lung cancer, particularly lung cancer harboring a mutant ERBB2 with exon 20 insertion mutations (such as ERBB2 A775_G776insYVMA), and/or metastases thereof.

15. A pharmaceutical composition comprising at least one compound of general formula (I) according to any of claims 1 to 4, together with at least one pharmaceutically acceptable auxiliary.

16. A composition according to claim 15 for the treatment of haematological tumours, solid tumours and/or metastases thereof.

17. A combination comprising one or more first active ingredients selected from a compound of general formula (I) according to any of claims 1 to 4, and one or more second active ingredients selected from chemotherapeutic anti-cancer agents and target-specific anti-cancer agents.

## Patentansprüche

1. Eine Verbindung der Formel (I) 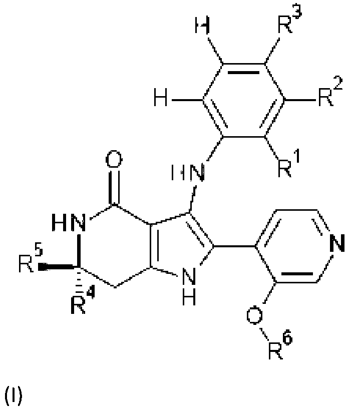 in der:
R¹ Wasserstoff, Methyl, Ethyl, Trifluormethyl, 2,2-Difluorethyl, Cyano, Chloro, Bromo, Methoxy oder Difluormethoxy darstellt;
R² Wasserstoff, Methyl, Ethyl, Fluoro, Chloro oder Bromo darstellt;
R³ Wasserstoff oder Fluoro darstellt;
R⁴ Wasserstoff, Methyl oder Trifluormethyl darstellt;
R⁵ Wasserstoff, Methyl oder Trifluormethyl darstellt, oder
R⁴ and R⁵ , zusammen mit dem Kohlenstoffatom, an das sie gebunden sind, eine C₃-C₆-Cycloalkylgruppe darstellen;
R⁶ eine Gruppe darstellt, die aus der Gruppe ausgewählt ist: 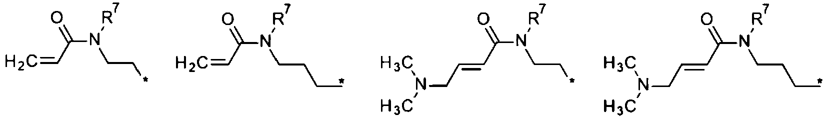 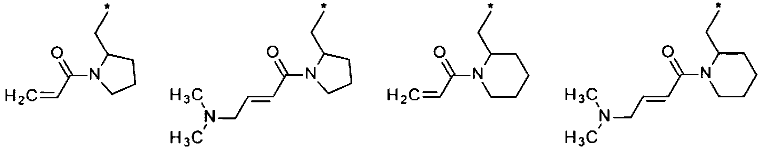 wobei * den Punkt der Anhaftung dieser Gruppe an das Sauerstoffatom der Verbindung der Formel (I) anzeigt;
R⁷ Wasserstoff oder Methyl darstellt;
oder ein N-Oxid, ein Salz oder ein Tautomer dieser Verbindung, oder ein Salz dieses N-Oxids oder Tautomers.

2. Die Verbindung der Formel (I) gemäß Anspruch 1, wobei:
R¹ Wasserstoff, Methyl, Methoxy oder Difluormethoxy darstellt;
R² Wasserstoff, Fluoro oder Chloro darstellt;
R³ Wasserstoff oder Fluoro darstellt;
R⁴ Wasserstoff oder Methyl darstellt;
R⁵ Wasserstoff oder Methyl darstellt, oder
R⁴ and R⁵ , zusammen mit dem Kohlenstoffatom, an das sie gebunden sind, eine C₃-C₅-Cycloalkylgruppe darstellen;
R⁶ eine Gruppe darstellt, die aus der Gruppe ausgewählt ist: 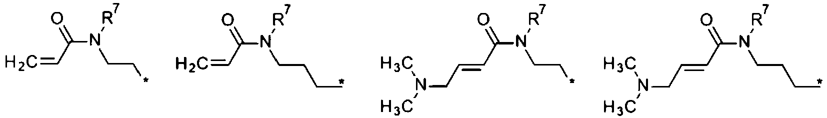 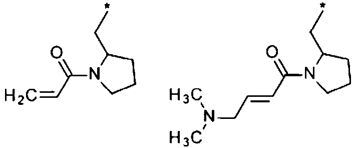 wobei * den Punkt der Anhaftung dieser Gruppe an das Sauerstoffatom der Verbindung der Formel (I) anzeigt;
R⁷ Wasserstoff oder Methyl darstellt;
oder ein N-Oxid, ein Salz oder ein Tautomer dieser Verbindung, oder ein Salz dieses N-Oxids oder Tautomers.

3. Die Verbindung der Formel (I) gemäß Anspruch 1 oder 2, wobei:
R¹ Methyl, Methoxy oder Difluormethoxy darstellt;
R² Wasserstoff, Fluoro oder Chloro darstellt;
R³ Wasserstoff darstellt;
R⁴ Wasserstoff oder Methyl darstellt;
R⁵ Wasserstoff oder Methyl darstellt, oder
R⁴ and R⁵ , zusammen mit dem Kohlenstoffatom, an das sie gebunden sind, eine Cyclobutylgruppe darstellen;
R⁶ eine Gruppe darstellt, die aus der Gruppe ausgewählt ist: 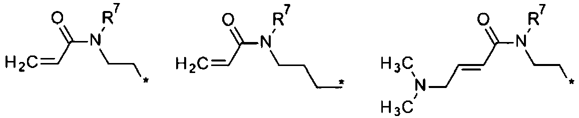 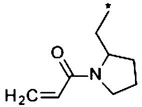 wobei * den Punkt der Anhaftung dieser Gruppe an das Sauerstoffatom der Verbindung der Formel (I) anzeigt;
R⁷ Wasserstoff oder Methyl darstellt;
oder ein N-Oxid, ein Salz oder ein Tautomer dieser Verbindung, oder ein Salz dieses N-Oxids oder Tautomers.

4. Die Verbindung der Formel (I) gemäß einem der Ansprüche 1 bis 3, die aus der Gruppe ausgewählt ist, bestehend aus:
N-[2-({4-[3-(3-chloro-2-methoxyanilino)-4-oxo-4,5,6,7-tetrahydro-1H-pyrrolo[3,2-c]pyridin-2-yl]pyridin-3-yl}oxy)ethyl]prop-2-enamid
N-[2-({4-[3-(3-fluoro-2-methylanilino)-4-oxo-4,5,6,7-tetrahydro-1H-pyrrolo[3,2-c]pyridin-2-yl]pyridin-3-yl}oxy)ethyl]prop-2-enamid
N-[2-({4-[3-(3-chloro-2-methylanilino)-4-oxo-4,5,6,7-tetrahydro-1H-pyrrolo[3,2-c]pyridin-2-yl]pyridin-3-yl}oxy)ethyl]prop-2-enamid
N-[3-({4-[3-(3-chloro-2-methoxyanilino)-4-oxo-4,5,6,7-tetrahydro-1H-pyrrolo[3,2-c]pyridin-2-yl]pyridin-3-yl}oxy)propyl]prop-2-enamid
N-[2-({4-[3-(3-chloro-2-methoxyanilino)-4-oxo-4,5,6,7-tetrahydro-1H-pyrrolo[3,2-c]pyridin-2-yl]pyridin-3-yl}oxy)ethyl]-N-methylprop-2-enamid
N-[2-({4-[3-(3-fluoro-2-methoxyanilino)-4-oxo-4,5,6,7-tetrahydro-1H-pyrrolo[3,2-c]pyridin-2-yl]pyridin-3-yl}oxy)ethyl]-N-methylprop-2-enamid
N-[2-({4-[3-(4-fluoroanilino)-4-oxo-4,5,6,7-tetrahydro-1H-pyrrolo[3,2-c]pyridin-2-yl]pyridin-3-yl}oxy)ethyl]-N-methylprop-2-enamid
(2E)-4-(dimethylamino)-N-[2-({4-[3-(3-fluoro-2-methoxyanilino)-4-oxo-4,5,6,7-tetrahydro-1H-pyrrolo[3,2-c]pyridin-2-yl]pyridin-3-yl}oxy)ethyl]-N-methylbut-2-enamid
(2E)-N-[2-({4-[3-(3-chloro-2-methoxyanilino)-4-oxo-4,5,6,7-tetrahydro-1H-pyrrolo[3,2-c]pyridin-2-yl]pyridin-3-yl}oxy)ethyl]-4-(dimethylamino)-N-methylbut-2-enamid
(2E)-N-[2-({4-[3'-(3-chloro-2-methoxyanilino)-4'-oxo-1',4',5',7'-tetrahydrospiro[cyclobutane-1,6'-pyrrolo[3,2-c]pyridin]-2'-yl]pyridin-3-yl}oxy)ethyl]-4-(dimethylamino)-N-methylbut-2-enamid
N-[2-({4-[3'-(3-chloro-2-methoxyanilino)-4'-oxo-1',4',5',7'-tetrahydrospiro[cyclobutane-1,6'-pyrrolo[3,2-c]pyridin]-2'-yl]pyridin-3-yl}oxy)ethyl]-N-methylprop-2-enamid
N-[2-({4-[3-(3-chloro-2-methoxyanilino)-6,6-dimethyl-4-oxo-4,5,6,7-tetrahydro-1H-pyrrolo[3,2-c]pyridin-2-yl]pyridin-3-yl}oxy)ethyl]-N-methylprop-2-enamid
3-(3-chloro-2-methoxyanilino)-2-(3-{[(2S)-1-(prop-2-enoyl)pyrrolidin-2-yl]methoxy}pyridin-4-yl)-1,5,6,7-tetrahydro-4H-pyrrolo[3,2-c]pyridin-4-on
N-(2-{[4-(3-{[2-(difluormethoxy)phenyl]amino}-4-oxo-4,5,6,7-tetrahydro-1H-pyrrolo[3,2-c]pyridin-2-yl)pyridin-3-yl]oxy}ethyl)-N-methylprop-2-enamid

5. Eine Verbindung der allgemeinen Formel (I) gemäß einem der Ansprüche 1 bis 4 zur Verwendung in der Behandlung oder Prophylaxe von Krankheiten.

6. Die Verbindung zur Verwendung gemäß Anspruch 5, wobei die Krankheiten hyperproliferative Krankheiten und/oder Störungen sind, die auf die Induktion des Zelltods ansprechen.

7. Die Verbindung zur Verwendung gemäß Anspruch 6, wobei die hyperproliferativen Krankheiten und/oder Störungen, die auf die Induktion des Zelltods ansprechen, hämatologische Tumore, solide Tumore und/oder deren Metastasen sind.

8. Die Verbindung zur Verwendung gemäß Anspruch 7, wobei der Tumor ein mutiertes EGFR und/oder dessen Metastasen aufweist.

9. Die Verbindung zur Verwendung gemäß Anspruch 7, wobei der Tumor Lungenkrebs ist, insbesondere Lungenkrebs mit einem mutierten EGFR mit Exon-20-Insertion-Mutation und/oder deren Metastasen.

10. Die Verbindung zur Verwendung gemäß Anspruch 7, wobei der Tumor Lungenkrebs ist, insbesondere Lungenkrebs mit einem mutierten EGFR mit In-Frame-Streichungen in Exon 19 (wie EGFR E746_A750del) oder Punktmutationen in Exon 21 (z. B. L858R) und/oder deren Metastasen.

11. Die Verbindung zur Verwendung gemäß Anspruch 7, wobei der Tumor Lungenkrebs ist, insbesondere Lungenkrebs mit einem mutierten EGFR mit einer Exon-20-Insertion und einer T790M-Mutation, z. B. einer D770_N771insSVD T790M-Mutation, und/oder deren Metastasen.

12. Die Verbindung zur Verwendung gemäß Anspruch 7, wobei der Tumor Lungenkrebs ist, insbesondere Lungenkrebs mit einer In-Frame-Streichung in Exon 19 wie E746_A750del und einer T790M-Mutation, und/oder deren Metastasen.

13. Die Verbindung zur Verwendung gemäß Anspruch 7, wobei der Tumor Lungenkrebs ist, insbesondere Lungenkrebs mit einem mutierten EGFR mit einer Punktmutation in Exon 21 wie L858R und einer T790M-Mutation, und/oder deren Metastasen.

14. Die Verbindung zur Verwendung gemäß Anspruch 7, wobei der Tumor Lungenkrebs ist, insbesondere Lungenkrebs mit einem mutierten ERBB2 mit Exon-20-Insertion-Mutationen (wie ERBB2 A775_G776insYVMA) und/oder deren Metastasen.

15. Eine pharmazeutische Zusammensetzung umfassend mindestens eine Verbindung der allgemeinen Formel (I) gemäß einem der Ansprüche 1 bis 4, zusammen mit mindestens einem pharmazeutisch akzeptablen Hilfsstoff.

16. Eine Zusammensetzung gemäß Anspruch 15 zur Behandlung von hämatologischen Tumoren, soliden Tumoren und/oder deren Metastasen.

17. Eine Kombination umfassend ein oder mehrere erste Wirkstoffe, ausgewählt aus einer Verbindung der allgemeinen Formel (I) gemäß einem der Ansprüche 1 bis 4, und ein oder mehrere zweite Wirkstoffe, ausgewählt aus chemotherapeutischen Anti-Krebs-Mitteln und zielgerichteten Anti-Krebs-Mitteln.

## Revendications

1. Un composé de formule (I) 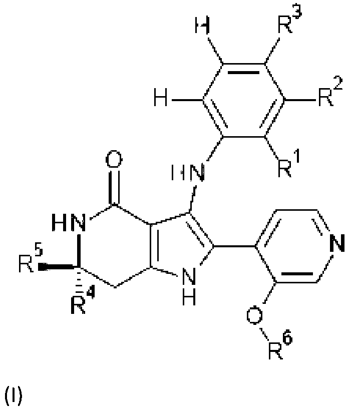 dans laquelle :
R¹ représente hydrogène, méthyle, éthyle, trifluorométhyle, 2,2-difluoroéthyle, cyano, chloro, bromo, méthoxy ou difluorométhoxy ;
R² représente hydrogène, méthyle, éthyle, fluoro, chloro ou bromo ;
R³ représente hydrogène ou fluoro ;
R⁴ représente hydrogène, méthyle ou trifluorométhyle ;
R⁵ représente hydrogène, méthyle ou trifluorométhyle, ou
R⁴ and R⁵ , avec l'atome de carbone auquel ils sont liés, représentent un groupe cycloalkyle C₃-C₆ ;
R⁶ représente un groupe choisi dans le groupe : 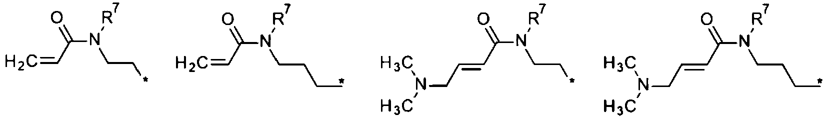 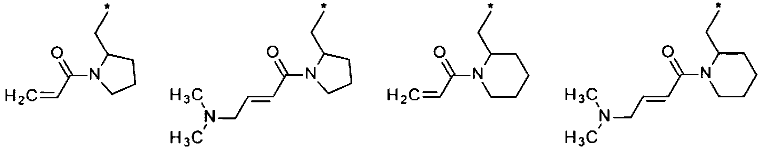 dans lequel * indique le point de liaison dudit groupe à l'atome d'oxygène du composé de formule (I) ;
R⁷ représente hydrogène ou méthyle ;
ou un N-oxyde, un sel ou un tautomère dudit composé, ou un sel dudit N-oxyde ou dudit tautomère.

2. Le composé de formule (I) selon la revendication 1, dans laquelle :
R¹ représente hydrogène, méthyle, méthoxy ou difluorométhoxy ;
R² représente hydrogène, fluoro ou chloro ;
R³ représente hydrogène ou fluoro ;
R⁴ représente hydrogène ou méthyle ;
R⁵ représente hydrogène ou méthyle, ou
R⁴ and R⁵ , avec l'atome de carbone auquel ils sont liés, représentent un groupe cycloalkyle C₃-C₅ ;
R⁶ représente un groupe choisi dans le groupe : 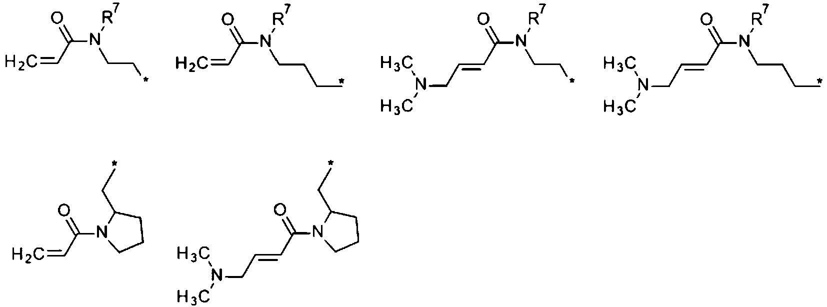 dans lequel * indique le point de liaison dudit groupe à l'atome d'oxygène du composé de formule (I) ;
R⁷ représente hydrogène ou méthyle ;
ou un N-oxyde, un sel ou un tautomère dudit composé, ou un sel dudit N-oxyde ou dudit tautomère.

3. Le composé de formule (I) selon la revendication 1 ou 2, dans laquelle :
R¹ représente méthyle, méthoxy ou difluorométhoxy ;
R² représente hydrogène, fluoro ou chloro ;
R³ représente hydrogène ;
R⁴ représente hydrogène ou méthyle ;
R⁵ représente hydrogène ou méthyle, ou
R⁴ and R⁵ , avec l'atome de carbone auquel ils sont liés, représentent un groupe cyclobutyle ;
R⁶ représente un groupe choisi dans le groupe : 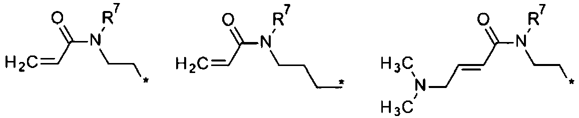 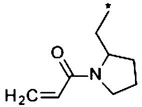 dans lequel * indique le point de liaison dudit groupe à l'atome d'oxygène du composé de formule (I) ;
R⁷ représente hydrogène ou méthyle ;
ou un N-oxyde, un sel ou un tautomère dudit composé, ou un sel dudit N-oxyde ou dudit tautomère.

4. Le composé de formule (I) selon l'une quelconque des revendications 1 à 3, qui est choisi dans le groupe constitué de :
N-[2-({4-[3-(3-chloro-2-méthoxyanilino)-4-oxo-4,5,6,7-tétrahydro-1H-pyrrolo[3,2-c]pyridin-2-yl]pyridin-3-yl}oxy)éthyl]prop-2-énamide
N-[2-({4-[3-(3-fluoro-2-méthylanilino)-4-oxo-4,5,6,7-tétrahydro-1H-pyrrolo[3,2-c]pyridin-2-yl]pyridin-3-yl}oxy)éthyl]prop-2-énamide
N-[2-({4-[3-(3-chloro-2-méthylanilino)-4-oxo-4,5,6,7-tétrahydro-1H-pyrrolo[3,2-c]pyridin-2-yl]pyridin-3-yl}oxy)éthyl]prop-2-énamide
N-[3-({4-[3-(3-chloro-2-méthoxyanilino)-4-oxo-4,5,6,7-tétrahydro-1H-pyrrolo[3,2-c]pyridin-2-yl]pyridin-3-yl}oxy)propyl]prop-2-énamide
N-[2-({4-[3-(3-chloro-2-méthoxyanilino)-4-oxo-4,5,6,7-tétrahydro-1H-pyrrolo[3,2-c]pyridin-2-yl]pyridin-3-yl}oxy)éthyl]-N-méthylprop-2-énamide
N-[2-({4-[3-(3-fluoro-2-méthoxyanilino)-4-oxo-4,5,6,7-tétrahydro-1H-pyrrolo[3,2-c]pyridin-2-yl]pyridin-3-yl}oxy)éthyl]-N-méthylprop-2-énamide
N-[2-({4-[3-(4-fluoroanilino)-4-oxo-4,5,6,7-tétrahydro-1H-pyrrolo[3,2-c]pyridin-2-yl]pyridin-3-yl}oxy)éthyl]-N-méthylprop-2-énamide
(2E)-4-(diméthylamino)-N-[2-({4-[3-(3-fluoro-2-méthoxyanilino)-4-oxo-4,5,6,7-tétrahydro-1H-pyrrolo[3,2-c]pyridin-2-yl]pyridin-3-yl}oxy)éthyl]-N-méthylbut-2-énamide
(2E)-N-[2-({4-[3-(3-chloro-2-méthoxyanilino)-4-oxo-4,5,6,7-tétrahydro-1H-pyrrolo[3,2-c]pyridin-2-yl]pyridin-3-yl}oxy)éthyl]-4-(diméthylamino)-N-méthylbut-2-énamide
(2E)-N-[2-({4-[3'-(3-chloro-2-méthoxyanilino)-4'-oxo-1',4',5',7'-tétrahydrospiro[cyclobutane-1,6'-pyrrolo[3,2-c]pyridin]-2'-yl]pyridin-3-yl}oxy)éthyl]-4-(diméthylamino)-N-méthylbut-2-énamide
N-[2-({4-[3'-(3-chloro-2-méthoxyanilino)-4'-oxo-1',4',5',7'-tétrahydrospiro[cyclobutane-1,6'-pyrrolo[3,2-c]pyridin]-2'-yl]pyridin-3-yl}oxy)éthyl]-N-méthylprop-2-énamide
N-[2-({4-[3-(3-chloro-2-méthoxyanilino)-6,6-diméthyl-4-oxo-4,5,6,7-tétrahydro-1H-pyrrolo[3,2-c]pyridin-2-yl]pyridin-3-yl}oxy)éthyl]-N-méthylprop-2-énamide
3-(3-chloro-2-méthoxyanilino)-2-(3-{[(2S)-1-(prop-2-énoyl)pyrrolidin-2-yl]méthoxy}pyridin-4-yl)-1,5,6,7-tétrahydro-4H-pyrrolo[3,2-c]pyridin-4-one
N-(2-{[4-(3-{[2-(difluorométhoxy)phényl]amino}-4-oxo-4,5,6,7-tétrahydro-1H-pyrrolo[3,2-c]pyridin-2-yl)pyridin-3-yl]oxy}éthyl)-N-méthylprop-2-énamide

5. Un composé de formule générale (I) selon l'une quelconque des revendications 1 à 4 pour une utilisation dans le traitement ou la prophylaxie de maladies.

6. Le composé pour une utilisation selon la revendication 5, dans laquelle les maladies sont des maladies hyperprolifératives et/ou des troubles répondant à l'induction de la mort cellulaire.

7. Le composé pour une utilisation selon la revendication 6, dans laquelle les maladies hyperprolifératives et/ou les troubles répondant à l'induction de la mort cellulaire sont des tumeurs hématologiques, des tumeurs solides et/ou leurs métastases.

8. Le composé pour une utilisation selon la revendication 7, dans laquelle la tumeur présente un EGFR mutant et/ou des métastases de celle-ci.

9. Le composé pour une utilisation selon la revendication 7, dans laquelle la tumeur est un cancer du poumon, en particulier un cancer du poumon présentant un EGFR mutant avec une mutation d'insertion dans l'exon 20, et/ou des métastases de celui-ci.

10. Le composé pour une utilisation selon la revendication 7, dans laquelle la tumeur est un cancer du poumon, en particulier un cancer du poumon présentant un EGFR mutant avec des délétions en phase dans l'exon 19 (telles que EGFR E746_A750del) ou des mutations ponctuelles dans l'exon 21 (par exemple L858R), et/ou des métastases de celui-ci.

11. Le composé pour une utilisation selon la revendication 7, dans laquelle la tumeur est un cancer du poumon, en particulier un cancer du poumon présentant un EGFR mutant avec une insertion dans l'exon 20 et une mutation T790M, par exemple une mutation D770_N771insSVD T790M, et/ou des métastases de celui-ci.

12. Le composé pour une utilisation selon la revendication 7, dans laquelle la tumeur est un cancer du poumon, en particulier un cancer du poumon présentant un EGFR mutant avec une délétion en phase dans l'exon 19 telle que E746_A750del et une mutation T790M, et/ou des métastases de celui-ci.

13. Le composé pour une utilisation selon la revendication 7, dans laquelle la tumeur est un cancer du poumon, en particulier un cancer du poumon présentant un EGFR mutant avec une mutation ponctuelle dans l'exon 21 telle que L858R et une mutation T790M, et/ou des métastases de celui-ci.

14. Le composé pour une utilisation selon la revendication 7, dans laquelle la tumeur est un cancer du poumon, en particulier un cancer du poumon présentant un ERBB2 mutant avec des mutations d'insertion dans l'exon 20 (telles que ERBB2 A775_G776insYVMA), et/ou des métastases de celui-ci.

15. Une composition pharmaceutique comprenant au moins un composé de formule générale (I) selon l'une quelconque des revendications 1 à 4, avec au moins un auxiliaire pharmaceutiquement acceptable.

16. Une composition selon la revendication 15 pour le traitement de tumeurs hématologiques, de tumeurs solides et/ou de leurs métastases.

17. Une combinaison comprenant un ou plusieurs premiers principes actifs choisis dans un composé de formule générale (I) selon l'une quelconque des revendications 1 à 4, et un ou plusieurs seconds principes actifs choisis dans des agents anticancéreux chimiothérapeutiques et des agents anticancéreux spécifiques de cible.
